# EUROPEAN PATENT APPLICATION

(11) **EP 2 072 522 A1**
(43) Date of publication of application: **24.06.2009**
(21) Application number: 07829669.6
(22) Date of filing: 12.10.2007
(51) Int. Cl.: C07H 7/06, A61K 31/7048, A61P 3/04, A61P 3/06, A61P 3/10, A61P 9/04, A61P 9/10, A61P 9/12, A61P 13/12, A61P 19/06, A61P 25/00, A61P 27/02, A61P 31/04, A61P 43/00

(54) **THIOGLUCOSE SPIROKETAL DERIVATIVE AND USE THEREOF AS THERAPEUTIC AGENT FOR DIABETES**

(30) Priority: 13.10.2006 JP 2006280159
(71) Applicant: CHUGAI SEIYAKU KABUSHIKI KAISHA, Tokyo, 115-8543 (JP)
(72) Inventor: SATO, Tsutomu, Shizuoka 412-8513 (JP)
(74) Representative: Vossius & Partner
(86) International application number: PCT/JP2007/069933
(87) International publication number: WO 2008/044762

(57) **Abstract**

The present invention provides a compound represented by Formula (II): 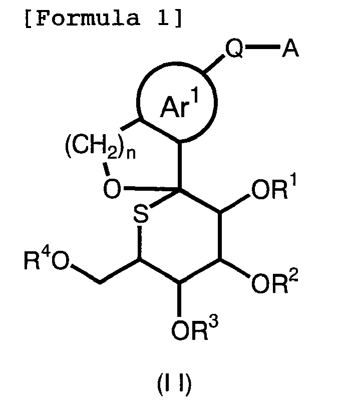 wherein R¹, R², R³, and R⁴ are each independently selected from a hydrogen atom, an optionally substituted C₁-C₆ alkyl group, an optionally substituted C₇-C₁₄ aralkyl group and - C(=O)Rx; Rx is an optionally substituted C₁-C₆ alkyl group, an optionally substituted aryl group, an optionally substituted heteroaryl group, an optionally substituted C₁-C₆ alkoxy group or -NReRf; Ar¹ is an optionally substituted aromatic carbocyclic ring, or an optionally substituted aromatic heterocyclic ring which may form a condensed ring; Q is - (CH₂)ₘ-(L)ₚ- or -(L)ₚ-(CH₂)ₘ-; m is an integer selected from 0 to 2, n is an integer selected from 1 and 2, and p is an integer selected from 0 and 1; L is -O-, -S- or -NR⁵-; A is an optionally substituted aryl group or an optionally substituted heteroaryl group; and a prodrug thereof and a pharmaceutically acceptable salt thereof, and a pharmaceutical agent or a pharmaceutical composition, which comprises the compound.

## Description

### TECHNICAL FIELD

The present invention relates to thioglucose spiro-derivatives useful as pharmaceutical agents, prodrugs thereof and pharmaceutically acceptable salts thereof. Particularly, the present invention relates to thioglucose spiro-derivatives which inhibit Na⁺-glucose cotransporter 2 (SGLT2) and are thereby useful as preventive or therapeutic agents for diabetes such as insulin-dependent diabetes (Type 1 diabetes), non-insulin-dependent diabetes (Type 2 diabetes), diabetic complications and diseases caused by hyperglycemia such as obesity, prodrugs thereof and salts thereof.

### BACKGROUND ART

In late years, the number of diabetic patients has been increasing due to westernization of dietary habits, chronic lack of exercise and so on. Decrease in insulin secretion and insulin sensitivity is observed in diabetic patients, which is caused by chronic hyperglycemia, further causes elevation of blood sugar level and leads to aggravation of symptoms. Biguanide drugs, sulphonylurea drugs, glycosidase inhibitors, insulin sensitizers, etc., have been used as therapeutic drugs for diabetes. However, side effects such as lactic acidosis as for biguanide drugs, hypoglycemia as for sulphonylurea drugs, diarrhea as for glycosidase inhibitors have been reported, and now the development of therapeutic drugs for diabetes according to new action mechanism different from these drugs is eagerly demanded.

It has been reported that Phloridzin, which is a naturally-occurring glucose derivative, inhibits sodium dependent glucose cotransporter 2 (SGLT2) occurring in S1 site of the renal proximal tubule, and thereby inhibits reabsorption of excessive glucose in the kidney, promotes glucose excretion, and exhibits hypoglycemic action (refer to Non-Patent Document 1). Thereafter, up to the present, studies on the therapeutic drugs for diabetes based on SGLT2 inhibition has been extensively performed.

Compounds usable as inhibitors of SGLT2 are reported, for example, in JP 2000-080041 A (Patent Document 1), WO01/068660 (Patent Document 2), WO04/007517 (Patent Document 3), etc. However, Phloridzin and the compounds described in the above-mentioned patent applications are considered to be problematic in that when they are orally administered, they are readily hydrolyzed by glycosidase and the like present in the small intestine and the pharmacological effect thereof immediately disappears. In addition, as for Phloridzin, there has been reported that phloretin, which is the aglycone moiety thereof, strongly inhibits a sugar transporter of the facilitated diffusion type and causes bad influences such that the glucose concentration in the brain decreases when phloretin is administered intravenously to a rat (for example, refer to Non-Patent Document 2).

Therefore, attempts to convert the compounds to prodrugs have been made for the purpose of preventing such decomposition and improving absorption efficiency. However, although it is desirable that the administered prodrugs are suitably metabolized and changed into an active compound in or in the vicinity of the target organ, there are so various metabolic enzymes in the living body and there are so many differences among individuals that stable action cannot be developed in many cases. Attempts to convert the glycoside bond of the compound to a carbon-carbon bond (refer to Patent Documents 4 to 21) or to convert glucose moiety to 5-thioglucose (refer to Patent Documents 22 to 26) have been also made, but further improvement is demanded in the properties as pharmaceutical agents including activity and metabolic stability.
[Patent Document 1] JP 2000-080041 A
[Patent Document 2] International Publication WO01/068660
[Patent Document 3] International Publication WO04/007517
[Patent Document 4] US Patent Application Pub. No. 2001/041,674
[Patent Document 5] US Patent Application Pub. No. 2002/137,903
[Patent Document 6] International Publication WO01/027128
[Patent Document 7] International Publication WO02/083066
[Patent Document 8] International Publication WO04/013118
[Patent Document 9] International Publication WO03/099836
[Patent Document 10]International Publication WO04/080990
[Patent Document 11]US Patent Application Pub. No. 2005/0,209,166
[Patent Document 12]International Publication WO05/085237
[Patent Document 13]International Publication WO05/085265
[Patent Document 14]International Publication WO05/012318
[Patent Document 15]International Publication WO05/012326
[Patent Document 16]US Patent Application Pub. No. 2006/0,063,722
[Patent Document 17]US Patent Application Pub. No. 2006/0,035,841
[Patent Document 18]US Patent Application Pub. No. 2006/0,074,031
[Patent Document 19]International Publication WO06/002912
[Patent Document 20] International Publication WO06/008038
[Patent Document 21]International Publication WO06/010557
[Patent Document 22]International Publication WO04/014931
[Patent Document 23]International Publication WO04/089967
[Patent Document 24] International Publication WO06/073197
[Patent Document 25]JP 2005-247834 A
[Patent Document 26]JP 2006-117651 A
[Non-Patent Document 1] J. Clin. Invest., Vol. 93, page 397, 1994
[Non-Patent Document 2] Stroke, Vol. 14, page 388, 1983

### DISCLOSURE OF THE INVENTION

### PROBLEMS TO BE SOLVED BY THE INVENTION

An object of the present invention is to provide a thioglucose spiro-derivative having preferable properties as pharmaceuticals. Particularly, an object of the present invention is to provide a thioglucose spiro-derivative having hypoglycemic action and preferable properties such as sustained efficacy, metabolic stability or safety. Another object of the present invention is to provide a pharmaceutical composition used for prevention or treatment of diabetes such as insulin-dependent diabetes (Type 1 diabetes) and non-insulin-dependent diabetes (Type 2 diabetes), diabetic complications and diseases caused by hyperglycemia such as obesity.

### MEANS FOR SOLVING THE PROBLEMS

The present inventors have filed a patent application (WO2006/080421) for spiroketal derivatives represented by Formula (I): 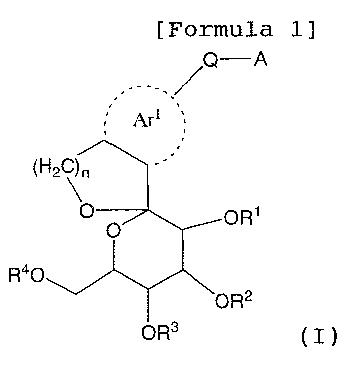 The present inventors have conducted intensive studies so as to achieve the above-mentioned objects and consequently have found that thioglucose spiro-derivatives represented by Formula (II) have excellent inhibitory activity against SGLT2 and thus completed the present invention.

Namely, according to one aspect of the present invention, there is provided a compound represented by Formula (II): 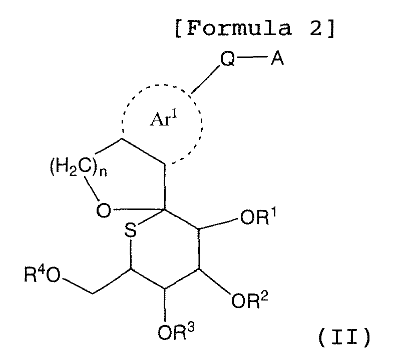 wherein R¹, R², R³, and R⁴ are each independently selected from a hydrogen atom, a C₁-C₆ alkyl group which may be substituted with one or more Ra, a C₇-C₁₄ aralkyl group which may be substituted with one or more Rb and -C(=O)Rx;
Rx is a C₁-C₆ alkyl group which may be substituted with one or more Ra, an aryl group which may be substituted with one or more Rb, a heteroaryl group which may be substituted with one or more Rb, a C₁-C₆ alkoxy group which may be substituted with one or more Ra or -NReRf;
Ar¹ is an aromatic carbocyclic ring which may be substituted with one or more Rb, or an aromatic heterocyclic ring which may be substituted with one or more Rb, in which the aromatic carbocyclic ring and the aromatic heterocyclic ring may form a condensed ring;
Q is -(CH₂)ₘ-(L)ₚ- or -(L)ₚ-(CH₂)ₘ-;
m is an integer selected from 0 to 2, n is an integer selected from 1 and 2, and p is an integer selected from 0 and 1;
L is -O-, -S- or -NR⁵-,
R⁵ is selected from a hydrogen atom, a C₁-C₆ alkyl group which may be substituted with one or more Ra, and -C(=O)Rx;
A is an aryl group which may be substituted with one or more Rb or a heteroaryl group which may be substituted with one or more Rb, wherein the aryl group or the heteroaryl group may form a condensed ring together with the aromatic carbocyclic ring or the aromatic heterocyclic ring;
Ra is each independently selected from a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRgRh, a C₁-C₆ alkoxycarbonyl group which may be substituted with one or more Rc and a C₁-C₆ alkylcarbonyl group which may be substituted with one or more Rc;
Rb is each independently selected from a C₁-C₆ alkyl group which may be substituted with one or more Rc, a C₃-C₈ cycloalkyl group which may be substituted with one or more Rc, a C₂-C₆ alkenyl group which may be substituted with one or more Rc, a C₂-C₆ alkynyl group which may be substituted with one or more Rc, a C₇-C₁₄ aralkyl group which may be substituted with one or more Rd, a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRiRj, a C₁-C₆ alkylcarbonyl group which may be substituted with one or more Rc, a C₁-C₆ alkoxycarbonyl group which may be substituted with one or more Rc, a C₁-C₃ alkylenedioxy group, a heterocyclyl group and a heterocyclyloxy group;
Rc is each independently selected from a halogen atom, a hydroxy group, a cyano group, a nitro group, a carboxy group, a C₁-C₆ alkoxy group, an aryl group which may be substituted with one or more Rd, an aryloxy group which may be substituted with one or more Rd, a heteroaryl group which may be substituted with one or more Rd, a heteroaryloxy group which may be substituted with one or more Rd, an amino group, a C₁-C₆ alkylamino group and a di(C₁-C₆ alkyl)amino group;
Rd is each independently selected from a C₁-C₆ alkyl group which may be substituted with one or more halogen atoms, a C₇-C₁₄ aralkyl group, a halogen atom, a hydroxy group, a cyano group, a nitro group, an amino group, a C₁-C₆ alkylamino group and a di(C₁-C₆ alkyl)amino group;
Re is a hydrogen atom, a C₁-C₆ alkyl group which may be substituted with one or more Rc, an aryl group which may be substituted with one or more Rd, or a heteroaryl group which may be substituted with one or more Rd;
Rf, Rg and Ri are each independently selected from a hydrogen atom, and a C₁-C₆ alkyl group which may be substituted with one or more Rc;
Rh and Rj are each independently selected from a hydrogen atom, a C₁-C₆ alkyl group which may be substituted with one or more Rc, a C₁-C₆ alkylcarbonyl group which may be substituted with one or more Rc, an aryl group which may be substituted with one or more Rd, a heteroaryl group which may be substituted with one or more Rd, a carbamoyl group, a C₁-C₆ alkoxycarbonyl group which may be substituted with one or more Rc, and a C₁-C₆ alkylsulfonyl group which may be substituted with one or more Rc; or
Re and Rf, Rg and Rh, and Ri and Rj together with the nitrogen atom to which they are attached may form a 4- to 7-membered heterocyclic ring;
or a prodrug thereof or a pharmaceutically acceptable salt thereof.

According to another aspect of the invention, there is provided a compound represented by Formula (IIa):

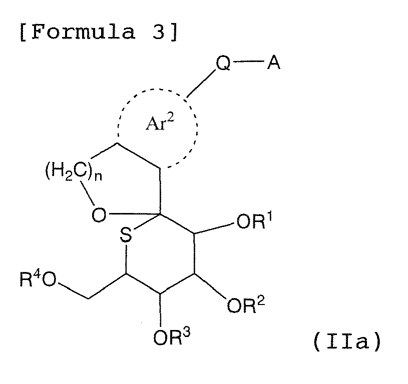

wherein R¹, R², R³, R⁴, n, Q, and A are as defined above; Ar² is a monocyclic aromatic carbocyclic ring which may be substituted with one or more Rb or a monocyclic aromatic heterocyclic ring which may be substituted with one or more Rb;
or a prodrug thereof or a pharmaceutically acceptable salt thereof.

According to another aspect of the invention, there is provided a compound represented by Formula (IIb): 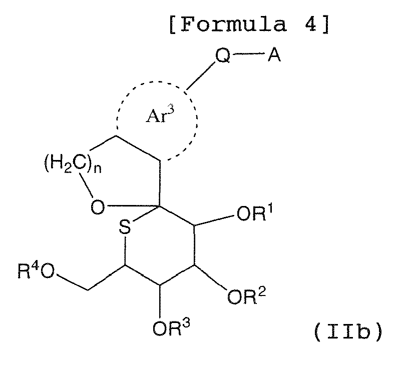 wherein Ar³ is selected from quinoline, isoquinoline, 4H-quinolidine, phthalazine, naphthyridine, quinoxaline, quinazoline, cinnoline, pteridine, indole, indoline, benzothiophene, 1-methyl-1H-indole, benzofuran, naphthalene, benzisothiazole, benzisoxazole, indazole, benzimidazole, benzotriazole and indene;
R¹, R², R³, R⁴, Q, A and n are as defined above or a prodrug thereof or a pharmaceutically acceptable salt thereof.

According to a further aspect of the invention, there is provided a compound represented by Formula (IIc): 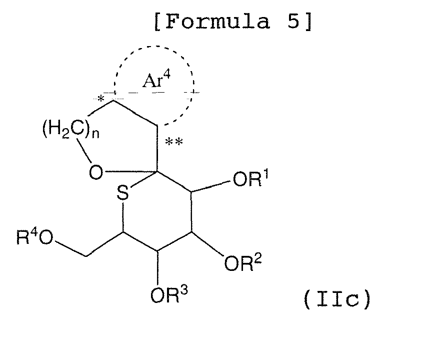 wherein ring Ar⁴ is selected from the groups represented by Formulae (a) to (m):

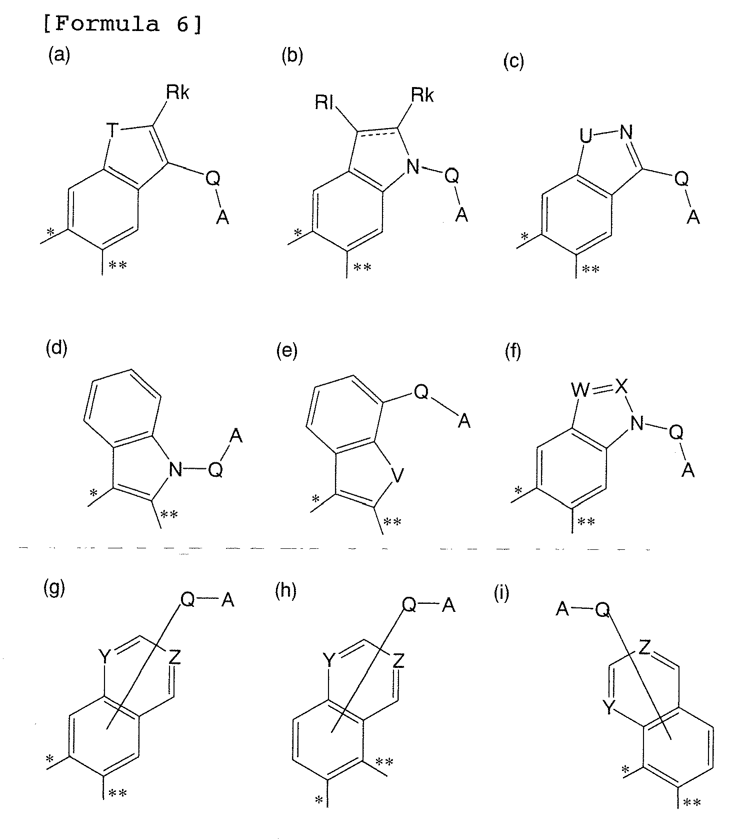

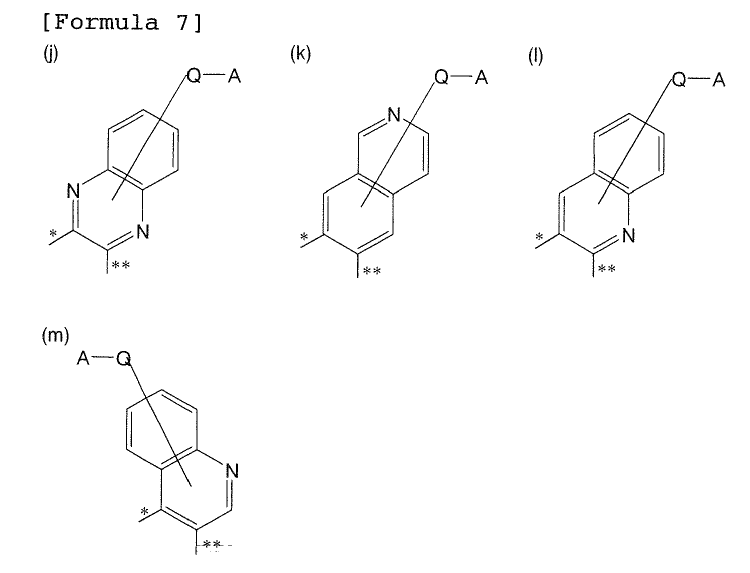

wherein T is an oxygen atom, a sulfur atom, CH₂ or N-Rm;
Rk and Rl are independently selected from a hydrogen atom, a halogen atom, and a C₁-C₆ alkyl group;
U is N-Rm, an oxygen atom or a sulfur atom;
V is a sulfur atom, an oxygen atom or N-Rm;
Rm is a hydrogen atom or C₁-C₆ alkyl group;
W and X are independently selected from a nitrogen atom and carbon atom, with the proviso that at least one of W and X is a nitrogen atom;
Y and Z are independently selected from a nitrogen atom and a carbon atom, with the proviso that when Y or Z is an nitrogen atom, the nitrogen atom does not have the substituent -Q-A; and

[Formula 8] -̅-̅-̅-̅-̅-̅

represents a single bond or a double bond;
Q and A are as defined above;
and * and ** respectively represent a bonding site;
or a prodrug thereof or a pharmaceutically acceptable salt thereof.

According to a further aspect of the invention, there is provided a compound represented by Formula (III): 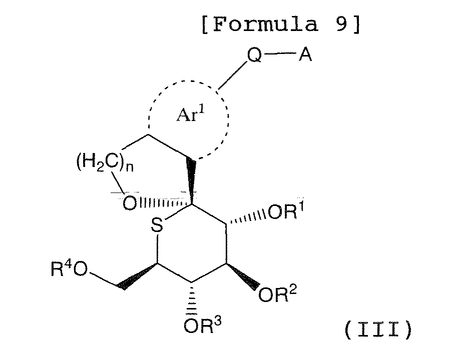 wherein Ar¹, Q, n, R¹, R², R³, R⁴, and A are as defined above;
or a prodrug thereof or a pharmaceutically acceptable salt thereof.

According to a further aspect of the invention, there is provided a compound represented by Formula (IV):

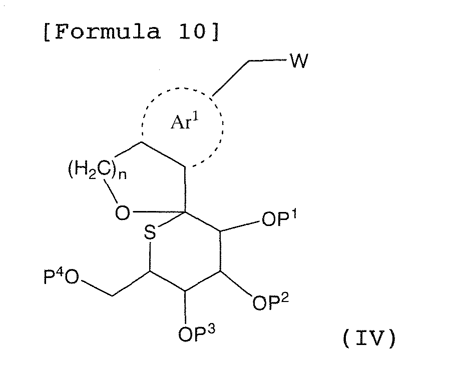

wherein n is an integer selected from 1 and 2;
Ar¹ is an aromatic carbocyclic ring or an aromatic heterocyclic ring, which may be substituted with one or more Rb and may form a condensed ring;
W is -O-Z or a halogen atom;
Z is a hydrogen atom, an acyl group or a benzyl group;
P¹, P², P³ and P⁴ are independently selected from a hydrogen atom, an acyl group and a benzyl group;
Rb is as defined above.
The compound is useful as a synthetic intermediate of the compound of the present invention, for example, represented by Formula (II). An acyl group is a generic term for a group represented by RCO-, and includes a formyl group; a C₁-C₆ alkylcarbonyl group such as an acetyl group and a propionyl group; an arylcarbonyl group such as benzoyl group and naphthoyl group; a C₇-C₁₄ aralkylcarbonyl group such as a benzylcarbonyl group.

According to still another aspect of the present invention, there is provided a pharamaceutical composition comprising a compound represented by Formula (II), (IIa), (IIb), (IIc) or (III) defined above, or a prodrug thereof, or a pharmaceutically acceptable salt thereof.

According to still another aspect of the present invention, there is provided a pharamaceutical composition comprising a compound represented by Formula (II), (IIa), (IIb), (IIc) or (III) defined above, or a prodrug thereof, or a pharmaceutically acceptable salt thereof, which is used as a Na⁺-glucose cotransporter inhibitor.

According to still another aspect of the present invention, there is provided a pharmaceutical composition comprising a compound represented by Formula (II), (IIa), (IIb), (IIc) or (III) defined above, or a prodrug thereof or a pharmaceutically acceptable salt thereof, which is used for prevention or treatment of diabetes (for example, insulin-dependent diabetes (Type 1 diabetes) or non-insulin-dependent diabetes (Type 2 diabetes)), diabetic complications caused hyperglycemia, or obesity.

According to still another aspect of the present invention, there is provided a method for preventing or treating diabetes (for example, insulin-dependent diabetes (Type 1 diabetes) or non-insulin-dependent diabetes (Type 2 diabetes)), hyperglycemia, or diabetic complications caused thereby, or obesity, comprising administering to a patient of an effective therapeutic dose of the compound represented by Formula (II), (IIa), (IIb), (IIc) or (III), or a prodrug thereof or a pharmaceutically acceptable salt thereof.

In the above Formulae (II), (IIa), (IIb), (IIc) and (III), groups defined as R¹, R², R³ and R⁴ include, for example, a hydrogen atom, a C₁-C₆ alkyl group, a C₁-C₆ alkoxy-C₁-C₆ alkyl group, a C₇-C₁₄ aralkyl group, a C₁-C₆ alkylcarbonyl group, a C₇-C₁₄ aralkylcarbonyl group, a C₁-C₆ alkoxycarbonyl group, and a C₇-C₁₄ aralkyloxycarbonyl group. These groups may be substituted with one or more substituents which are each independently selected from a halogen atom, a hydroxy group, a C₁-C₆ alkoxy group, a C₁-C₆ alkylcarbonyl group, a carboxy group, an amino group and a substituted amino group. A hydrogen atom is particularly preferred as R¹, R², R³ and R⁴.

In the above Formulae (II) and (III), Ar¹ may be substituted with the same or different 1 to 4 substituents, for example, which are each independently selected from a halogen atom; a hydroxy group; a C₁-C₆ alkyl group, a C₃-C₈ cycloalkyl group, a C₁-C₆ alkoxy group and a C₁-C₆ alkylthio group (these 4 groups may be substituted with 1 to 4 substituents selected from a halogen atom, a hydroxy group and an amino group); a methylenedioxy group; a cyano group; a C₁-C₆ alkylsulfonyl group; a C₁-C₆ alkylsulfonylamino group; a nitro group; a carboxy group; a substituted amino group; and a 4- to 6-membered heterocyclyl group.

Among the groups defined as Ar¹, the aromatic carbocyclic ring preferably refers to an aromatic monocyclic or bicyclic carbocyclic ring containing 5 to 10 carbon atoms, including a benzene ring, a naphthalene ring, indene ring and the like. The aromatic heterocyclic ring preferably refers to a 5- to 10-membered monocyclic or bicyclic aromatic heterocyclic group, which contains one or more heteroatoms selected from an oxygen atom, a nitrogen atom, sulfur atom and the like, including a pyrrole ring, a thiophene ring, a furan ring, a pyridine ring, a thiazole ring, an isothiazole ring, a pyrazole ring, an oxazole ring, an isoxazole ring, an imidazole ring, a triazole ring, a pyrimidine ring, a uridine ring, a pyrazine ring, a pyridazine ring, a quinoline ring, an isoquinoline ring, a 4H-quinolidine ring, a phthalazine ring, a naphthyridine ring, a quinoxaline ring, a quinazoline ring, a cinnoline ring, a pteridine ring, an indole ring, an indoline ring, a benzothiophene ring, a 1-methyl-1H-indole ring, a benzofuran ring, a benzisothiazole ring, a benzisoxazole ring, an indazole ring, a benzimidazole ring, a benzotriazole ring and the like. Among them, Ar¹ is preferably a benzene ring, a naphthalene ring, a thiophene ring, a furan ring, a benzothiophene ring, a benzofuran ring, an indole ring, an indoline ring, a benzisothiazole ring, a benzisoxazole ring, an indazole ring, a quinoline ring, an isoquinoline ring, and a quinoxaline ring, and more preferably a benzene ring, a thiophene ring, a benzothiophene ring, and an indole ring.

In the above Formulae (II), (IIa), (IIb), (IIc) and (III), A may be substituted with the same or different 1 to 3 substituents, for example, which are each independently selected from, for example, a halogen atom; a hydroxy group; a C₁-C₆ alkyl group, a C₃-C₈ cycloalkyl group, a C₁-C₆ alkyloxy group and a C₁-C₆ alkylthio group (these 4 groups may be substituted with 1 to 4 substituents which are each independently selected from a halogen atom or a hydroxy group or an amino group); a methylenedioxy group; a cyano group; a C₁-C₆ alkylsulfonyl group; a C₁-C₆ alkylsulfonylamino group; a nitro group; a carboxy group; a substituted amino group; a 5- or 6-membered heteroaryl group; and a 4- to 6-membered heterocyclyl group.

Groups defined as A include, for example, a phenyl group, a naphthyl group, an azulenyl group, a pyrrolyl group, an indolyl group, a pyridyl group, a quinolinyl group, an isoquinolinyl group, a thienyl group, a benzothienyl group, a furyl group, a benzofuranyl group, a thiazolyl group, a benzothiazolyl group, an isothiazolyl group, a benzoisothiazolyl group, a pyrazolyl group, an indazolyl group, an oxazolyl group, a benzoxazolyl group, an isoxazolyl group, a benzoisoxazolyl group, an imidazolyl group, a benzoimidazolyl group, a triazolyl group, a benzotriazolyl group, a pyrimidinyl group, a uridyl group, a pyrazinyl group, a pyridazinyl group, an imidazopyridyl group, a triazolopyridyl group and a pyrrolopyridyl group. Preferred are a phenyl group, a naphthyl group, a thienyl group, a benzothienyl group, a furyl group and a benzofuranyl group, and more preferred are a phenyl group, a thienyl group and a benzothienyl group.

As used herein, the term "C₁-C₆ alkyl group" refers to a linear or branched alkyl group containing 1 to 6 carbon atoms. Examples include methyl, ethyl, n-propyl, i-propyl, n-butyl, s-butyl, i-butyl, t-butyl, n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl and 2-ethylbutyl. Preferred C₁-C₆ alkyl groups are, for example, linear or branched alkyl groups containing 1 to 3 carbon atoms, and methyl and ethyl are particularly preferred.

As used herein, the term "C₂-C₆ alkenyl group" refers to a linear or branched alkenyl group containing 2 to 6 carbon atoms. Examples include ethenyl (vinyl), 1-propenyl, 2-propenyl (allyl), propen-2-yl and 3-butenyl (homoallyl).

As used herein, the term "C₂-C₆ alkynyl group" refers to a linear or branched alkynyl group containing 2 to 6 carbon atoms. Examples include ethynyl, 1-propynyl, 2-propynyl, 1-butynyl, 2-butynyl and 3-butynyl.

As used herein, the term "C₃-C₈ cycloalkyl group" refers to a cyclic alkyl group containing 3 to 8 carbon atoms. Examples include cyclopropyl, cyclobutyl, cyclopentyl, cyclohexyl, cycloheptyl and cyclooctyl.

As used herein, the term "C₁-C₆ alkoxy group" refers to an alkyloxy group whose alkyl moiety is a linear or branched alkyl group containing 1 to 6 carbon atoms. Examples include methoxy, ethoxy, n-propoxy, i-propoxy, n-butoxy, s-butoxy, i-butoxy, t-butoxy, n-pentoxy, 3-methylbutoxy, 2-methylbutoxy, 1-methylbutoxy, 1-ethylpropoxy, n-hexyloxy, 4-methylpentoxy, 3-methylpentoxy, 2-methylpentoxy, 1-methylpentoxy and 3-ethylbutoxy.

As used herein, the term "C₇-C₁₄ aralkyl group" refers to an arylalkyl group containing 7 to 14 carbon atoms, which contains an aryl group. Examples include benzyl, 1-phenethyl, 2-phenethyl, 1-naphthylmethyl and 2-naphthylmethyl.

As used herein, the term "C₇-C₁₄ aralkyloxy group" refers to an arylalkyloxy group containing 7 to 14 carbon atoms, which contains the already-defined aralkyl group. Examples include benzyloxy, 1-phenethyloxy, 2-phenethyloxy, 1-naphthylmethyloxy and 2-naphthylmethyloxy.

As used herein, the term "aryl group" refers to an aryl group having an aromatic hydrocarbon ring containing 6 to 10 carbon atoms. Examples include phenyl, 1-naphthyl and 2-naphthyl.

As used herein, the term "heteroaryl group" refers to a 5- to 10-membered aromatic heterocyclic group containing one or more heteroatoms independently selected from an oxygen atom, a nitrogen atom and a sulfur atom. Examples include furyl, thienyl, pyrrolyl, imidazolyl, pyrazolyl, oxazolyl, isoxazolyl, thiazolyl, isothiazolyl, oxadiazolyl, thiadiazolyl, triazolyl, tetrazolyl, pyridinyl, pyrimidinyl, pyrazinyl, pyridazinyl, indolyl, quinolinyl and isoquinolinyl. Preferred heteroaryl groups are 5- to 6-membered cyclic heteroaryl groups such as a furyl group, a pyrazolyl group, a thienyl group and a pyridinyl group. A thienyl group is particularly preferred.

As used herein, the term "aryloxy group" refers to an aryloxy group whose aryl moiety is the already-defined aromatic hydrocarbon group containing 6 to 10 carbon atoms. Examples include phenoxy, 1-naphthoxy and 2-naphthoxy.

As used herein, the term "heteroaryloxy group" refers to a heteroaryloxy group whose heteroaryl moiety is the already-defined 5- to 10-membered aromatic heterocyclic group containing one or more heteroatoms selected from an oxygen atom, a nitrogen atom and a sulfur atom. Examples include furyloxy, thienyloxy, pyrrolyloxy, imidazolyloxy, pyrazolyloxy, oxazolyloxy, isoxazolyloxy, thiazolyloxy, isothiazolyloxy, oxadiazolyloxy, thiadiazolyloxy, triazolyloxy, tetrazolyloxy, pyridinyloxy, pyrimidinyloxy, pyrazinyloxy, pyridazinyloxy, indolyloxy, quinolinyloxy and isoquinolinyloxy. Preferred heteroaryloxy groups are 5- to 6-membered heteroaryloxy groups.

As used herein, the term "C₁-C₆ alkylamino group" refers to an alkylamino group whose alkyl moiety is a linear or branched alkyl group containing 1 to 6 carbon atoms. Examples include methylamino, ethylamino, n-propylamino, I-propylamino, n-butylamino, s-butylamino, i-butylamino, t-butylamino, n-pentylamino, 3-methylbutylamino, 2-methylbutylamino, 1-methylbutylamino, 1-ethylpropylamino, n-hexylamino, 4-methylpentylamino, 3-methylpentylamino, 2-methylpentylamino, 1-methylpentylamino, 3-ethylbutylamino and 2-ethylbutylamino.

As used herein, the term "di-(C₁-C₆ alkyl)amino group" refers to a dialkylamino group whose two alkyl moieties are the same or different linear or branched alkyl groups containing 1 to 6 carbon atoms. Examples of the "di-(C₁-C₆ alkyl)amino group" include dimethylamino, diethylamino, di-n-propylamino, di-i-propylamino, di-n-butylamino, methyl-n-butylamino, methyl-s-butylamino, methyl-i-butylamino, methyl-t-butylamino, ethyl-n-butylamino, ethyl-s-butylamino, ethyl-i-butylamino and ethyl-t-butylamino.

As used herein, the term "C₁-C₆ alkylthio group" refers to an alkylthio group whose alkyl moiety is a linear or branched alkyl group containing 1 to 6 carbon atoms. Examples include methylthio, ethylthio, n-propylthio, i-propylthio, n-butylthio, s-butylthio, i-butylthio, t-butylthio, n-pentylthio, 3-methylbutylthio, 2-methylbutylthio, 1-methylbutylthio, 1-ethylpropylthio, n-hexylthio, 4-methylpentylthio, 3-methylpentylthio, 2-methylpentylthio, 1-methylpentylthio, 3-ethylbutylthio and 2-ethylbutylthio.

As used herein, the term "C₁-C₆ alkylsulfinyl group" refers to an alkylsulfinyl group (-SO-R) whose alkyl moiety is a linear or branched alkyl group containing 1 to 6 carbon atoms. Examples include methylsulfinyl, ethylsulfinyl, n-propylsulfinyl, i-propylsulfinyl, n-butylsulfinyl, s-butylsulfinyl, i-butylsulfinyl, t-butylsulfinyl, n-pentylsulfinyl, 3-methylbutylsulfinyl, 2-methylbutylsulfinyl, 1-methylbutylsulfinyl, 1-ethylpropylsulfinyl, n-hexylsulfinyl, 4-methylpentylsulfinyl, 3-methylpentylsulfinyl, 2-methylpentylsulfinyl, 1-methylpentylsulfinyl, 3-ethylbutylsulfinyl and 2-ethylbutylsulfinyl.

As used herein, the term "C₁-C₆ alkylsulfonyl group" refers to an alkylsulfonyl group whose alkyl moiety is a linear or branched alkyl group containing 1 to 6 carbon atoms. Examples include methylsulfonyl, ethylsulfonyl, n-propylsulfonyl, i-propylsulfonyl, n-butylsulfonyl, s-butylsulfonyl, i-butylsulfonyl, t-butylsulfonyl, n-pentylsulfonyl, 3-methylbutylsulfonyl, 2-methylbutylsulfonyl, 1-methylbutylsulfonyl, 1-ethylpropylsulfonyl, n-hexylsulfonyl, 4-methylpentylsulfonyl, 3-methylpentylsulfonyl, 2-methylpentylsulfonyl, 1-methylpentylsulfonyl, 3-ethylbutylsulfonyl and 2-ethylbutylsulfonyl.

As used herein, "-C(=O)-Rx" encompasses a C₁-C₆ alkylcarbonyl group, a C₇-C₁₄ aralkylcarbonyl group, a C₁-C₆ alkoxycarbonyl group, a C₇-C₁₄ aralkyloxycarbonyl group and the like.

As used herein, the term "C₁-C₆ alkylcarbonyl group" refers to a group represented by the formula -C(=O)-(C₁-C₆ alkyl). Examples of a C₁-C₆ alkylcarbonyl group include an acetyl group, a propionyl group, a butyryl group, an isobutyryl group and a pivaloyl group. Preferred C₁-C₆ alkylcarbonyl groups include an acetyl group.

As used herein, the term "C₇-C₁₄ aralkylcarbonyl group" includes a benzylcarbonyl group and a naphthylmethylcarbonyl group. Preferred C₇-C₁₄ aralkylcarbonyl groups include a benzylcarbonyl group.

As used herein, the term "C₁-C₆ alkoxycarbonyl group" refers to a group represented by the formula -C(=O)-O-(C₁-C₆ alkyl). Examples include a methoxycarbonyl group and an ethoxycarbonyl group. Preferred C₁-C₆ alkoxycarbonyl groups include a methoxycarbonyl group.

As used herein, the term "C₇-C₁₄ aralkyloxycarbonyl group" includes a benzyloxycarbonyl group and a naphthylmethyloxycarbonyl group. Preferred C₇-C₁₄ aralkyloxycarbonyl groups include a benzyloxycarbonyl group.

As used herein, the term "C₁-C₆ alkoxy-C₁-C₆ alkyl group" refers to a group represented by the formula -(C₁-C₆ alkyl)-O-(C₁-C₆ alkyl). Examples include a methoxymethyl group, an ethoxymethyl group and a 1-ethyoxymethyl group. Preferred C₁-C₆ alkoxy-C₁-C₆ alkyl groups include methoxymethyl.

As used herein, the term "halogen atom" encompasses a fluorine atom, a chlorine atom, a bromine atom, an iodine atom and the like.

As used herein, the term "4- to 7-membered heterocyclic ring" refers to a heterocyclic ring which may be completely saturated or partially or completely unsaturated and which contains one nitrogen atom and may further contain one or more heteroatoms independently selected from an oxygen atom, a nitrogen atom and a sulfur atom. Examples include azetidine, pyrrolidine, piperidine and morpholine. Piperidine is particularly preferred.

As used herein, the term "substituted amino group" encompasses -NReRf, wherein Re represents a hydrogen atom, a C₁-C₆ alkyl group, a C₁-C₆ alkylcarbonyl group, carbamoyl or a C₁-C₆ alkoxycarbonyl group, and Rf represents a hydrogen atom or a C₁-C₆ alkyl group, or Re and Rf may form a 4- to 7-membered heterocyclic ring together with the nitrogen atom to which they are attached, and the like; -NRgRh, wherein Rg represents a hydrogen atom, or a C₁-C₆ alkyl group which may be substituted with one or more Rc, and Rh represents a hydrogen atom, a C₁-C₆ alkyl group which may be substituted with one or more Rc, a C₁-C₆ alkylcarbonyl group which may be substituted with one or more Rc, an aryl group which may be substituted with one or more Rd, a heteroaryl group which may be substituted with one or more Rd, a carbamoyl group, a C₁-C₆ alkoxycarbonyl group which may be substituted with one or more Rc, a C₁-C₆ alkylsulfonyl group which may be substituted with one or more Rc, or Rg and Rh together with the nitrogen atom to which they are attached may form a 4- to 7-membered heterocyclic ring; and -NRiRj, wherein Ri represents a hydrogen atom, or a C₁-C₆ alkyl group which may be substituted with one or more Rc, and Rj represents a hydrogen atom, a C₁-C₆ alkyl group which may be substituted with one or more Rc, a C₁-C₆ alkylcarbonyl group which may be substituted with one or more Rc, an aryl group which may be substituted with one or more Rd, a heteroaryl group which may be substituted with one or more Rd, a carbamoyl group, a C₁-C₆ alkoxycarbonyl group which may be substituted with one or more Rc, a C₁-C₆ alkylsulfonyl group which may be substituted with one or more Rc, or Ri and Rj together with the nitrogen atom to which they are attached may form a 4- to 7-membered heterocyclic ring. Preferred examples include a methylamino group, a dimethylamino group, an ethylamino group, a diethylamino group, an ethylmethylamino group, a methoxycarbonylamino group, an ethoxycarbonylamino group, an acetamide group, a benzamide group, a piperidino group, a pyrrolidino group, a morpholino group, a piperadino group, an anilino group, a 2-pyridylamino group, a 3-pyridylamino group, and a 4-pyridylamino group.

As used herein, the term "C₁-C₃ alkylenedioxy group" refers to a divalent group represented by the formula -O-(C₁-C₃ alkylene)-O-. Examples include a methylenedioxy group, an ethylenedioxy group and a dimethylmethylenedioxy group.

As used herein, the term "heterocyclyl group" refers to a 4- to 7-membered heterocyclic group which may be completely saturated or partially or completely unsaturated and which contains one or more heteroatoms independently selected from an oxygen atom, a nitrogen atom and a sulfur atom. Examples include azetidinyl, pyrrolidinyl, piperidinyl, piperazinyl, pyrrolyl, imidazolyl, imidazolinyl, pyrazolyl, pyrazolinyl, oxazolinyl, morpholinyl, thiomorpholinyl, pyridinyl, pyrazinyl, pyrimidinyl, pyridazinyl, hexamethyleneimino, furyl, tetrahydrofuryl, thienyl, tetrahydrothienyl, dioxolanyl, oxathiolanyl and dioxanyl. Such a heterocyclic group may be substituted at any substitutable position on its carbon or nitrogen atom(s).

As used herein, the term "heterocyclyloxy group" refers to an oxy group attached to a 4- to 7-membered heterocyclic ring which may be completely saturated or partially or completely unsaturated and which contains one or more heteroatoms independently selected from an oxygen atom, a nitrogen atom and a sulfur atom. Examples include azetidinyloxy, pyrrolidinyloxy, piperidinyloxy, piperazinyloxy, pyrrolyloxy, imidazolyloxy, imidazolinyloxy, pyrazolyloxy, pyrazolinyloxy, oxazolinyloxy, morpholinyloxy, thiomorpholinyloxy, pyridinyloxy, pyrazinyloxy, pyrimidinyloxy, pyridazinyloxy, hexamethyleneiminoxy, furyloxy, tetrahydrofuryloxy, thienyloxy, tetrahydrothienyloxy, dioxolanyloxy, oxathiolanyloxy and dioxanyloxy. Such a heterocyclic group may be substituted at any substitutable position on its carbon or nitrogen atom(s).

The range of the present invention includes mixtures and isolated products of various stereoisomers such as optical isomers and tautomers of the compound defined as the present invention.

The compounds of the present invention may form acid addition salts. The compounds may form salts with a base depending on the kind of the substituent. Such salts specifically include acid addition salts with mineral acids such as hydrochloric acid, hydrobromic acid, hydroiodic acid, sulfuric acid, nitric acid, phosphoric acid; organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid; and acidic amino acids such as aspartic acid and glutamic acid. The salts formed with bases include salts with inorganic bases such as sodium, potassium, magnesium, calcium and aluminum; salts with organic bases such as methylamine, ethylamine, ethanolamine; salts with basic amino acids such as lysin, ornithine and ammonium salts.

Furthermore, hydrates, pharmaceutically acceptable various solvates and crystal polymorphs are included in the compounds of the present invention.

It should be understood that the compound of the present invention is not limited to compounds described in Examples indicated hereinafter. The present invention encompasses all thioglucose spiro-derivatives represented by Formula (II) and pharmaceutically acceptable salts thereof.

The present invention also includes so-called prodrugs which are compounds metabolized in the living body and converted into the compounds of the above Formula (IV) and pharmaceutically acceptable salts thereof. Groups to form prodrugs of the compounds of the present invention include groups described in Prog. Med. Vol. 5, pages 2157-2161 (1985) and in "Iyakuhin no Kaihatsu" ("Development of medicinal drugs"), Vol. 7 (molecular design), pages 163-198, Hirokawa Shoten published in 1990.

The compounds of the present invention can be produced by applying various kinds of a publicly known synthesis method in accordance with characteristics based on the basic structure or the kind of the substituents. Depending on the kind of functional groups, it may be preferable in terms of production technology to protect a functional group with a suitable protecting group at the stage of raw materials or intermediates, and desired compounds can be obtained by removing the protecting group in the later steps. Examples of the functional groups needed to be protected in the production process include a hydroxy group and a carboxy group and examples of the protecting groups thereof include the protecting groups described in Greene and Wuts, "Protective Groups in Organic Synthesis", second edition. The protecting group to be used and reaction conditions at the time of introducing and removing the protecting group can be appropriately selected based on the publicly known technology such as those described in the above-mentioned documents.

The compounds of the present invention have inhibitory activity on sodium dependent glucose cotransporter 2 (SGLT2) involved in glucose reabsorption in the kidney (J. Clin. Invest., Vol. 93, page 397, 1994). Inhibition of SGLT2 suppresses reabsorption of glucose, excretes excessive glucose to outside of the body and thereby leads to therapeutic effect on the diabetes and an effect of improving insulin resistance by correcting hyperglycemia without a burden to pancreatic β cells.

Therefore, according to one aspect of the present invention, there is provided pharmaceutical agents to prevent or treat diseases or conditions which can be improved by inhibiting the activity of SGLT2, for example, diabetes, diabetes-related diseases and diabetic complications.

Here, the "diabetes" includes Type 1 diabetes, Type 2 diabetes, and the other types of diabetes by specific causes. The "diabetes-related diseases" includes, for example, obesity, hyperinsulinemia, abnormality of glucose metabolism, hyperlipidemia, hypercholesterolemia, hypertriglyceridemia, lipidosis, hypertension, congestive heart failure, edema, hyperuricemia and gout.

The "diabetic complications" include both acute and chronic complications. Examples of "acute complications" include hyperglycemia (ketoacidosis, etc.), infectious diseases (infection in the skin, soft tissue, biliary system, respiratory system, urinary tract, etc.) and examples of "chronic complication" include microangiopathy (nephropathy, retinopathy), arteriosclerosis (atherosclerosis, myocardial infarction, cerebral infarction, lower limbs arterial occlusion, etc.), neuropathy (in sensory nerve, motor nerves, autonomous nerve, etc.), foot gangrene. Major diabetic complications include diabetic retinopathy, diabetic nephropathy and diabetic neuropathy.

The compounds of the present invention can be used together with therapeutic drugs for diabetes and diabetic complications, which have different action mechanism other than SGLT2 activity inhibitor, antihyperlipemic drugs, or antihypertensive drugs, etc. Additive effect can be expected by combining the compounds of the present invention with the other drugs as compared with the effect obtained by singly using the respective drugs for the above-mentioned diseases.

Examples of the "therapeutic drug for diabetes and diabetic complications" which can be used together include insulin sensitivity enhancing drugs (PPAR γ agonist, PPAR α/γ agonist, PPAR δ agonist, PPAR α/γ/δ agonist), glycosidase inhibitors, biguanide drugs, insulin secretion enhancers, insulin formulations, glucagon receptor antagonists, insulin receptor kinase enhancers, tripeptidyl peptidase II inhibitors, dipeptidyl peptidase IV inhibitors, protein tyrosine phosphatase-1B inhibitors, glycogen phosphorylase inhibitors, glucose-6-phosphatase inhibitors, gluconeogenesis inhibitors, fructose bisphosphatase inhibitors, pyruvic acid dehydrogenase inhibitors, glucokinase activators, D-chiro-inositol, glycogen synthetase kinase-3 inhibitors, glucagons-like peptide-1, glucagons-like peptide-1 analogues, glucagons-like peptide-1 agonists, amylin, amylin analogues, amylin agonists, glucocorticoid receptor antagonists, 11β-hydroxysteroid dehydrogenase inhibitors, aldose reductase inhibitors, protein kinase C inhibitors, γ-aminobutyric acid receptor antagonists, sodium channel antagonists, transcription factor NF-κB inhibitors, IKKβ inhibitors, lipid peroxidase inhibitors, N-acetylated-α-linked-acid-dipeptidase inhibitors, insulin-like growth factor-I, platelet-derived growth factors (PDGF), platelet-derived growth factor (PDGF) analogues, epidermal growth factors (EGF), nerve growth factors, carnitine derivatives, uridine, 5-hydroxy-1-methylhydantoin, EGB-761, bimoclomol, sulodexide, Y-128 and TAR-428.

The "therapeutic drug for diabetes and diabetic complications" can be exemplified as follows.

Metoformine hydrochloride and fenformine are included as "biguanide drugs".

Among insulin secretion enhancers, examples of sulphonylurea drugs include glyburide (glibenclamide), glipizide, gliclazide, chlorpropamide and examples of non-sulphonylurea drugs include nateglinide, repaglinide and mitiglinide.

The "insulin formulations" include genetic recombinant human insulin and animal origin insulin. They are classified into three types by duration of action, and include immediate-acting type (human insulin, human neutral insulin), intermediate-acting type (insulin-human isofen insulin aqueous suspension, human neutral insulin-human isofen insulin aqueous suspension, human insulin zinc aqueous suspension, insulin zinc aqueous suspension), sustained-acting type (human crystalline insulin zinc suspension).

The "glycosidase inhibitors" include acarbose, voglibose and miglitol.

Among "insulin sensitivity enhancing drugs", PPARγ agonists include troglitazone, pioglitazone, and rosiglitazone; PPARα/γ dual agonists include MK-767 (KRP-297), tesaglitazar, LM4156, LY510929, DRF-4823, and TY-51501; and PPAR δ agonists include GW-501516.

The "tripeptidyl peptidase II inhibitors" include UCL-139.

The "dipeptidyl peptidase IV inhibitors" include NVP-DPP728A, LAF-237, MK-0431, P32/98 and TSL-225.

The "aldose reductase inhibitors" include ascorbyl gamolenate, tolrestat, epalrestat, fidarestat, sorbynyl, ponalrestat, risarestat and zenarestat.

The "γ-aminobutyric acid receptor antagonists" include topiramate.

The "sodium channel antagonists" include mexiletine hydrochloride.

The "transcription factor NF-κB inhibitors" include dexlipotam.

The "lipid peroxidase inhibitors" include tirilazad mesylate.

The "N-acetylated-α-linked-acid-dipeptidase inhibitors" include GPI-5693.

The "carnitine derivatives" include carnitine, levacecarnine hydrochloride.

The "antihyperlipemica drugs and antihypertensive drugs" which can be used together include, for example, hydroxymethylglutaryl coenzyme A reductase inhibitors, fibrate compounds, β₃-adrenaline receptor agonists, AMPK activators, acyl coenzyme A:cholesterol transacylase inhibitors, probcol, thyroid hormone receptor agonists, cholesterol absorption inhibitors, lipase inhibitors, microsome triglyceride transfer protein inhibitors, lipoxygenase inhibitors, carnitine palmitoyltransferase inhibitors, squalene synthetase inhibitors, low-density lipoprotein receptor enhancers, nicotine acid derivatives, bile acid adsorbing drugs, sodium conjugate bile acid transporter inhibitors, cholesterol ester transportation protein inhibitors, angiotensin converting enzyme inhibitors, angiotensin II receptor antagonists, endothelin converting enzyme inhibitors, endothelin receptor antagonists, diuretic drugs, calcium antagonists, vasodilatory hypotensive agents, sympatholytic drugs, central hypotensive agents, α₂-adrenaline receptor agonists, antiplatelets, uric acid generation inhibitors, uric acid excretion enhancers, urine alkalizer, anorectic drugs, ACE inhibitors, adiponectin receptor agonists, GPR40 agonists, GPR40 antagonists.

The therapeutic drugs for hyperlipemia and antihypertensive drugs can be exemplified as follows.

The "hydroxymethylglutaryl coenzyme A reductase inhibitors" include fluvastatin, lovastatin, pravastatin, cerivastatin and pitavastatin.

The "fibrate compounds" include bezafibrate, beclobrate and binifibrate.

The "squalene synthetase inhibitors" include TAK-475, α-phosphonosulphonate derivatives (specification of US Patent No. 5712396).

The "acyl coenzyme A: cholesterol transacylase inhibitors" include CI-1011, NTE-122, FCE-27677, RP-73163, MCC-147 and DPU-129.

The "low-density lipoprotein receptor enhancers" include MD-700 and LY-295427.

The "microsome triglyceride transfer protein inhibitors" (MTP inhibitors) include compounds described in the specifications of US Patent No. 5739135, US Patent No. 5712279 and US Patent No. 5760246.

The "anorectic drugs" include adrenaline-noradrenalin agonists (mazindol, ephedrine, etc.), serotonin agonists (selective serotonin reuptake inhibitors, for example, fluvoxamine, etc.), adrenaline-serotonin agonists (sibutramine, etc.), melanocortin-4 receptor (MC4R) agonists, α-melanocyte stimulating hormones (α-MCH), leptin, cocaine and amphetamine-regulated transcript (CART).

The "thyroid hormone receptor agonists" include liothyronine sodium, repothyroxine sodium.

The "cholesterol absorption inhibitors" include ezetimibe.

The "lipase inhibitors" include orlistat.

The "carnitine palmitoyltransferase inhibitors" include etomoxir.

The "nicotine acid derivatives" include nicotinic acid, nicotinic acid amides, nicomol, nicorandil.

The "bile acid adsorbing drugs" include cholestyramine, cholestyirane and colesevelam hydrochloride.

The "angiotensin converting enzyme inhibitors" include captoril, enalapril maleate, alacepril and cilazapril.

The "angiotensin II receptor antagonists" include candesartan cilexetil, losartan potassium and eprosartan mesylate.

The "endothelin converting enzyme inhibitors" include CGS-31447, CGS-35066.

The "endothelin receptor antagonists" include L-749805, TBC-3214 and BMS-182874.

For example, it is considered to be preferable that the compounds of the present invention are used in combination with at least one kind of drugs selected from the group consisting of insulin sensitivity enhancing drugs (PPAR γ agonists, PPAR α/γ agonists, PPAR δ agonists, PPAR α/γ/δ agonists, etc.), glycosidase inhibitors, biguanide drugs, insulin secretion enhancers, insulin formulations and dipeptidyl peptidase IV inhibitors in the treatment of diabetes and the like.

Alternatively, it is considered to be preferable that the compounds of the present invention are used in combination with at least one kind of drugs selected from the group consisting of hydroxymethylglutaryl coenzyme A reductase inhibitors, fibrate compounds, squalene synthetase inhibitors, acyl coenzyme A:cholesterol transacylase inhibitors, low-density lipoprotein receptor enhancers, microsome triglyceride transfer protein inhibitors and anorectic drugs.

The pharmaceutical agents of the present invention can be systemically or topically administered orally or parenterally, for example, intrarectally, subcutaneously, intramuscularly, intravenously and percutaneously.

For the purpose of using a compound of the present invention as a pharmaceutical agent, it can be in a form of a solid composition, a liquid composition or any other form of composition, and the most suitable form may be selected as required. The pharmaceutical agent of the present invention can be produced by blending a pharmaceutically acceptable carrier with a compound of the present invention. Specifically, commonly used excipients, expanders, binding agents, disintegrating agents, coating agents, sugar-coating agents, pH regulators, resolvents or aqueous or a non-aqueous solvents may be added to prepare tablets, pills, capsules, granules, powders, powdered drugs, liquid drugs, emulsion, suspension, injection agents by conventional drug preparing techniques. Examples of excipients and expanders include lactose, magnesium stearate, starch, talc, gelatin, agar, pectin, Arabian gum, olive oil, sesame oil, cacao butter, ethylene glycol and those commonly used.

In addition, the compounds of the present invention can be formulated into a drug by forming a clathrate compound with α-, β- or γ-cyclodextrin or methylated cyclodextrin.

The dose of the compounds of the present invention varies depending on disease, conditions, weight, age, sex, administration route, etc. but 0.1 to 1000 mg/kg weight/day for an adult is preferable and 0.1-200 mg/kg weight/day is more preferable, which can be administered once a day or divided into several times a day.

The compound of the present invention can be synthesized, for example, by a production process shown below.

The compound of the present invention can be synthesized by a process shown in Scheme 1: 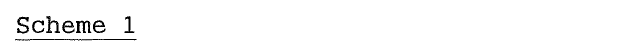 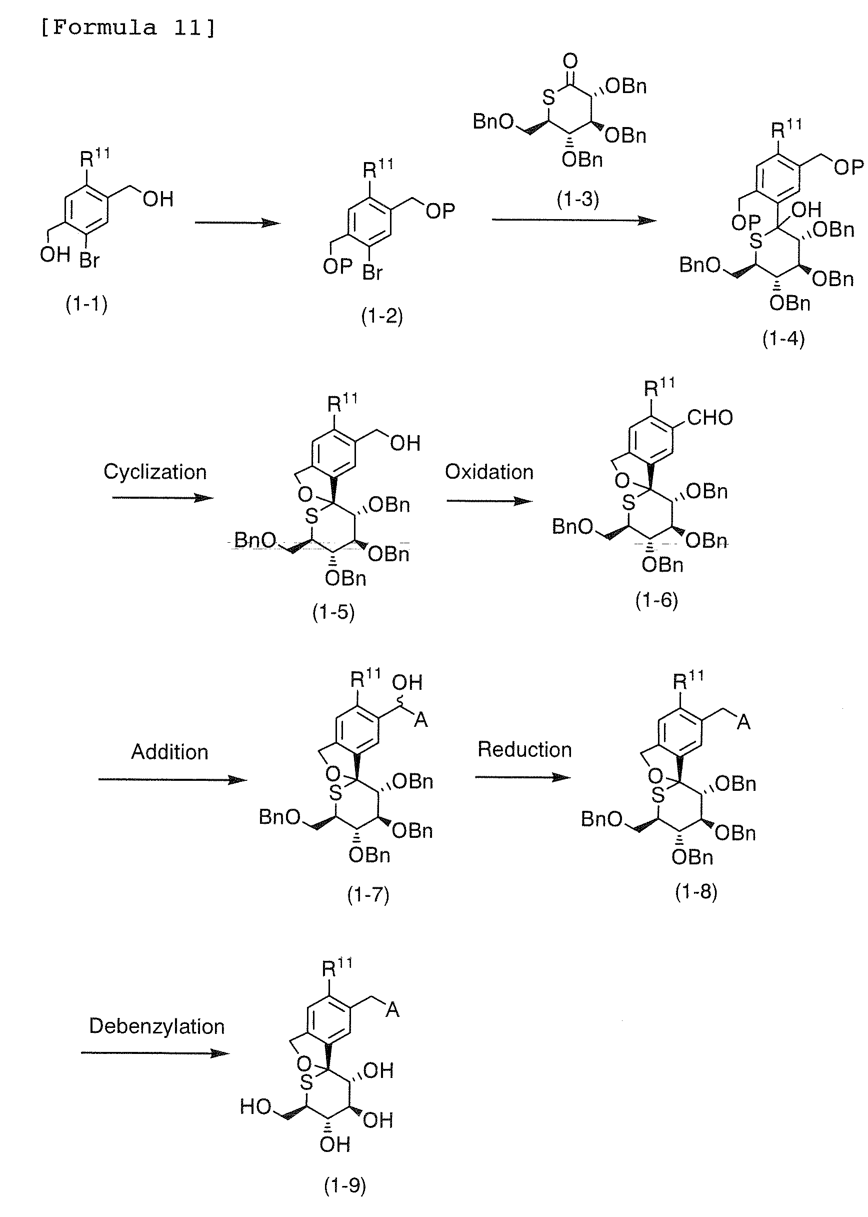 wherein R¹¹ means the same as defined above for R¹, P represents an appropriate protecting group, and A is as defined above.

The reaction converting Compound (1-1) to Compound (1-2) can be achieved by performing a reaction with a suitable protecting group introducing reagent in a suitable solvent. The suitable solvent includes THF, diethyl ether, N,N-dimethylformamide, dichloromethane, 1,2-dichloroethane, toluene and xylene. The suitable protecting group introducing reagent includes a reagent for introducing a protecting group, which can be removed in an acidic conditions, such as trityl chloride, tert-butyldimethylsilyl chloride, methoxymethyl chloride, 3,4-dihydro-2H-pyran, 2-methoxypropene, and preferably 2-methoxypropene is used. It is necessary to carry out this reaction of introducing a protecting group in the presence of a suitable base or acid. Specifically, in the case of using 2-methoxypropene, it is preferable to allow a catalytic amount of p-toluenesulfonic acid to be present as an acid. The above reaction can be performed normally from about -20°C to about 50°C, preferably from about 0°C to about 25°C (room temperature) for about 10 minutes to about 5 hours, preferably for about 30 minutes to about 2 hours.

The reaction converting Compound (1-2) to Compound (1-4) can be achieved by performing a reaction with a suitable alkyllithium reagent in a suitable solvent and then a reaction with Compound (1-3) ((3R,4S,5R,6R)-3,4,5-tris(benzyloxy)-6-(benzyloxymethyl)tetrahydrothiopyran-2-one). The suitable solvent includes THF, diethyl ether, dimethoxyethane, diethoxyethane, dichloromethane and toluene and preferably THF and toluene. The suitable alkyllithium reagent includes n-butyllithium, sec-butylithium, tert-butylithium, methyllithium, and preferably n-butyllithium is used. The above reaction can be performed normally from about -78°C to about 25°C (room temperature) for about 10 minutes to about 2 hours, preferably for about 1 hour to about 2 hours.

The reaction converting Compound (1-4) to Compound (1-5) can be achieved by performing a reaction with a suitable acid catalyst in a suitable solvent together with a deprotection step. The suitable solvent includes THF, dimethoxyethane, diethoxyethane, dichloromethane, toluene, methanol, ethanol, isopropanol, and preferably a mixture solvent of THF and methanol is used. The suitable acid catalyst includes p-toluenesulfonic acid, pyridinium-p-toluenesulfonic acid, methanesulfonic acid, trifluoromethanesulfonic acid, trifluoroacetic acid, camphorsulfonic acid, hydrochloric acid, sulfuric acid, acetic acid, and preferably p-toluenesulfonic acid is used. The above reaction can be performed normally from about -78°C to about 100°C, preferably from about 0°C to about 60°C for about 10 minutes to about 24 hours, preferably for about 2 hours to about 5 hours. In this step, isomerization of the spiro moiety occurs simultaneously with cyclization, and a compound with desired steric configuration can be obtained.

The reaction converting Compound (1-5) to Compound (1-6) can be achieved by performing a reaction with a suitable oxidizing agent in a suitable solvent. The suitable solvent includes dichloromethane, 1,2-dichloroethane, toluene and xylene, and preferably dichloromethane is used. The suitable oxidizing agent includes Dess-Martin reagent, TPAP-NMO, DMSO-acetic anhydride, DMSO-oxalyl chloride, manganese dioxide, chromic acid-sulfuric acid, SO₃-pyridine, and preferably manganese dioxide is used. The above reaction can be performed normally from about -78°C to about 40°C, preferably from about 0°C to about 25°C (room temperature) for about 10 minutes to about 24 hours, preferably for about 1 hour.

The reaction converting Compound (1-6) to Compound (1-7) can be achieved by performing a reaction with a suitable aryl metal reagent in a suitable solvent. The suitable solvent includes THF, diethyl ether, dimethoxyethane, diethoxyethane, dichloromethane and toluene, and preferably THF or diethyl ether is used. The suitable aryl metal reagent includes aryl magnesium halide and aryl lithium. The above reaction can be performed normally from about -78°C to about 25°C (room temperature) for about 10 minutes to about 2 hours, preferably for about 1 hour.

The reaction converting Compound (1-7) to Compound (1-8) can be achieved by performing a reaction with a suitable reducing reagent in a suitable solvent. The suitable solvent includes dichloromethane, dichloroethane, acetonitrile and toluene, and preferably dichloromethane or acetonitrile is used. The suitable reducing reagent preferably includes trifluoroboron-diethyl ether complex and triethylsilane. The above reaction can be performed normally from about -78°C to about 25°C (room temperature), preferably from about -40°C to about 25°C (room temperature) for about 10 minutes to about 6 hours, preferably for about 1 hour to about 2 hours.

The reaction converting Compound (1-8) to Compound (1-9) of the present invention can be achieved by performing a reaction with a suitable debenzylation reagent in a suitable solvent. The suitable solvent includes THF, ethyl acetate, methanol, ethanol and dichloromethane. The suitable debenzylation reagent includes palladium-carbon and hydrogen gas, palladium hydroxide-carbon and hydrogen gas, boron trichloride, boron tribromide, boron trichloride-dimethyl sulfide complex, boron trifluoride-diethyl ether complex and ethane thiol, boron trifluoride-diethyl ether complex and dimethyl sulfide, boron trichloride-pentamethylbenzene, sodium cyanide, sodium methanethiol, and preferably palladium-carbon and hydrogen gas, or boron trichloride-pentamethylbenzene is used. The above reaction can be performed normally from about -78°C to about 100°C, preferably from about -78°C to about 25°C (room temperature) for about 1 hour to about 24 hours, preferably for about 2 hours. In the case of reaction using palladium-carbon and hydrogen gas, the reaction may proceed smoothly in the presence of a catalytic amount of an acid, specifically diluted hydrochloric acid.

For example, Compound (1-1) can be synthesized by a process described in a document: J, Org. Chem., 1964, vol.29, page 2034, and Compound (1-3) can be synthesized by a process described in a doumunet: J. Chem. Soc. Perkin Trans.1, 1990, page 2763. Alternatively, Compound (1-1) of Scheme 1 can be synthesized by a process shown in Scheme 2, Scheme 3 or Scheme 4: 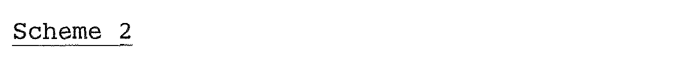 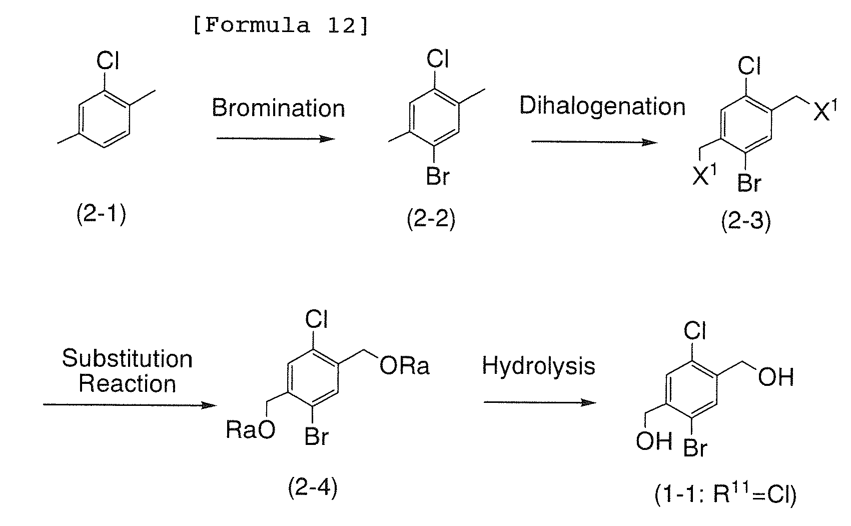 wherein X¹ is a halogen atom such as a bromine atom and a chlorine atom, and Ra is an acyl group such as C₁-C₆ alkylcarbonyl and arylcarbonyl.

The reaction converting Compound (2-1) to Compound (2-2) can be achieved by performing a treatment with bromine in the presence of the iron powders. Specifically, it can be performed following a process described in a document (J. Prakt. Chem., 1889, <2>39, page 402).

The reaction converting Compound (2-2) to Compound (2-3) can be achieved by performing a reaction with a suitable halogenation reagent in a suitable solvent. The suitable solvent includes ethyl acetate, ethyl acetate-water, and preferably ethyl acetate is used. The suitable halogenation reagent includes N-bromosuccinimide-2,2'-azobis(isobutyronitrile), N-bromosuccinimide-benzoyl peroxide, sodium bromate-sodium hydrogen sulfite, N-chlorosuccinimide-2,2'-azobis(isobutyronitrile), N-chlorosuccinimide-benzoyl peroxide, sulfuryl chloride-2,2'-azobis(isobutyronitrile), and preferably N-bromosuccinimide-2,2'-azobis(isobutyronitrile) is used. The above reaction can be performed normally from about 25°C (room temperature) to about 150°C, preferably from about 100°C to about 120°C for about 10 hours to about 24 hours, preferably for 15 minutes to about 1 hour.

The reaction converting Compound (2-3) to Compound (2-4) can be achieved by performing a reaction with a suitable carboxylate reagent in a suitable solvent. The suitable solvent includes dimethylformamide, acetonitrile, dimethoxyethane, ethyl acetate, and preferably dimethylformamide is used. The suitable carboxylate reagent includes sodium acetate, potassium acetate and sodium benzoate, and preferably sodium acetate is used. The above reaction can be performed normally from about 25°C (room temperature) to about 100°C, preferably at about 80°C for about 1 hour to about 24 hours, preferably for about 3 hours.

The reaction converting Compound (2-4) to Compound (1-1) can be achieved by performing a reaction with a suitable base reagent in a suitable solvent. The suitable solvent includes tetrahydrofuran-ethanol-water, tetrahydrofuran-methanol-water, ethanol-water, methanol-water, and preferably tetrahydrofuran-ethanol-water is used. The suitable base reagent includes sodium hydroxide, potassium hydroxide, lithium hydroxide, potassium carbonate, sodium carbonate, and preferably potassium hydroxide is used. The above reaction can be performed normally from about 0°C to about 100°C, preferably 25°C (room temperature) to about 80°C for about 15 minutes to about 24 hours, preferably for about 3 hours to about 5 hours.

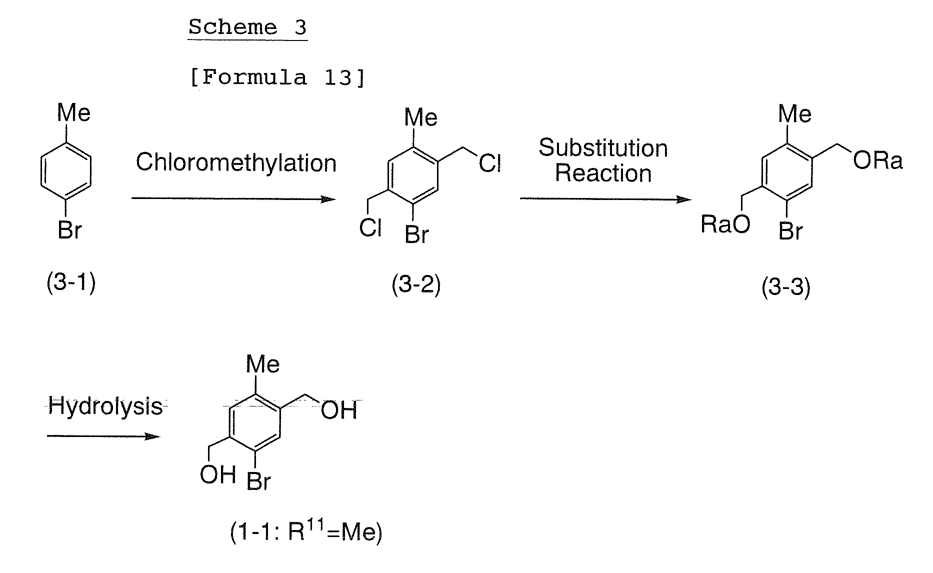

wherein Ra is an acyl group such as C₁-C₆ alkylcarbonyl and arylcarbonyl.

The reaction converting Compound (3-1) to Compound (3-2) can be achieved following a method described in a document: J. Org. Chem., 1975, 40 (21), page 3101.

The reaction converting Compound (3-2) to Compound (3-3) can be achieved by a similar process as that for converting Compound (2-3) to Compound (2-4) in Scheme 2.

The reaction converting Compound (3-3) to Compound (1-1) can be achieved by a similar process as that for converting Compound (2-4) to Compound (1-1) in Scheme 2. 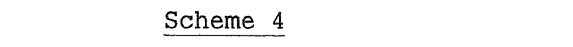 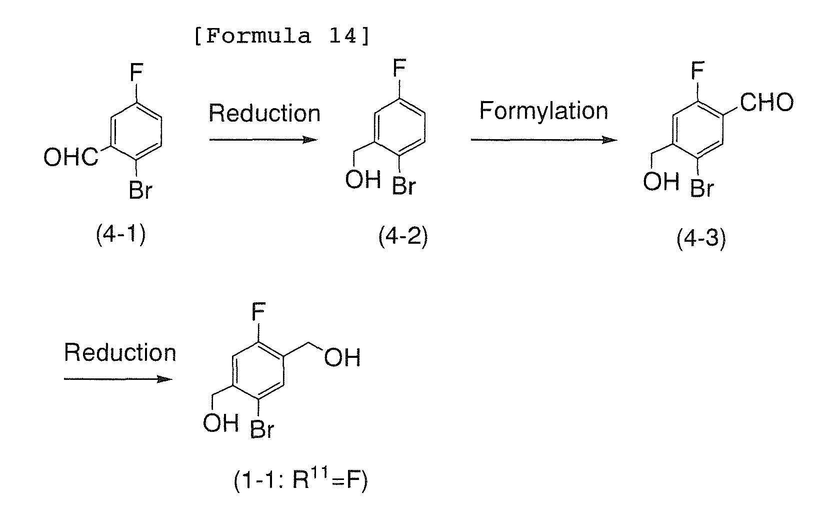 The reaction converting Compound (4-1) to Compound (4-2) can be achieved by performing a reaction with a suitable reduction reagent in a suitable solvent. The suitable solvent includes methanol, ethanol and tetrahydrofuran. The suitable reduction reagent includes sodium borohydride, lithium borohydride, lithium aluminum hydride and diisobutyl aluminum hydride, and preferably sodium borohydride or diisobutyl aluminum hydride is used. The above reaction can be performed normally from about -20°C to about 50°C, preferably at about 0°C for about 10 minutes to about 5 hours, preferably about 20 minutes to about 3 hours. As a reference cited for this reaction, there is J. Org. Chem., No. 70, page 756, 2005.

The reaction converting Compound (4-2) to Compound (4-3) can be achieved by performing a reaction with a suitable organic base reagent in a suitable solvent and then a reaction with a suitable formylation reagent. The suitable solvent includes THF, diethyl ether, dimethoxyethane, diethoxyethane, toluene, and preferably THF is used. The suitable organic base reagent includes n-butyllithium, sec-butylithium, tert-butylithium, methyllithium, n-butyllithium-2,2,6,6-tetramethylpiperidine, n-butyllithium-diisopropylamine, and preferably n-butyllithium-2,2,6,6-tetramethylpiperidine is used. The suitable formylation reagent includes dimethylformamide, 1-formylpiperidine. The above reaction can be performed normally from about -78°C to about 25°C (room temperature), for about 10 minutes to about 5 hours, preferably about 1 hour to about 4 hours.

The reaction converting Compound (4-3) to Compound (1-1) can be achieved by performing a reaction with a suitable reduction reagent in a suitable solvent. The suitable solvent includes methanol, ethanol, tetrahydrofuran. The suitable reduction reagent includes sodium borohydride, lithium borohydride, lithium aluminum hydride, and preferably sodium borohydride is used. The above reaction can be performed normally from about -20°C to about 50°C, preferably from about 0°C to about 25°C (room temperature), for about 5 minutes to about 24 hours, preferably about 10 minutes to about 1 hour.

The compound of the present invention can be also produced by a method of the following Scheme 5: 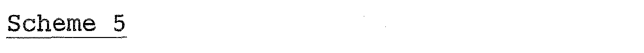 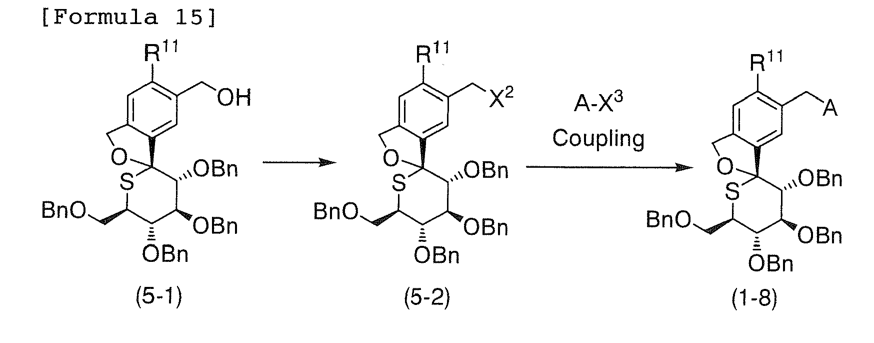 wherein R¹¹ means the same as R¹ defined above, A means the same as defined above, X² represents a leaving group such as a halogen atom and an alkylcarbonyloxy group, and X³ represents a boron atom, a silyl atom, a magnesium atom, a zinc atom, a tin atom or the like, each of which has a substituent(s).

The reaction converting Compound (5-1) to Compound (5-2) can be achieved: (i) by performing a reaction with a suitable halogenation reagent in a suitable solvent, or (ii) by performing a reaction with a suitable carbonate reagent in the presence of a suitable base in a suitable solvent. In the reaction (i), the suitable solvent includes tetrahydrofuran, dichloromethane, dichloroethane, toluene, acetonitrile, and preferably dichloromethane is used. The suitable halogenation reagent includes carbon tetrachloride-triphenyl phosphine, carbon tetrabromide-triphenyl phosphine, thionyl chloride, thionyl bromide, and preferably carbon tetrachloride-triphenyl phosphine, or thionyl chloride is used. The above reaction can be performed normally from about -20°C to about 60°C, preferably from about 0°C to about 25°C (room temperature), for about 1 hour to about 24 hours, preferably about 1 hour to about 2 hours. In the reaction of (ii), the suitable solvent includes tetrahydrofuran, dichloromethane, dichloroethane, toluene, and acetonitrile. The suitable base includes triethylamine, diisopropylethylamine, N-methyl-morphorline, 4-dimethylaminopyridine. The suitable carbonate reagent includes methyl chloroformate, ethyl chloroformate, benzyl chloroformate, and dimethyl carbonate. The above reaction can be performed normally from about 0°C to about 100°C, for about 10 minutes to about 24 hours.

The reaction converting Compound (5-2) to Compound (1-8) can be achieved by performing a reaction with a suitable arylation be achieved in a suitable solvent in the presence of a suitable transition metal catalyst, a suitable ligand, a suitable base and a suitable additive. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile, and water. The suitable transition metal catalyst includes palladium, nickel, cobalt, and iron. The suitable ligand includes triphenylphosphine, tri-tert-butylphosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf). The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo[5,4,0]-7-undecene (DBU), 1,5-diazabicyclo[4,3,0]-5-nonene (DBN), sodium tert-butoxide, potassium tert-butoxide, tetramethylguanidine. The suitable additive includes tetra-n-butyl-ammonium bromide, tetra-n-butyl-ammonium iodide, sodium bromide, sodium iodide, potassium bromide, and potassium iodide. The suitable arylation agent (A-X³) includes arylboronic acid, arylboronic acid ester, aryl magnesium halide, aryl zinc, aryl lithium, aryl tin, aryl silane, and preferably arylboronic acid is used. The above reaction can be performed normally from about 0°C to about 200°C, preferably from about 80°C to about 100°C, for about 10 minutes to about 24 hours, preferably about 1 hour to about 16 hours. As for arylboronic acid preferable as an arylation agent (A-X³), commercially available reagents can be used. When not commercially available, it can be synthesized following a process described in a reference (D. G. Hall, Boronic Acids: Preparation And Applications In Organic Synthesis And Medicines. (WILEY-VCH)).

The compounds of the present invention can be produced by a method of the following Scheme 6: 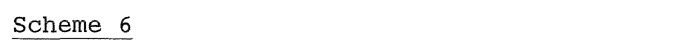 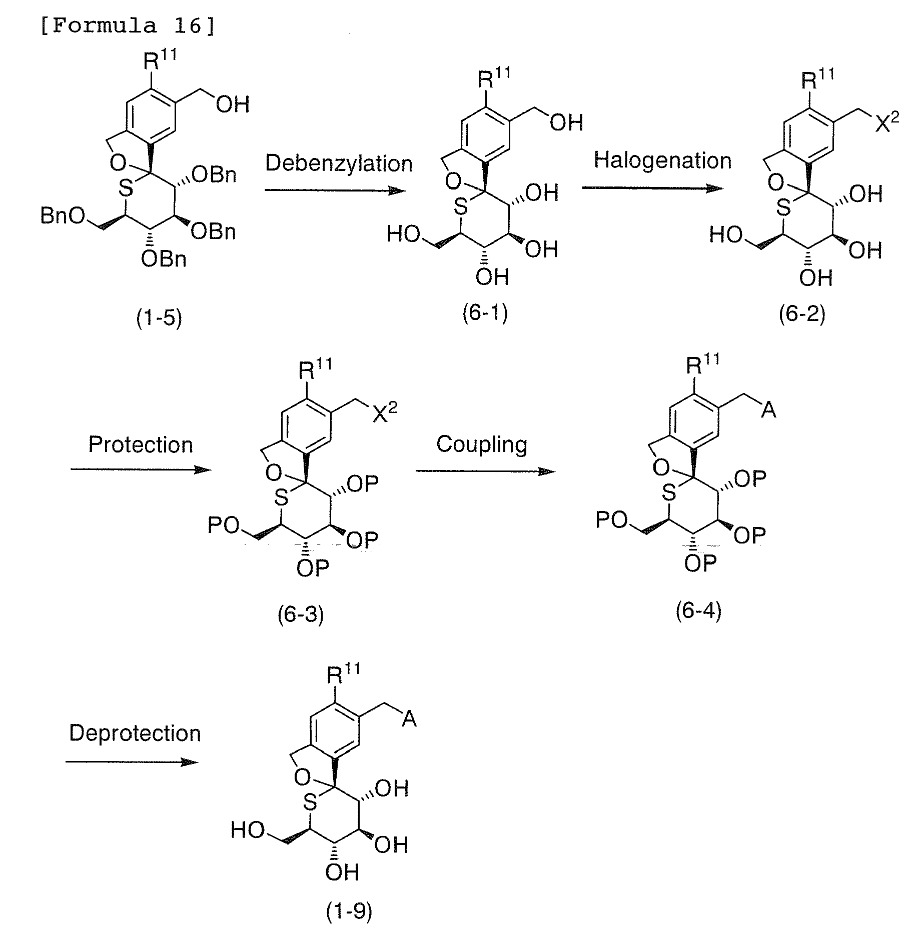 wherein R¹¹ means the same as R¹ defined above, A means the same as defined above, P represents a protecting group of a hydroxy group such as C₁-C₆ alkylcarbonyl, C₁-C₆ alkoxycarbonyl, arylcarbonyl, and X² represents a halogen atom.

The reaction converting Compound (1-5) to Compound (6-1) can be achieved by performing a reaction with a suitable debenzylation reagent in a suitable solvent. The suitable solvent includes dichloromethane, 1,2-dichloroethane, hexane, toluene, and preferably dichloromethane is used. The suitable debenzylation reagent includes boron trichloride; boron tribromide; boron trichloride-dimethyl sulfide complex; boron trifluoride-diethyl ether complex and ethane thiol; boron trifluoride-diethyl ether complex and dimethylsulfide; boron trichloride-pentamethylbenzene; sodium cyanide; sodium methanethiol; and the like. The above reaction can be performed normally from about -78°C to about 100°C, preferably from about -78°C to about 25°C (room temperature) for about 1 hour to about 24 hours.

The reaction converting Compound (6-1) to Compound (6-2) can be achieved by performing a reaction with a suitable halogenation reagent in a suitable solvent. The suitable solvent includes dimethylsulfoxide, dimethylformamide, and preferably dimethylsulfoxide is used. The suitable halogenation reagent includes trimethylsilyl chloride, trimethylsilyl bromide, and preferably trimethylsilyl chloride is used. The above reaction can be performed normally from about -78°C to about 50°C, preferably at room temperature, for about 1 hour to about 5 hours.

The reaction converting Compound (6-2) to Compound (6-3) can be achieved by performing a reaction with a suitable protecting group introducing reagent in the presence of a suitable base in a suitable solvent. The suitable solvent includes tetrahydrofuran, dichloromethane, acetonitrile, ethyl acetate, dimethylformamide. The suitable base includes N-methylmorpholine, N,N-dimethylaminopyridine, triethylamine. The suitable protecting group introducing reagent includes acetic anhydride, acetyl chloride, methyl chlorocarbonate, ethyl chlorocarbonate, benzoyl chloride, and preferably acetic anhydride is used. The above reaction can be performed normally from about 0°C to about 50°C, preferably at room temperature, for about 15 minutes to about 3 hours.

The reaction converting Compound (6-3) to Compound (6-4) can be achieved by a similar process to the reaction converting Compound (5-2) to Compound (1-8) in Scheme 5 indicated above.

The reaction converting Compound (6-4) to Compound (1-9) can be achieved by performing a reaction with a suitable base reagent in a suitable solvent. The suitable solvent includes methanol, ethanol, ethanol-water, methanol-water, tetrahydrofuran-ethanol-water, tetrahydrofuran-methanol-water, and preferably methanol is used. The suitable base reagent includes sodium hydroxide, potassium hydroxide, lithium hydroxide, potassium carbonate, sodium carbonate, and preferably potassium carbonate is used. The above reaction can be performed normally from about 0°C to about 100°C, preferably at about 25°C (room temperature), for about 15 minutes to about 24 hours, preferably for about 1 hour to about 2 hours.

The compound of the present invention can be produced by a process of the following Scheme 7: 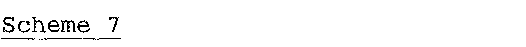 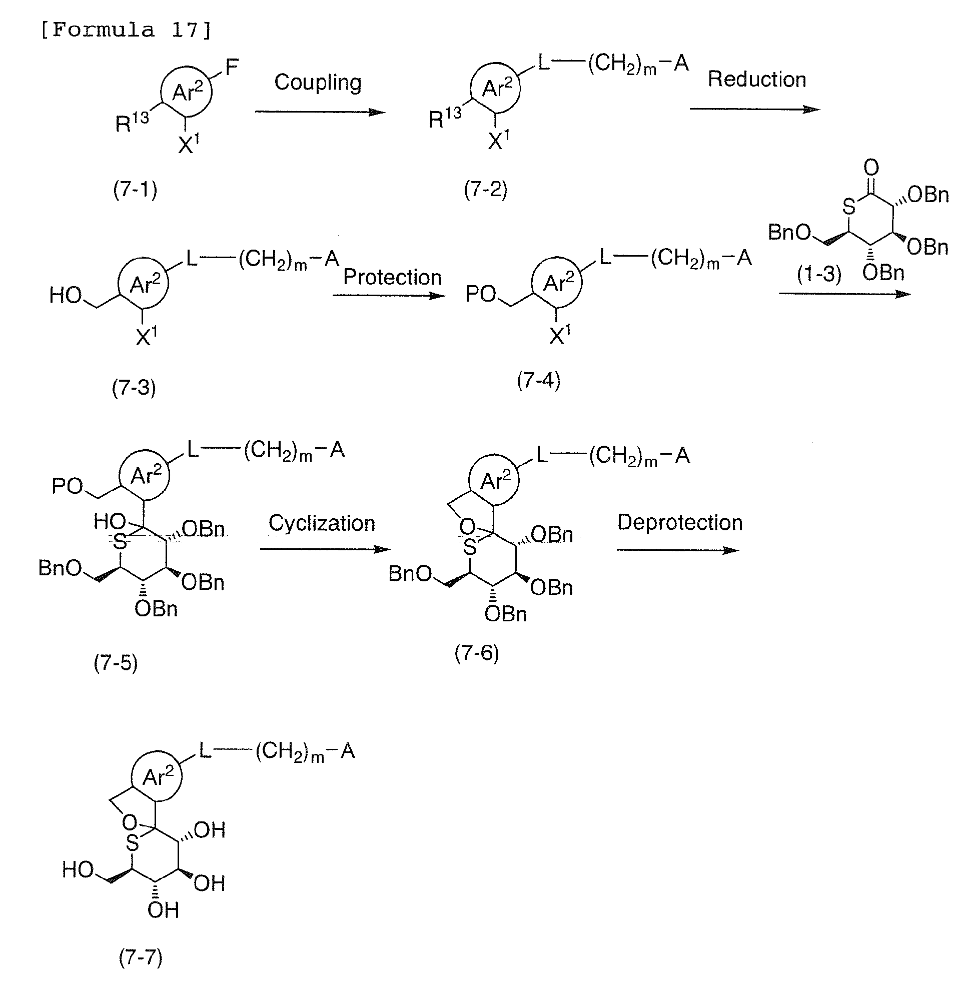 wherein R¹³ represents an ester group, Ar², L, m, and A are as defined above, P represents a protecting group for a hydroxy group, and X¹ represents a halogen atom.

The reaction converting Compound (7-1) to Compound (7-2) can be achieved by performing a reaction with a suitable nucleophile (A-(CH₂)ₘ-LH) in a suitable solvent in the presence of a suitable base. The suitable solvent includes dimethylformamide, 1,3-dimethylimidazolidinone, dimethylacetamide, dimethylsulfoxide, acetonitrile, tetrahydrofuran, and preferably dimethylformamide is used. The suitable base reagent includes potassium carbonate, sodium carbonate, triethylamine, diisopropylethylamine, N-methylmorpholine, and preferably potassium carbonate is used. The suitable nucleophile includes a substituted phenol (A-OH), a substituted benzyl alcohol (ACH₂OH), a substituted thiophenol (A-SH), a substituted aniline (A-NH₂), a substituted benzylamine (ACH₂NH₂). The above reaction can be performed normally from room temperature to about 180°C, for about 1 hour to about 36 hours.

The reaction converting Compound (7-2) to Compound (7-3) can be achieved by performing a reaction with a suitable reduction reagent in a suitable solvent. The suitable solvent includes tetrahydrofuran, diethyl ether, 1,2-dimethoxyethane, toluene, methanol, and ethanol. The suitable reduction reagent includes lithium aluminum hydride, sodium borohydride, lithium borohydride, zinc borohydride, and diisobutyl aluminum hydride. The above reaction can be performed normally from about -78°C to about 100°C, for about 5 minutes to about 10 hours.

The reaction converting Compound (7-3) to Compound (7-4) can be achieved by performing a reaction with a suitable protecting group introducing reagent in a suitable solvent. The suitable solvent includes THF, diethyl ether, N,N-dimethylformamide, dichloromethane, 1,2-dichloroethane, toluene and xylene. The suitable protecting group introducing reagent includes a reagent for introducing a protecting group, which can be removed in an acidic conditions, such as trityl chloride, tert-butyldimethylsilyl chloride, methoxymethyl chloride, 3,4-dihydro-2H-pyran, 2-methoxypropene, and preferably 2-methoxypropene is used. It is necessary to carry out this reaction of introducing a protecting group in the presence of a suitable base or acid. Specifically, in the case of using 2-methoxypropene, it is preferable to allow a catalytic amount of p-toluenesulfonic acid to be present as an acid. In the case of using trityl chloride, it is preferable to allow triethylamine and 4-dimethylaminopyridine to be present. The above reaction can be performed normally from about -20°C to about 50°C, for about 10 minutes to about 20 hours.

The reaction converting Compound (7-4) to Compound (7-5) can be achieved by performing a reaction with a suitable alkyllithium reagent in a suitable solvent and then a reaction with Compound (1-3). The suitable solvent includes THF, diethyl ether, dimethoxyethane, diethoxyethane, dichloromethane and toluene and preferably THF and toluene. The suitable alkyllithium reagent includes n-butyllithium, sec-butylithium, tert-butylithium, methyllithium, and preferably n-butyllithium is used. The above reaction can be performed normally from about -78°C to about 25°C (room temperature) for about 10 minutes to about 5 hours.

The reaction converting Compound (7-5) to Compound (7-6) can be achieved by performing a reaction with a suitable acid catalyst in a suitable solvent together with a deprotection step. The suitable solvent includes THF, dimethoxyethane, diethoxyethane, dichloromethane, toluene, methanol, ethanol, isopropanol, and preferably a mixture solvent of THF and methanol is used. The suitable acid catalyst includes p-toluenesulfonic acid, pyridinium-p-toluenesulfonic acid, methanesulfonic acid, trifluoromethanesulfonic acid, trifluoroacetic acid, camphorsulfonic acid, hydrochloric acid, sulfuric acid, acetic acid, and preferably p-toluenesulfonic acid is used. The above reaction can be performed normally from about -78°C to about 100°C, preferably from about 0°C to about 60°C for about 10 minutes to about 24 hours, preferably for about 1 hours to about 5 hours. In this step, isomerization of the spiro moiety occurs simultaneously with cyclization, and a compound with desired steric configuration can be obtained.

The reaction converting Compound (7-6) to Compound (7-7) of the present invention can be achieved by performing a reaction with a suitable debenzylation reagent in a suitable solvent. The suitable solvent includes THF, ethyl acetate, methanol, ethanol and dichloromethane. The suitable debenzylation reagent includes palladium-carbon and hydrogen gas, palladium hydroxide-carbon and hydrogen gas, boron trichloride, boron tribromide, boron trichloride-dimethyl sulfide complex, boron trifluoride-diethyl ether complex and ethane thiol, boron trifluoride-diethyl ether complex and dimethyl sulfide, boron trichloride-pentamethylbenzene, sodium cyanide, sodium methanethiol, and preferably palladium-carbon and hydrogen gas, or boron trichloride-pentamethylbenzene is used. The above reaction can be performed normally from about -78°C to about 100°C, preferably from about -78°C to about 25°C (room temperature) for about 1 hour to about 24 hours. In the case of reaction using palladium-carbon and hydrogen gas, the reaction may proceed smoothly in the presence of a catalytic amount of an acid, specifically diluted hydrochloric acid.

The compound of the present invention can be produced by a process of the following Scheme 8: 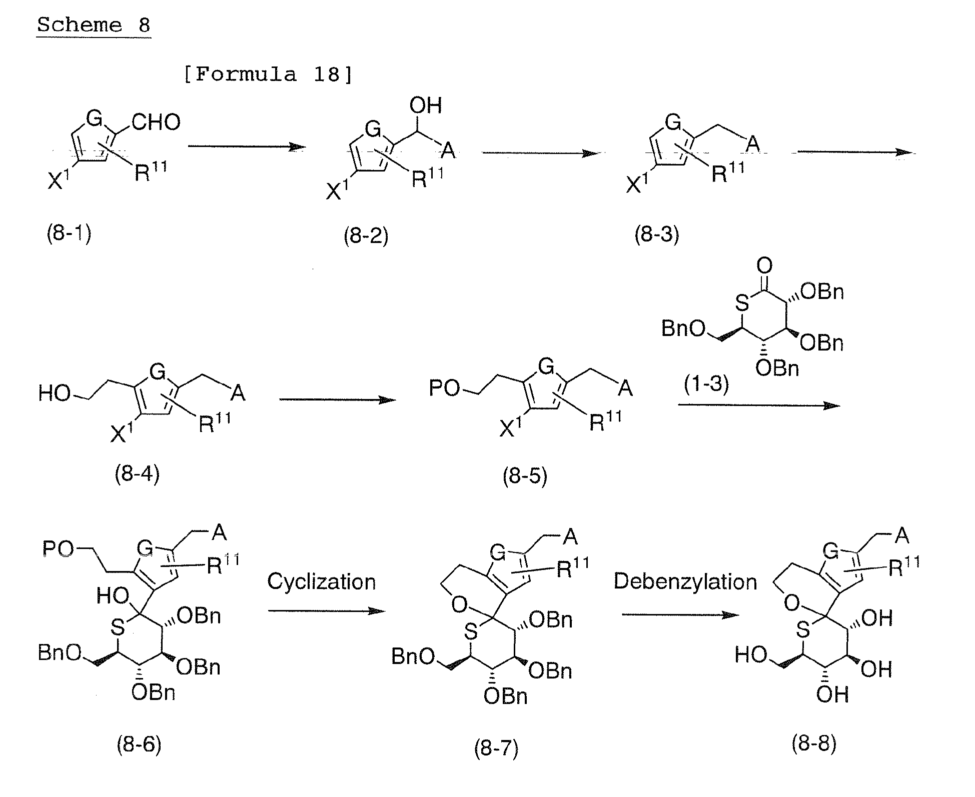 wherein R¹¹ means the same as defined above for substituents on Ar¹, G represents -O-, -S- or -NP-, P represents a protecting group for an amino group, A means the same as defined above, and X¹ represents a halogen atom.

The reaction converting Compound (8-1) to Compound (8-2) can be achieved by performing a reaction with a suitable aryl metal reagent in a suitable solvent. The suitable solvent includes THF, diethyl ether, dimethoxyethane, diethoxyethane, and toluene. The suitable aryl metal reagent includes an aryl lithium reagent and an aryl Grignard reagent. The above reaction can be performed normally from about -78°C to about 25°C (room temperature) for about 10 minutes to about 5 hours.

The reaction converting Compound (8-2) to Compound (8-3) can be achieved in the same manner as convertion of Compound (1-7) to Compound (1-8) in Scheme 1.

The reaction converting Compound (8-3) to Compound (8-4) can be achieved by performing a reaction with a suitable organic base reagent in a suitable solvent and then a reaction with ethylene oxide. The suitable solvent includes THF, diethyl ether, dimethoxyethane, diethoxyethane, and toluene. The suitable organic base reagent includes n-butyllithium, sec-butylithium, tert-butylithium, methyllithium, n-butyllithium-2,2,6,6-tetramethylpiperidine, n-butyllithium-diisopropylamine. The above reaction can be performed normally from about -78°C to about 25°C (room temperature) for about 10 minutes to about 5 hours, preferably for about 1 hour to about 6 hour.

The reaction converting Compound (8-4) to Compound (8-5) can be achieved in the same manner as convertion of Compound (7-3) to Compound (7-4) in Scheme 7.

The reaction converting Compound (8-5) to Compound (8-6) followed by conversion of Compound (8-6) to Compound (8-7) can be achieved in the same manner as convertion of Compound (1-2) to Compound (1-4) followed by conversion of Compound (1-4) to Compound (1-5) in Scheme 1.

The reaction converting Compound (8-7) to Compound (8-8) can be achieved in the same manner as convertion of Compound (1-8) to Compound (1-9) in Scheme 1.

The compound of the present invention in which R¹ is an ethynyl group can be produced by a process of the following Scheme 9: 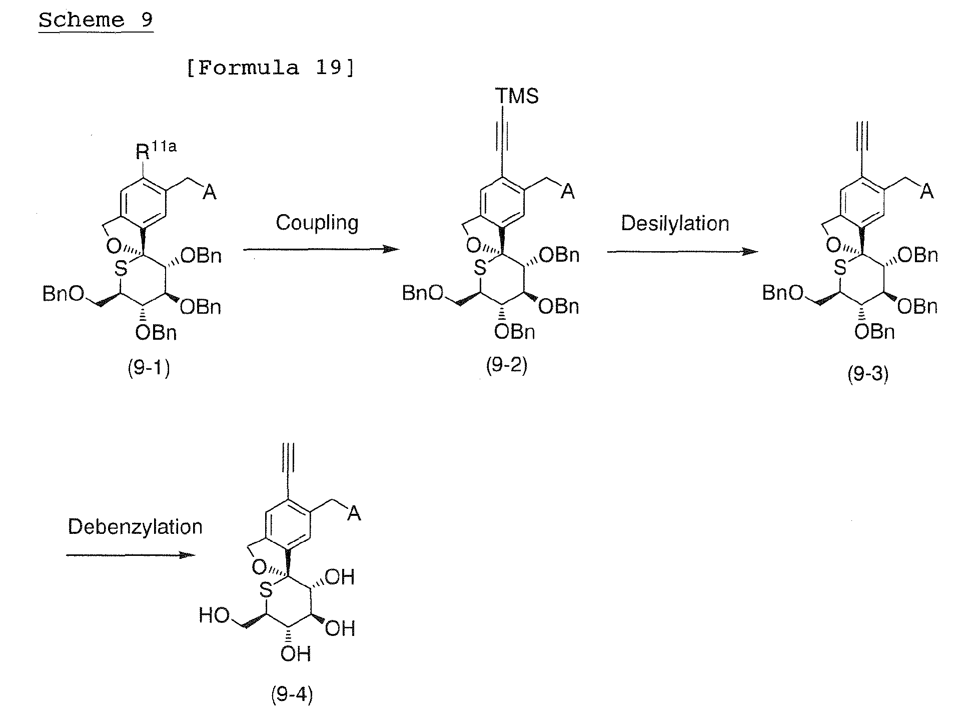 wherein R^{11a} is a leaving group suitable for coupling reaction (for example, chlorine atom, bromine atom, a trifluoromethanesulfonyloxy group, etc.) and A means the same as defined above.

The reaction converting Compound (9-1) to Compound (9-2) can be achieved by performing a reaction with ethynyltrimethylsilane in a suitable solvent in the presence of a suitable transition metal catalyst, a suitable ligand and a suitable base. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile. The suitable transition metal catalyst includes palladium, nickel, cobalt, iron. The suitable ligand includes triphenylphosphine, 2-dicyclohexylphosphino-2',4',6'-triisopropylbiphenyl, tri-tert-butylphosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf), acetonitrile. The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, DBU, DBN, sodium tert-butoxide, potassium tert-butoxide, tetramethylguanidine. The above reaction can be performed normally from about 0°C to about 200°C, preferably from about 25°C (room temperature) to about 100°C, for about 10 minutes to about 24 hours, preferably for about 1 hour to about 4 hours.

The reaction converting Compound (9-2) to Compound (9-3) can be achieved by performing a reaction with a suitable desilylation agent in a suitable solvent. The suitable solvent includes methanol, ethanol, water, tetrahydrofuran, and methanol is preferable. The suitable desilylation agent includes potassium carbonate, sodium carbonate, sodium hydroxide, potassium hydroxide, sodium methoxide, tetrabutylammonium fluoride, potassium fluoride. The above reaction can be performed normally from about 0°C to about 100°C, preferably at room temperature, for about 1 hour to about 24 hours.

The reaction converting Compound (9-3) to Compound (9-4) can be achieved by performing a reaction with a suitable debenzylation reagent in a suitable solvent. The suitable solvent includes dichloromethane, 1,2-dichloroethane. The suitable debenzylation reagent includes boron trichloride, boron tribromide, boron trichloride-dimethyl sulfide complex, boron trifluoride-diethyl ether complex and ethanethiol, boron trifluoride-diethyl ether complex and dimethylsulfide, boron trichloride-pentamethylbenzene, sodium cyanide, sodium methanethiolate. The above reaction can be performed normally from about -78°C to about 100°C, preferably from about -78°C to about 25°C (room temperature) for about 1 hour to about 24 hours.

The compound of the present invention in which R¹ is an ethynyl group can be produced by a process of the following Scheme 10: 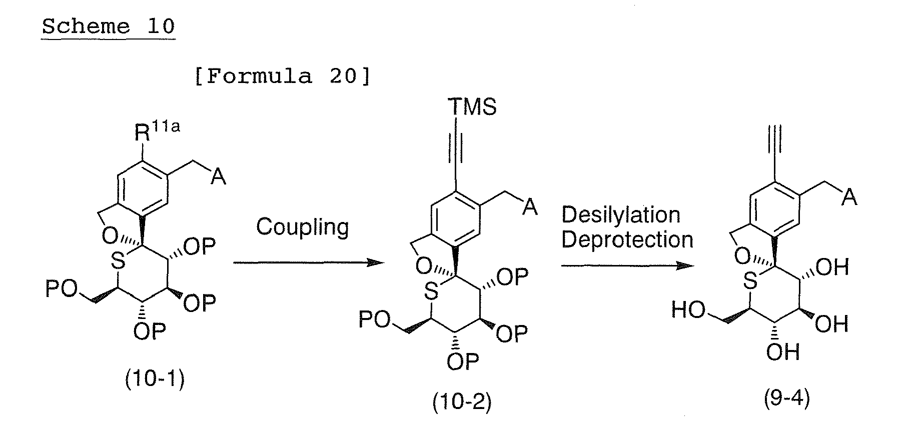 wherein R^{11a} is a leaving group suitable for coupling reaction (for example, chlorine atom, bromine atom, trifluoromethanesulfonyloxy group, etc.), P represents an appropriate protecting group and A means the same as defined above.

The reaction converting Compound (10-1) to Compound (10-2) can be achieved by performing a reaction with ethynyltrimethylsilane in a suitable solvent in the presence of a suitable transition metal catalyst, a suitable ligand and a suitable base. The suitable solvent includes acetonitrile, tetrahydrofuran, dimethylformamide, dioxane, dimethylsulfoxide, toluene, dimethoxyethane, and preferably acetonitrile is used. The suitable transition metal catalyst includes palladium, nickel, cobalt, iron, and preferably palladium is used. The suitable ligand includes triphenylphosphine, 2-dicyclohexylphosphino-2',4',6'-triisopropylbiphenyl, tri-tert-butylphosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf), acetonitrile, and preferably 2-dicyclohexylphosphino-2',4',6'-triisopropylbiphenyl is used. The suitable base includes potassium carbonate, sodium carbonate, cesium carbonate, sodium acetate, potassium acetate, sodium hydroxide, potassium hydroxide, potassium phosphate, sodium phosphate, dipotassium hydrogen phosphate, triethylamine, and preferably sodium carbonate or cesium carbonate is used. The above reaction can be performed normally from about 0°C to about 120°C, preferably at about 25°C (room temperature), for about 1 hour to about 24 hours, preferably for about 1 hour to about 4 hours.

For protective group P of Compound (10-1), an acetyl group, a benzoyl group, a methoxycarbonyl group, an ethoxycarbonyl group or the like is preferable.

The reaction converting Compound (10-2) to Compound (9-4) can be achieved by performing a reaction with a suitable base in a suitable solvent. The suitable solvent includes methanol, ethanol, water, tetrahydrofuran, acetonitrile, and preferably methanol is used. The suitable base includes potassium carbonate, sodium carbonate, potassium hydroxide, sodium hydroxide, sodium methoxide, and preferably potassium carbonate is used. The above reaction can be performed normally from about 0°C to about 100°C, preferably at about 25°C (room temperature), for about 1 hour to about 24 hours, preferably for about 1 hour to about 3 hours.

The compound of the present invention in which R² is an alkynyl group can be produced by a process of the following Scheme 11: 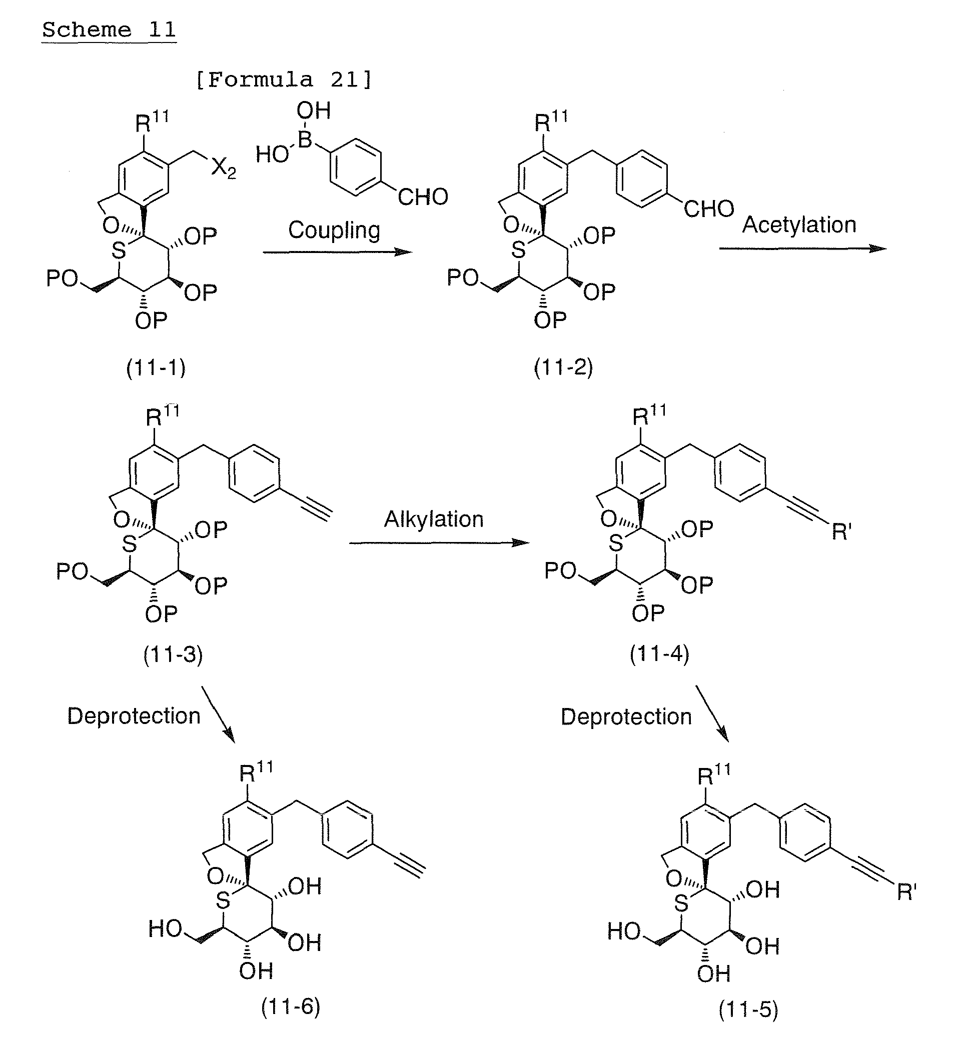 wherein R¹¹ means the same as R¹ defined above, X² represents a halogen atom, and P represents an appropriate protecting group of a hydroxy group, and R' is a C₁-C₄ alkyl which may be substituted with -OR⁴.

For protecting group P of Compound (11-1), an ether-type protecting group such as a benzyl group, a p-methoxybenzyl group and an allyl group is preferable, and a benzyl group is particularly preferable.

The reaction converting Compound (11-1) to Compound (11-2) can be achieved by performing a reaction with a p-formylphenylation agent (preferably, p-formylphenylboronic acid) in a suitable solvent in the presence of a suitable transition metal catalyst, a suitable ligand, a suitable base and a suitable additive. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile, water. The suitable transition metal catalyst includes palladium, nickel, cobalt, iron. The suitable ligand includes triphenylphosphine, tri-tert-butylphosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), and 1,1'-bis(diphenylphosphino)ferrocene (dppf). The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo[5,4,0]-7-undecene (DBU), 1,5-diazabicyclo[4,3,0]-5-nonene (DBN), sodium tert-butoxide, potassium tert-butoxide, tetramethylguanidine. The suitable additive includes tetra-n-butylammonium bromide, tetra-n-butylammonium iodide, sodium bromide, sodium iodide, potassium bromide, potassium iodide. The above reaction can be performed normally from about 0°C to about 200°C, preferably from about 80°C to about 160°C, for about 10 minutes to about 24 hours, preferably about 15 minutes to about 16 hours.

The reaction converting Compound (11-2) to Compound (11-3) can be achieved by reacting a suitable base and a suitable ethynylation agent in a suitable solvent. The suitable solvent includes THF, dimethoxyethane, diethoxyethane, dioxane, dichloromethane, 1,2-dichloroethane, toluene, xylene, methanol, ethanol, and preferably a mixture solvent of THF and methanol is used. The suitable base includes potassium carbonate, sodium carbonate, sodium hydroxide, potassium hydroxide. The suitable ethynylation agent includes dimethyl(1-diazo-2-oxopropyl)phosphonate. The above reaction can be performed normally from about 0°C to about 120°C, preferably from about 0°C to about 25°C (room temperature), for about 10 minutes to about 16 hours, preferably about 3 hours to about 5 hours. Dimethyl(1-diazo-2-oxopropyl) phosphonate usable as an ethynylation agent can be synthesized, for example, by following a process described in a document (Eur. J. Org. Chem., page 821, 2003).

The reaction converting Compound (11-3) to Compound (11-4) can be achieved by performing a reaction with a suitable base in a suitable solvent and then a reaction with a suitable alkylating agent. The suitable solvent includes THF, diethyl ether, dimethoxyethane, diethoxyethane, toluene, and preferably THF is used. The suitable base includes n-butyllithium, sec-butylithium, tert-butylithium, methyllithium, and preferably n-butyllithium is used. The suitable alkylating agent includes alkyl halide, aldehyde, ketone, and preferably alkyl halide is used. The above reaction can be performed normally from about -78°C to about 25°C (room temperature), for about 1 hour to about 5 hours.

The reaction converting Compound (11-3) to Compound (11-6) or Compound (11-4) to Compound (11-5) is a deprotection reaction, and removal of benzyl group, which is preferred as a protecting group, can be achieved by performing a reaction with a suitable debenzylation reagent in a suitable solvent. The suitable solvent includes dichloromethane, 1,2-dichloroethane. The suitable debenzylation reagent includes boron trichloride, boron tribromide, boron trichloride-dimethyl sulfide complex, boron trifluoride-diethyl ether complex and ethanethiol, boron trifluoride-diethyl ether complex and dimethylsulfide, boron trichloride-pentamethylbenzene, sodium cyanide, sodium methanethiolate, and preferably boron trichloride-pentamethylbenzene is used. The above reaction can be performed normally from about -78°C to about 25°C (room temperature), preferably from about -78°C to about 0°C, for about 1 hour to about 7 hours, preferably for about 2 hours to about 3 hours.

The compound of the present invention in which R² is an alkynyl group can be produced by a process of the following Scheme 12: 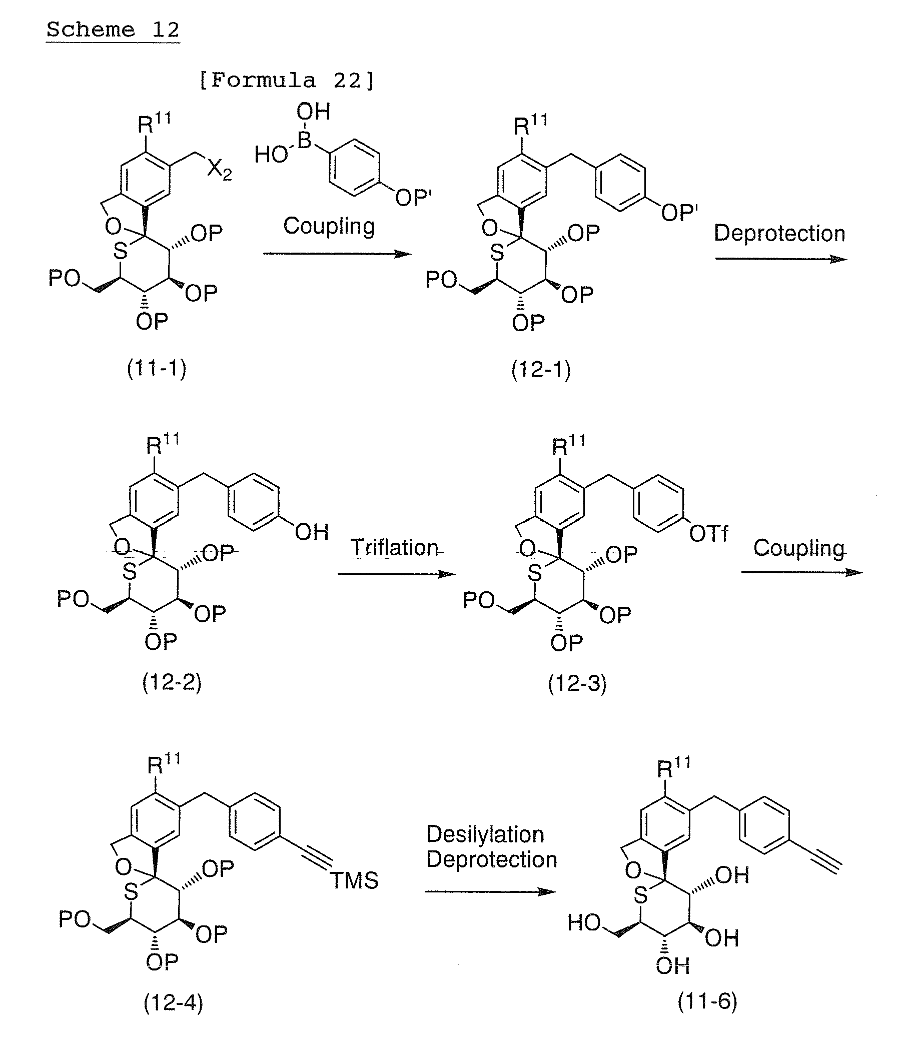 wherein R¹¹ means the same as R¹ defined above, X² represents a halogen atom, and P and P' respectively represent an appropriate protecting group of a hydroxy group.

The reaction converting Compound (11-1) to Compound (12-1) can be achieved by performing a reaction with a suitable aryl boronic acid in a suitable solvent in the presence of a suitable transition metal catalyst, a suitable ligand, a suitable base and a suitable additive. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile, water. The suitable transition metal catalyst includes palladium, nickel, cobalt, iron. The suitable ligand includes triphenylphosphine, tri-tert-butyl phosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf). The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo[5,4,0]-7-undecene (DBU), 1,5-diazabicyclo[4,3,0]-5-nonene (DBN), sodium tert-butoxide, potassium tert-butoxide, tetramethylguanidine. The suitable additive includes tetra-n-butylammonium bromide, tetra-n-butylammonium iodide, sodium bromide, sodium iodide, potassium bromide, potassium iodide. The suitable O-protecting group of a suitable aryl boronic acid includes silane protecting groups such as trimethylsilyl group, tert-butyldimethylsilyl group, triisopropylsilyl group, triethylsilyl group, tert-butyldiphenylsilyl group; and ether protecting groups such as methoxymethyl group, methoxyethoxymethyl group, tetrahydropyranyl group, trityl group, benzyl group, p-methoxy benzyl group, and preferably silane protecting groups are used. The above reaction can be performed normally from about 0°C to about 200°C, preferably from about 80°C to about 100°C, for about 10 minutes to about 24 hours, preferably about 1 hour to about 16 hours. The aryl boronic acid can be obtained by protecting a phenolic hydroxy group of commercially available 4-hydroxyphenyl boronic acid with a suitable protecting group.

The reaction converting Compound (12-1) to Compound (12-2) can be achieved by performing a reaction with a suitable desilylation agent in a suitable solvent in the case that a silane protecting group, which is preferred as a protecting group of a phenol hydroxy group, is used. The suitable solvent includes tetrahydrofuran, diethyl ether, dimethoxyethane, diethoxyethane, dioxane, N,N-dimethylformamide, N,N-dimethylacetamide, dimethylsulfoxide, dichloromethane, 1,2-dichloroethane, toluene, xylene, ethanol, acetonitrile, water, and preferably tetrahydrofuran is used. The suitable desilylation agent includes tetrabutylammonium fluoride, potassium fluoride, cesium fluoride, hydrogen fluoride, acetic acid, hydrochloric acid, sulfuric acid, trifluoroacetic acid, p-toluenesulfonic acid, triethylamine-hydrogen fluoride, pyridine-hydrogen fluoride, and preferably tetrabutylammonium fluoride is used. The above reaction can be performed normally from about -20°C to about 100°C, preferably from about 0°C to about 25°C (room temperature), for about 10 minutes to about 24 hours, preferably about 15 minutes to about 5 hours.

The reaction converting Compound (12-2) to Compound (12-3) can be achieved by performing a reaction with a suitable triflation agent in a suitable solvent in the presence of a suitable base. The suitable solvent includes tetrahydrofuran, diethyl ether, dimethoxyethane, diethoxyethane, dioxane, N,N-dimethylformamide, N,N-dimethyl acetamide, dichloromethane, 1,2-dichloroethane, toluene, xylene, acetonitrile, and preferably dichloromethane is used. The suitable base includes pyridine, triethylamine, diisopropylethylamine, N,N-dimethylaminopyridine, and preferably pyridine is used. The suitable triflation agent includes trifluoromethanesulfonic anhydride. The above reaction can be performed normally from about -78°C to about 25°C (room temperature), preferably from about -20°C to about 25°C (room temperature), for about 10 minutes to about 24 hours, preferably about 1 hour to about 6 hours.

The reaction converting Compound (12-3) to Compound (12-4) can be achieved by performing a reaction with trimethylsilyl acetylene in a suitable solvent in the presence of a suitable transition metal catalyst, a suitable ligand, a suitable base and a suitable additive. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile, water, and preferably N,N-dimethylformamide is used. The suitable transition metal catalyst includes palladium, nickel, cobalt, iron, and preferably palladium is used. The suitable ligand includes triphenylphosphine, tri-tert-butylphosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf). The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo[5,4,0]-7-undecene (DBU), 1,5-diazabicyclo[4,3,0]-5-nonene (DBN), sodium tert-butoxide, potassium tert-butoxide, tetramethylguanidine. The suitable additive includes copper (I) iodide. The above reaction can be performed normally from about 0°C to about 200°C, preferably from about 80°C to about 100°C, for about 10 minutes to about 24 hours, preferably about 1 hour to about 6 hours.

The reaction converting Compound (12-4) to Compound (11-6) can be achieved by performing a deprotection reaction simultaneously with desilylation or performing a deprotection reaction after performing desilylation. When the protecting group is an acyl group such as an acetyl group, a benzoyl group, a methoxycarbonyl group, an ethoxycarbonyl group, deprotection and desilylation can be performed simultaneously by performing a reaction with a suitable base in a suitable solvent. The suitable solvent includes methanol, ethanol, water, tetrahydrofuran, acetonitrile, and preferably methanol is used. The suitable base includes potassium carbonate, sodium carbonate, potassium hydroxide, sodium hydroxide, sodium methoxide, and preferably potassium carbonate is used. The above reaction can be performed normally from about 0°C to about 100°C, preferably at about 25°C (room temperature), for about 1 hour to about 24 hours, preferably about 1 hour to about 3 hours. When the protecting group is a benzyl group, this step can be achieved by performing a reaction with a suitable debenzylation reagent in a suitable solvent after conducting desilylation reaction by the above-mentioned method. The suitable solvent includes dichloromethane, 1,2-dichloroethane. The suitable debenzylation reagent includes boron trichloride, boron tribromide, boron trichloride-dimethyl sulfide complex, boron trifluoride-diethyl ether complex and ethanethiol, boron trifluoride-diethyl ether complex and dimethylsulfide, boron trichloride-pentamethylbenzene, sodium cyanide, sodium methanethiolate, and preferably boron trichloride-pentamethylbenzene is used. The above reaction can be performed normally from about -78°C to about 25°C (room temperature), preferably from about -78°C to about 0°C, for about 1 hour to about 7 hours, preferably for about 2 hours to about 3 hours.

Compound (11-3) and Compound (11-4) of Scheme 11 and Compounds (12-4) of Scheme 12 can also be produced by a process of the following Scheme 13:
Scheme 13

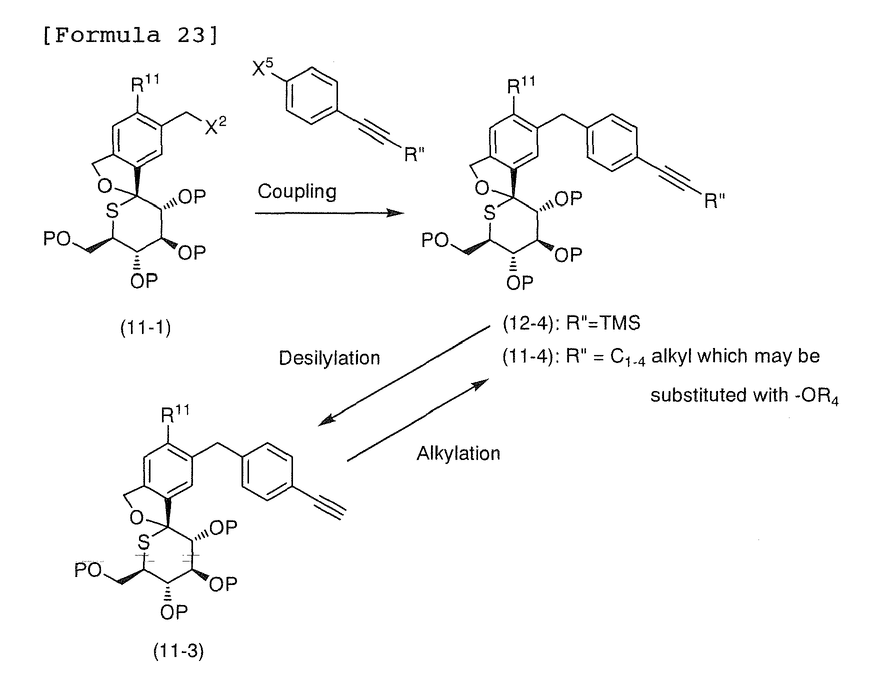

wherein R¹¹ means the same as R¹ defined above, X² represents a halogen atom, X⁵ represents a boron atom, a silyl atom, a magnesium atom, a zinc atom, a tin atom, each of which has a substituent(s), P represents an appropriate protecting group of a hydroxy group and R" is a C₁-C₄ alkyl which may be substituted with -OR⁴ or trimethylsilyl.

Compound (12-4) and Compound (11-4) can be synthesized by reacting Compound (11-1) with a suitable p-alkynyl substituted phenylation agent in a suitable solvent in the presence of a suitable transition metal catalyst, a suitable ligand, a suitable base and a suitable additive. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile, water. The suitable transition metal catalyst includes palladium, nickel, cobalt, iron. The suitable ligand includes triphenylphosphine, tri-tert-butylphosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf). The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo[5,4,0]-7-undecene (DBU), 1,5-diazabicyclo[4,3,0]-5-nonene (DBN), sodium tert-butoxide, potassium tert-butoxide, tetramethylguanidine. The suitable additive includes tetra-n-butylammonium bromide, tetra-n-butylammonium iodide, sodium bromide, sodium iodide, potassium bromide, potassium iodide. The suitable p-alkynyl substituted phenylation agent includes compounds in which X⁵ is boronic acid, boronic acid ester, magnesium halide, zinc, lithium, tin, silane, and preferably boronic acid compound is used. The above reaction can be performed normally from about 25°C (room temperature) to about 200°C, preferably from about 80°C to about 120°C, for about 10 minutes to about 24 hours, preferably about 1 hour to about 16 hours.

Compound (11-3) can be synthesized by reacting Compound (12-4) with a suitable base in a suitable solvent. As for this case, however, the protecting group is suitably a group such as a benzyl group which can stand against a basic condition. The suitable solvent includes methanol, ethanol, water, tetrahydrofuran, acetonitrile, and preferably methanol is used. The suitable base includes potassium carbonate, sodium carbonate, potassium hydroxide, sodium hydroxide, sodium methoxide, and preferably potassium carbonate is used. The above reaction can be performed normally from about 0°C to about 100°C, preferably at about 25°C (room temperature), for about 1 hour to about 24 hours, preferably about 1 hour to about 3 hours.

Compounds (11-3) can be converted to the desired compound of the present invention by conducting appropriate combination of the processes in Schemes 10 to 13 indicated above.

The compound wherein ring Ar¹ is represented by Formula (a) can be synthesized according to a process of the following Scheme 14: 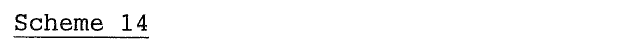 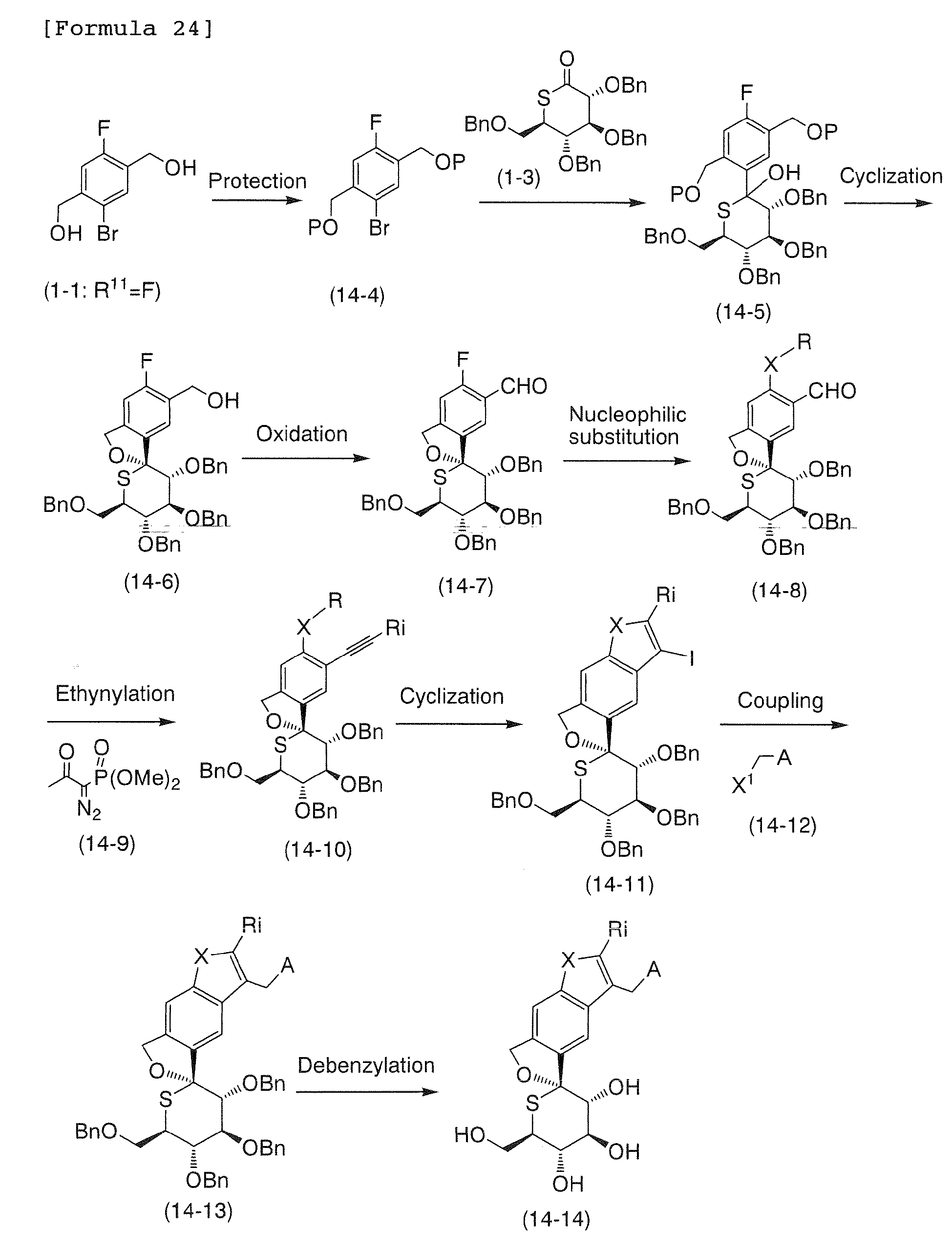 wherein A is an aromatic ring which may have a substituent(s); P is a protecting group of a hydroxy group; Ri represents a hydrogen atom, a halogen atom, a C₁-C₆ alkyl group or a silyl group which may have a substituent(s); R represents a methyl group or an ethyl group; X represents an oxygen atom, a nitrogen atom or a sulfur atom; X¹ represents a halogen atom or a boron atom, silicon atom, magnesium atom, zinc atom, tin atom or the like, each of which has a substituent(s).

The reaction converting Compound (1-1) to Compound (14-4) can be achieved by a reaction with a suitable protecting group introducing reagent in a suitable solvent. Examples of a suitable solvent include THF, diethyl ether, N,N-dimethylformamide, dichloromethane, 1,2-dichloroethane, toluene, xylene and the like. Examples of a suitable protecting group introducing reagent include a reagent for introducing a protecting group which can be removed under acidic conditions, such as trityl chloride, tert-butyldimethylsilyl chloride, methoxymethyl chloride, 3,4-dihydro-2H-pyran, 2-methoxypropene and the like, and 2-methoxypropene is preferred. The reaction can generally be carried out at about -20°C to about 50°C, and preferably at about 0°C to about 25°C (room temperature), for about 10 minutes to 5 hours, and preferably for about 1 hour.

The reaction converting Compound (14-4) to Compound (14-5) can be achieved by a reaction with a suitable alkyllithium reagent in a suitable solvent, followed by a reaction with Compound (1-3). Examples of a suitable solvent include THF, diethyl ether, dimethoxyethane, diethoxyethane, dichloromethane, toluene and the like, and THF and toluene are preferred. Examples of a suitable alkyllithium include n-butyllithium, sec-butyllithium, tert-butyllithium, methyllithium and the like, and n-butyllithium is preferred. The reaction can generally be carried out at about -78°C to about 25°C (room temperature) for about 10 minutes to about 2 hours, and preferably for about 1 hour.

The reaction converting Compound (14-5) to Compound (14-6) can be achieved by a reaction with a suitable acid catalyst in a suitable solvent, while carrying out deprotecting step. Examples of a suitable solvent include THF, dimethoxyethane, diethoxyethane, dichloromethane, toluene, methanol, ethanol, isopropanol and the like, and a mixed solvent of THF and methanol is preferred. Examples of a suitable acid catalyst include p-toluenesulfonic acid, pyridinium p-toluenesulfonic acid, methanesulfonic acid, trifluoromethanesulfonic acid, trifluoroacetic acid, camphorsulphonic acid, hydrochloric acid, sulfuric acid, acetic acid and the like, and p-toluenesulfonic acid is preferred. The reaction can generally be carried out at about -78°C to about 100°C, and preferably at about 0°C to about 60°C, for about 10 minutes to about 24 hours, and preferably for about 2 hours. Further, in this step the spiro moiety undergoes isomerization at the same time as cyclization, whereby the compound is obtained as a single product having a desired configuration.

The reaction converting Compound (14-6) to Compound (14-7) can be achieved by a reaction with a suitable oxidizing agent in a suitable solvent. Examples of a suitable solvent include dichloromethane, 1,2-dichloroethane, toluene, xylene and the like, and dichloromethane is preferred. Examples of a suitable oxidizing agent include a Dess-Martin reagent, TPAP-NMO, DMSO-acetic anhydride, DMSO-oxalyl chloride, manganese dioxide, chromic acid-sulfuric acid, SO₃-pyridine and the like, and manganese dioxide is preferred. The reaction can generally be carried out at about -78°C to about 40°C, and preferably at about 0°C to about 25°C (room temperature) for about 10 minutes to about 24 hours, and preferably for about 2 hours.

The reaction converting Compound (14-7) to Compound (14-8) can be achieved by a reaction with a suitable nucleophilic reagent in a suitable solvent. Examples of a suitable solvent include THF, dimethoxyethane, diethoxyethane, dioxane, N,N-dimethylformamide, N,N-dimethylacetamide, dichloromethane, 1,2-dichloroethane, toluene, xylene and the like, and N,N-dimethylformamide and N,N-dimethylacetamide are preferred. Examples of a suitable nucleophilic reagent include sodium thiomethoxide, sodium methoxide and the like. The reaction can generally be carried out at about 0°C to about 120°C, and preferably at about 0°C to about 25°C (room temperature) for about 10 minutes to about 5 hours, and preferably for about 30 minutes.

The reaction converting Compound (14-8) to Compound (14-10) can be achieved by a reaction with a suitable base and a suitable ethynylating agent in a suitable solvent. Examples of a suitable solvent include THF, dimethoxyethane, diethoxyethane, dioxane, dichloromethane, 1,2-dichloroethane, toluene, xylene, methanol, ethanol and the like, and a mixed solvent of THF and methanol is preferred. Examples of a suitable base include potassium carbonate, sodium carbonate, sodium hydroxide, potassium hydroxide and the like. Examples of a suitable ethynylating agent include dimethyl(1-diazo-2-oxopropyl)phosphonate (14-9). The reaction can generally be carried out at about 0°C to about 120°C, and preferably at about 0°C to about 25°C (room temperature) for about 10 minutes to about 5 hours, and preferably for about 30 minutes. Further, the ethynylating agent (14-9) can be synthesized according to a process described in a document, for example, Eur. J. Org. Chem., p. 821, 2003.

The reaction converting Compound (14-10) to Compound (14-11) can be achieved by a reaction with iodine in a suitable solvent. Examples of a suitable solvent include dichloromethane, 1,2-dichloroethane and the like, and dichloromethane is preferred. The reaction can generally be carried out at about -20°C to about 50°C, and preferably at about 0°C to about 25°C (room temperature), for about 10 minutes to about 4 hours, and preferably for about 15 minutes.

The reaction converting Compound (14-11) to Compound (14-13) can be achieved by a reaction with a suitable alkylating agent (14-12) in the presence of a suitable transition metal catalyst, a suitable ligand and a suitable base in a suitable solvent. Examples of a suitable solvent include THF, dimethoxyethane, diethoxyethane, dioxane, N,N-dimethylformamide, N,N-dimethylacetamide, DMSO, 1,2-dichloroethane, toluene, xylene, ethanol, acetonitrile and the like. Examples of a suitable transition metal catalyst include palladium, nickel, cobalt chloride, iron and the like. Examples of a suitable ligand include triphenylphosphine, tri(tert-butyl)phosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf) and the like. Examples of a suitable base include potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo[5,4,0]-7-undecene (DBU), 1,5-diazabicyclo-[4,3,0]-5-nonene (DBN), sodium tert-butoxide, potassium tert-butoxide, tetramethylguanidine and the like. Examples of a suitable alkylating agent include alkylboronic acid, alkylboronic acid dialkyl ester, an alkylmagnesium halide, dialkylzinc, alkyllithium, alkyltin, alkylsilane and the like. The reaction can generally be carried out at about 0°C to about 200°C, and preferably at about 25°C (room temperature) to about 100°C, for about 10 minutes to about 24 hours, and preferably for about 3 hours.

The reaction converting Compound (14-13) to Compound (14-14) wherein ring Ar is represented by Formula (a) can be achieved by a reaction with a suitable debenzylating reagent in a suitable solvent. Examples of a suitable solvent include THF, ethyl acetate, methanol, ethanol, dichloromethane and the like. Examples of a suitable debenzylating reagent include palladium on carbon and hydrogen gas, palladium hydroxide on carbon and hydrogen gas, Raney nickel and hydrogen gas, boron trichloride, boron tribromide, ethanethiol sodium salt, trimethylsilyl iodide and the like, and preferred examples are palladium on carbon and hydrogen gas, and boron trichloride. The reaction can generally be carried out at about -78°C to about 100°C, and preferably at about -78°C to room temperature, for about 1 hour to about 24 hours, and preferably for about 3 hours.

The compound wherein ring Ar is represented by Formula (b) can be produced according to the following Scheme 15: 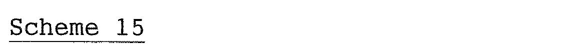 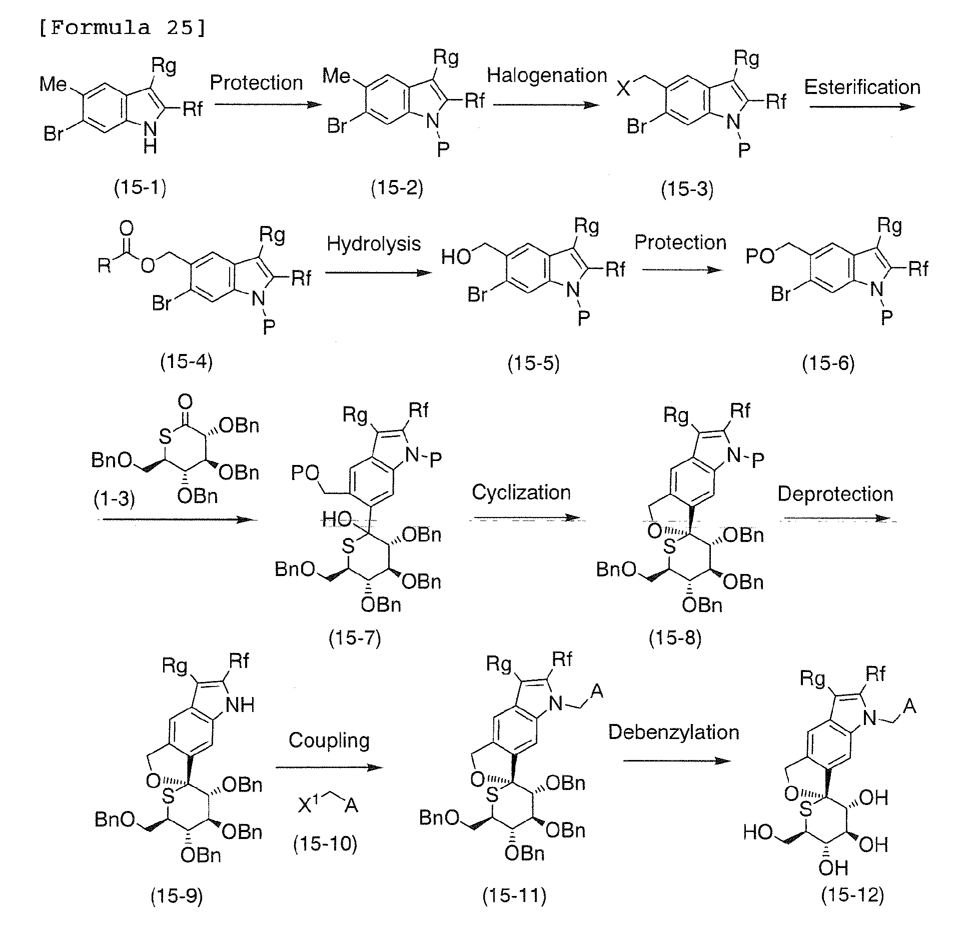 wherein A and X¹ are defined in the same manner as those described above; P represents a suitable protecting group; Rf and Rg each independently represents a hydrogen atom, a halogen atom or a C₁-C₆ alkyl group; R represents a C₁-C₆ alkyl group or aryl group; and X represents a halogen atom.

The reaction converting Compound (15-1) to Compound (15-2) can be achieved by a reaction with a suitable protecting group introducing reagent in a suitable solvent. Examples of a suitable solvent include THF, dimethoxyethane, diethoxyethane, dioxane, N,N-dimethylformamide, N,N-dimethylacetamide, DMSO, 1,2-dichloroethane, toluene, xylene, acetonitrile and the like. Examples of a suitable protecting group introducing reagent include benzenesulfonyl chloride, p-toluenesulfonyl chloride and the like, and benzenesulfonyl chloride is preferred. The reaction can generally be carried out at about 0°C to about 100°C, and preferably at about 0°C to about 25°C (room temperature), for about 10 minutes to about 24 hours, and preferably for about 1 hour. Further, Compound (15-1) can be synthesized according to the method described in a document, for example, Synlett, 10, p. 1594, 1999.

The reaction converting Compound (15-2) to Compound (15-3) can be achieved by a reaction with a suitable halogenating reagent in the presence of a suitable radical initiator in a suitable solvent. Examples of a suitable solvent include dichloromethane, 1,2-dichloroethane, carbon tetrachloride, benzene, nitrobenzene, heptane and the like, and carbon tetrachloride is preferred. Examples of a suitable radical initiator include 2,2'-azobis(isobutyronitrile) (AIBN), benzoyl peroxide, tert-butyl peroxide, triethylborane and the like, and AIBN is preferred. Examples of a suitable halogenating reagent include N-chlorosuccinimide (NCS), N-bromosuccinimide (NBS), 1,3-dichloro-5,5-dimethylhydantoin, 1,3-dibromo-5,5-dimethylhydantoin and the like, and NBS is preferred. The reaction can generally be carried out at about -78°C to about 100°C, and preferably at about 80°C, for about 10 minutes to about 12 hours, and preferably for about 1 hour.

The reaction converting Compound (15-3) to Compound (15-4) can be achieved by a reaction with a suitable metal salt of a carboxylic acid in a suitable solvent. Examples of a suitable solvent include THF, dimethoxyethane, diethoxyethane, dioxane, N,N-dimethylformamide, N,N-dimethylacetamide, dimethylsulfoxide (DMSO), 1,2-dichloroethane, toluene, xylene, acetonitrile and the like, and N,N-dimethylformamide is preferred. Examples of a suitable metal salt of a carboxylic acid include sodium acetate, potassium acetate, cesium acetate, sodium benzoate, potassium benzoate and the like, and sodium acetate is preferred. The reaction can generally be carried out at about 0°C to about 100°C, and preferably at about 80°C, for about 10 minutes to about 24 hours, and preferably for about 2 hours.

The reaction converting Compound (15-4) to Compound (15-5) can be achieved by a reaction with a suitable base in a suitable solvent. Examples of a suitable solvent include THF, dimethoxyethane, diethoxyethane, dioxane, 1,2-dichloroethane, toluene, xylene, acetonitrile, methanol, ethanol and the like, and methanol is preferred. Examples of a suitable base include lithium hydroxide, sodium hydroxide, potassium hydroxide, barium hydroxide, tetrabutylammonium hydroxide, potassium carbonate, sodium carbonate and the like, and potassium carbonate is preferred. The reaction can generally be carried out at 0°C to room temperature, and preferably at room temperature, for about 10 minutes to about 12 hours, and preferably for about 1 hour.

The reaction converting Compound (15-5) to Compound (15-6) can be achieved according to the same conditions as those in the above-described reaction in which Compound (14-4) was synthesized from Compound (14-3) in Scheme 14.

The reaction converting Compound (15-5) to Compound (15-6) can be achieved according to the same conditions as those in the above-described reaction in which Compound (14-5) was synthesized from Compound (14-4) in Scheme 14.

The reaction converting Compound (15-7) to Compound (15-8) can be achieved according to the same conditions as those in the above-described reaction in which Compound (14-6) was synthesized from Compound (14-5) in Scheme 14.

The reaction converting Compound (15-8) to Compound (15-9) can be achieved by a reaction with a suitable deprotecting reagent that is appropriate for the protecting group on the nitrogen atom in a suitable solvent. Examples of a suitable solvent include THF, dimethoxyethane, diethoxyethane, dioxane, N,N-dimethylformamide, N,N-dimethylacetamide, DMSO, 1,2-dichloroethane, toluene, xylene, acetonitrile, methanol, ethanol and the like, and a mixed solvent of THF and ethanol is preferred. Examples of a suitable deprotecting reagent include sodium hydroxide, potassium hydroxide and the like, and potassium hydroxide is preferred. The reaction can generally be carried out at about 0 to about 100°C, and preferably at about 25°C (room temperature) to about 50°C, for about 10 minutes to 24 hours, and preferably for about 3 hours.

The reaction converting Compound (15-9) to Compound (15-11) can be achieved by a reaction with a suitable base and a suitable benzyl halide derivative in a suitable solvent. Examples of a suitable solvent include THF, dimethoxyethane, diethoxyethane, dioxane, N,N-dimethylformamide, N,N-dimethylacetamide, DMSO, 1,2-dichloroethane, toluene, xylene, acetonitrile and the like, and N,N-dimethylformamide is preferred. Examples of a suitable base include sodium hydride, potassium hydride, sodium hydroxide, potassium hydroxide and the like, and sodium hydride is preferred. Examples of a suitable benzyl halide derivative include para-substituted benzyl bromide, para-substituted benzyl chloride, meta-substituted benzyl bromide, meta-substituted benzyl chloride, ortho-substituted benzyl bromide, ortho-substituted benzyl chloride and the like. The reaction can generally be carried out at about 0°C to about 100°C, and preferably at about 0°C to about 25°C (room temperature), for about 10 minutes to 12 hours, and preferably for about 2 hours.

The reaction converting Compound (15-11) to Compound (15-12) wherein ring Ar is represented by Formula (b) can be achieved according to the same conditions as those in the above-described reaction in which Compound (14-14) wherein ring Ar is represented by Formula (a) was synthesized from Compound (14-13) in Scheme 14.

The compound wherein ring Ar is represented by Formula (c) can also be produced according to a process of the following Scheme 16: 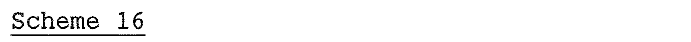 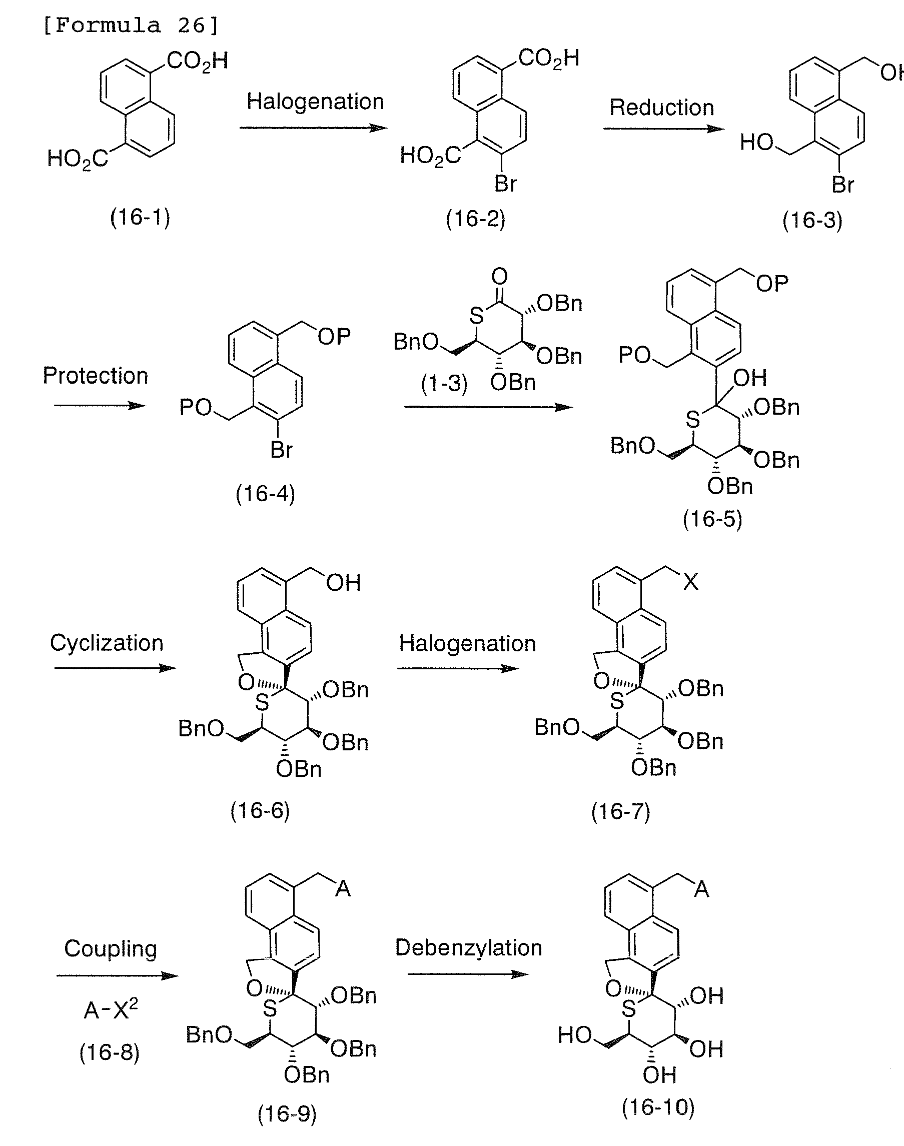 wherein A is defined in the same manner as described above; P represents a protecting group of a hydroxy group; X represents a halogen atom; and X² represents a boron atom, a silicon atom, a magnesium atom, a zinc atom, a tin atom or the like, each of which has a substituent(s).
The reaction converting Compound (16-1) to Compound (16-2) can be achieved by a reaction with a suitable base and a suitable halogenating agent in a suitable solvent. Examples of a suitable solvent include THF, dimethoxyethane, diethoxyethane, dioxane, 1,2-dichloroethane, toluene, xylene and the like. Examples of a suitable base include the combination of n-butyllithium and tert-butoxypotassium. Examples of a suitable halogenating agent include 1,2-dibromotetrachloroethane, bromine, iodine and the like. The reaction can generally be carried out at about -78°C to about 0°C, and preferably at about -78°C to about -50°C for about 1 hour to about 5 hours, and preferably for about 3 hours. Further, Compound (16-1) can be synthesized according to a process described in a document, for example, Bull. Chem., Soc. Jpn., 71, p. 1285, 1998. In addition, this reaction can be carried out with reference to a process described in Chem. Lett., 34, p. 446, 2005.

The reaction converting Compound (16-2) to Compound (16-3) can be achieved by reducing two carboxy groups with a suitable reducing agent in a suitable solvent. Examples of a suitable solvent include THF, diethyl ether, dimethoxyethane, diethoxyethane, dichloromethane, 1,2-dichloroethane, toluene, xylene and the like. Examples of a suitable reducing agent include the combination of sodium borohydride and a boranetrifluoride-diethyl ether complex, lithium aluminum hydride, diisopropylaluminum hydride, diborane, a borane-THF complex, a borane-dimethyl sulfide complex and the like, and a borane-THF complex is preferred. The reaction can generally be carried out at about -78°C to about 60°C, and preferably at about 0°C to about 25°C (room temperature), for about 10 minutes to about 24 hours, and preferably for about 2 hours.

The reaction converting Compound (16-3) to Compound (16-4) can be achieved according to the same conditions as those in the above-described reaction in which Compound (14-4) was synthesized from Compound (14-3)in Scheme 14.

The reaction converting Compound (16-4) to Compound (16-5) can be achieved according to the same conditions as those in the above-described reaction in which Compounds (14-5) was synthesized from Compound (14-4).

The reaction converting Compound (16-5) to Compound (16-6) can be achieved according to the same conditions as those in the above-described reaction in which Compound (14-6) was synthesized from Compound (14-5).

The reaction converting Compound (16-6) to Compound (16-7) can be achieved by a reaction with a suitable halogenating agent in a suitable solvent. Examples of a suitable solvent include dichloromethane, 1,2-dichloroethane, benzene, carbon tetrachloride and the like. Examples of suitable halogenating agents include carbon tetrachloride and triphenylphosphine, carbon tetrabromide and triphenylphosphine, sulfonyl chloride, oxalyl chloride, phosphorus trichloride, phosphorus tribromide and the like. The reaction can generally be carried out at about -20°C to about 50°C, and preferably at about 0°C to about 25°C (room temperature), for about 1 hour to about 24 hours, and preferably for about 2 hours.

The reaction converting Compound (16-7) to Compound (16-9) can be achieved by a reaction with an arylating agent (16-8) in the presence of a suitable transition metal catalyst, a suitable ligand and a suitable base in a suitable solvent. Examples of a suitable solvent include THF, dimethoxyethane, diethoxyethane, dioxane, N,N-dimethylformamide, N,N-dimethylacetamide, DMSO, 1,2-dichloroethane, toluene, xylene, ethanol, acetonitrile and the like. Examples of a suitable transition metal catalyst include palladium, nickel, cobalt, iron and the like. Examples of a suitable base include potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, DBU, DBN, sodium tert-butoxide, potassium tert-butoxide, tetramethylguanidine and the like. Examples of a suitable arylating agent include arylboronic acid, an arylmagnesium halide, arylzinc, aryllithium, aryltin, arylsilane and the like. The reaction can generally be carried out at about 0°C to about 200°C, and preferably at about 25°C (room temperature) to about 100°C, for about 10 minutes to about 24 hours, and preferably for about 2 hours.

The reaction converting Compound (16-9) to Compound (16-10) wherein ring Ar is represented by Formula (d) can be achieved according to the same conditions as those in the above-described reaction in which Compound (14-14) wherein ring Ar is represented by Formula (a) was synthesized from Compound (14-13) in Scheme 14.

The compound wherein ring Ar is represented by Formula (d) can also be produced according to a process of the following Scheme 17: 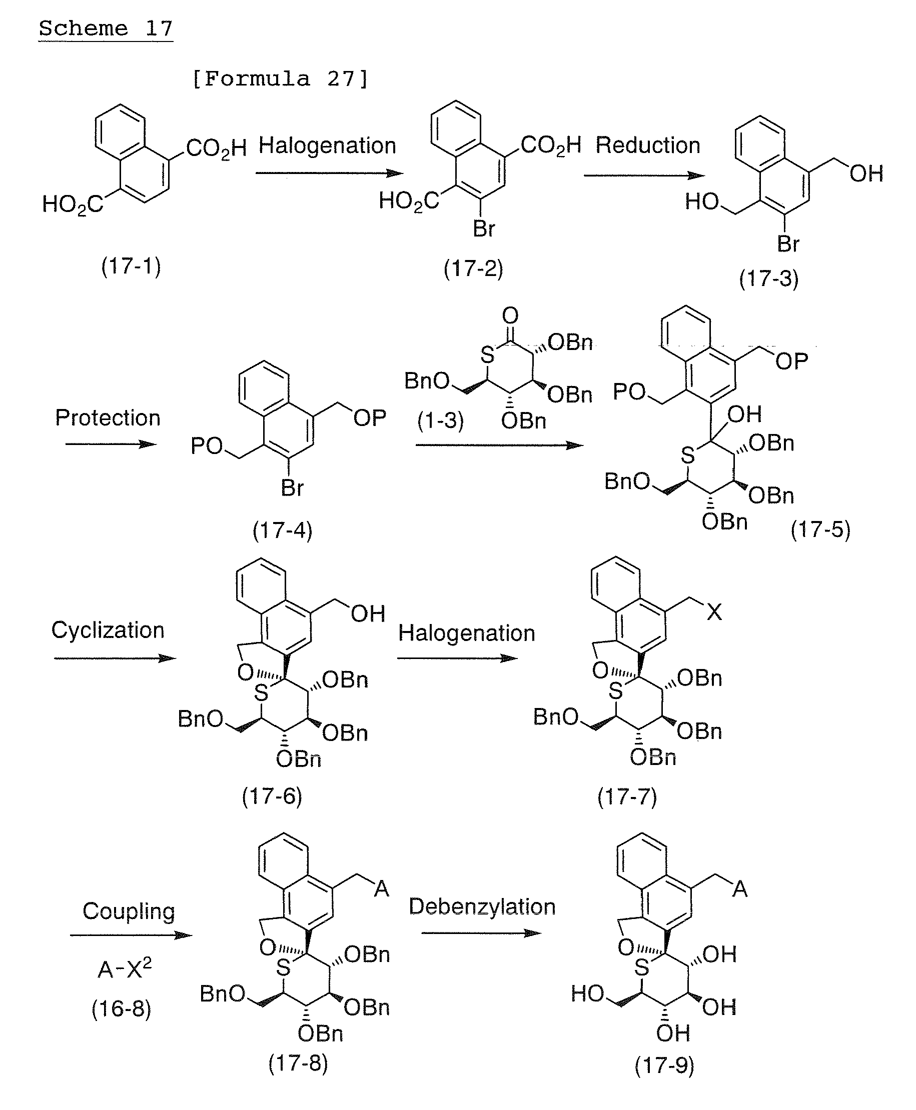 wherein A is defined in the same manner as described above; P represents a protecting group of a hydroxy group; X represents a halogen atom; and X² represents a boron atom, a silicon atom, a magnesium atom, a zinc atom, a tin atom or the like, each of which has a substituent(s).
The reaction converting Compound (17-1) to Compound (17-2) can be achieved according to the same conditions as those in the above-described reaction in which Compound (16-2) was synthesized from Compound (16-1) in Scheme 16.

The reaction converting Compound (17-2) to Compound (17-3) can be achieved according to the same conditions as those in the above-described reaction in which Compound (16-3) was synthesized from Compound (16-2) in Scheme 16.

The reaction converting Compound (17-3) to Compound (17-4) can be achieved according to the same conditions as those in the above-described reaction in which Compound (14-4) was synthesized from Compound (14-3) in Scheme 14.

The reaction converting Compound (17-4) to Compound (17-5) can be achieved according to the same conditions as those in the above-described reaction in which Compound (14-5) was synthesized from Compound (14-4) in Scheme 14.

The reaction converting Compound (17-5) to Compound (17-6) can be achieved according to the same conditions as those in the above-described reaction in which Compound (14-6) was synthesized from Compound (14-5) in Scheme 14.

The reaction converting Compound (17-6) to Compound (17-7) can be achieved according to the same conditions as those in the above-described reaction in which Compound (16-7) was synthesized from Compound (16-6) in Scheme 16.

The reaction converting Compound (17-7) to Compound (17-8) can be achieved according to the same conditions as those in the above-described reaction in which Compound (16-9) was synthesized from Compound (16-7) in Scheme 16.

The reaction converting Compound (17-8) to Compound (17-9) wherein ring Ar is represented by Formula (d) can be achieved according to the same conditions as those in the above-described reaction in which Compound (14-14) of the present invention wherein ring Ar is represented by Formula (a) was synthesized from Compound (14-13).

The compound of the present invention wherein ring Ar is represented by Formula (e) can also be produced according to a process of the following Scheme 18: 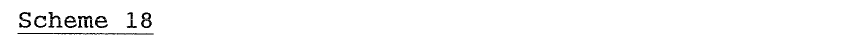 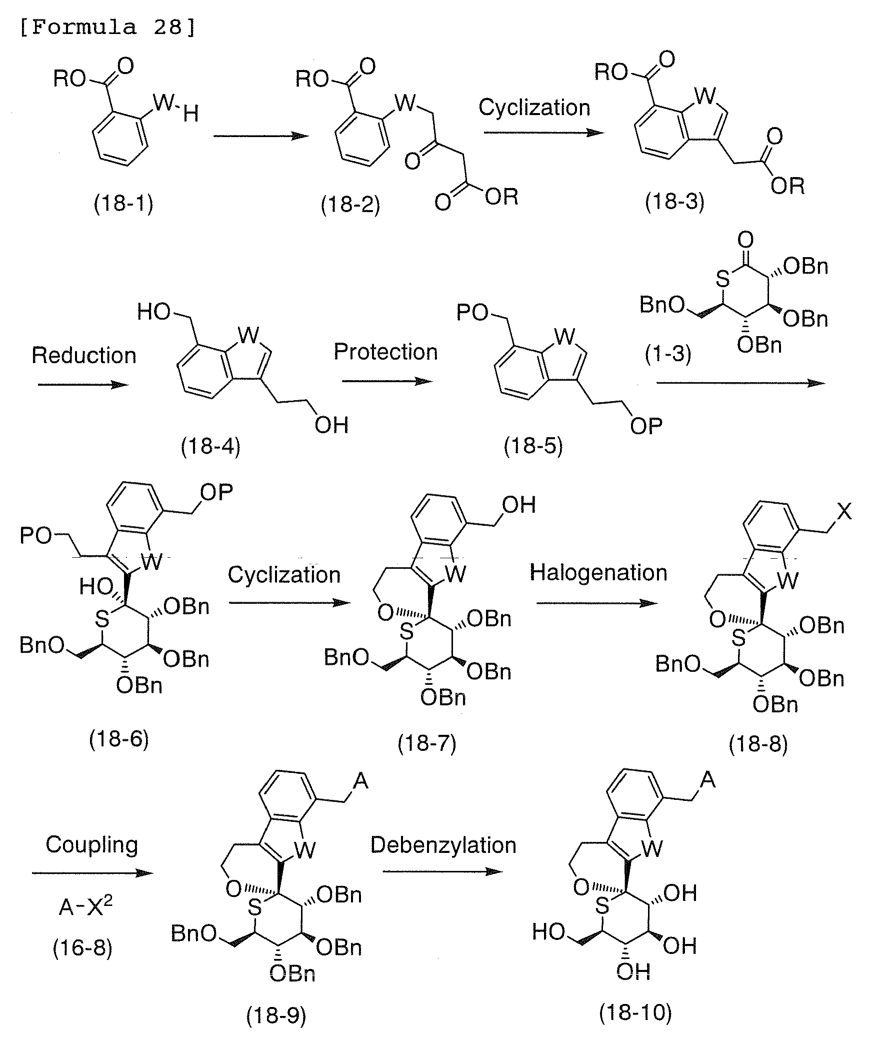 wherein A is defined in the same manner as described above; W represents a sulfur atom, an oxygen atom or a nitrogen atom; P represents a protecting group of a hydroxy group; R represents a methyl group or an ethyl group; and X² represents a boron atom, a silicon atom, a magnesium atom, a zinc atom, a tin atom or the like, each of which has a substituent(s).
The reaction converting Compound (18-1) to Compound (18-2) can be achieved by a reaction with a suitable alkyl halide in the presence of a suitable base in a suitable solvent. Examples of a suitable solvent include THF, dimethoxyethane, diethoxyethane, dioxane, N,N-dimethylformamide, N,N-dimethylacetamide, DMSO, 1,2-dichloroethane, toluene, xylene, acetonitrile and the like, and THF is preferred. Examples of a suitable base include triethylamine, diisopropylethylamine, pyridine, 4-(N,N-dimethylamino)pyridine, N-methylpiperidine, N-methylmorpholine and the like, and triethylamine is preferred. Examples of a suitable alkyl halide include methyl 4-chloroacetoacetate, ethyl 4-chloroacetoacetate and the like. The reaction can generally be carried out at about -20°C to about 100°C, and preferably at about 0°C to about 25°C (room temperature), for about 10 minutes to 12 hours, and preferably for about 2 hours.

The reaction converting Compound (18-2) to Compound (18-3) can be achieved by a reaction with a suitable acid in a suitable solvent or in the absence of a solvent. Examples of a suitable solvent include dichloromethane, 1,2-dichloroethane, nitrobenzene, chlorobenzene and the like. Examples of a suitable acid include polyphosphoric acid, trifluoroacetic acid, methanesulfonic acid, trifluoromethanesulfonic acid, sulfuric acid, phosphoric acid, aluminum trichloride, titanium tetrachloride and the like, and polyphosphoric acid is preferred. The reaction can generally be carried out at about -78°C to about 100°C, and preferably at about 0°C to about 80°C, for about 1 hour to about 12 hours, and preferably for about 1 hour.

The reaction converting Compound (18-3) to Compound (18-4) can be achieved by a reaction with a suitable reducing agent in a suitable solvent. Examples of a suitable solvent include THF, diethyl ether, dimethoxyethane, diethoxyethane, dioxane, dichloromethane, 1,2-dichloroethane, toluene, xylene, methanol, ethanol and the like, and THF is preferred. Examples of a suitable reducing agent include sodium borohydride, lithium borohydride, lithium aluminum hydride, diisopropylaluminum hydride and the like, and lithium aluminum hydride is preferred. The reaction can generally be carried out at about -78°C to about 50°C, and preferably at about 0°C to about 25°C (room temperature), for about 1 minute to 1 hour, and preferably for about 10 minutes.

The reaction converting Compound (18-4) to Compound (18-5) can be achieved according to the same conditions as those in the above-described reaction in which Compound (14-4) was synthesized from Compound (14-3) in Scheme 14.

The reaction converting Compound (18-5) to Compound (18-6) can be achieved according to the same conditions as those in the above-described reaction in which Compound (14-5) was synthesized from Compound (14-4) in Scheme 14.

The reaction converting Compound (18-6) to Compound (18-7) can be achieved according to the same conditions as those in the above-described reaction in which Compound (14-6) was synthesized from Compound (14-5) in Scheme 14.

The reaction converting Compound (18-7) to Compound (18-8) can be achieved according to the same conditions as those in the above-described reaction in which Compound (16-7) was synthesized from Compound (16-6) in Scheme 16.

The reaction converting Compound (18-8) to Compound (18-9) can be achieved according to the same conditions as those in the above-described reaction in which Compound (16-7) was synthesized from Compound (16-9) in Scheme 16.

The reaction converting Compound (18-9) to Compound (18-10) wherein ring Ar is represented by Formula (e) can be achieved according to the same conditions as those in the above-described reaction in which Compound (14-14) wherein ring Ar is represented by Formula (a) was synthesized from Compound (14-13) in Scheme 14.

The process for producing the compounds of the present invention is not limited to the methods described above. The compounds of the invention can be synthesized, for example, by combining steps included in Schemes 1 to 18 appropriately.

### [Examples]

The subject matter of the present invention will now be described in more detail with reference to the following examples and test examples. However, the present invention shall not be limited to such subject matter.

In the following examples, the respective abbreviations have the following meaning: NMR: Nuclear magnetic resonance spectrum (TMS internal standard); MS: mass spectrometry value.

The NMR and HPLC were carried out using the following equipments.

NMR: JEOL JNM-EX-270 (270 MHz), Varian Mercury 300 (300 MHz), or JEOL JNM-ECP400 (400 MHz).
MS: LCQ manufactured by Thermo Finigan or Micromass ZQ manufactured by Waters.

### Example 1

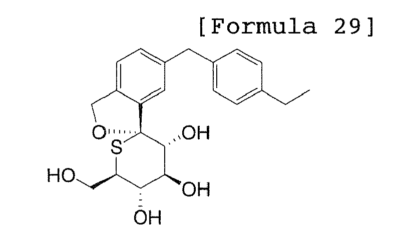

### (1S,3'R,4'S,5'S,6'R)-6-[(4-ethylphenyl)methyl]-3',4',5',6'-tetrahydro-6'-(hydroxymethyl)-spiro[isobenzofuran-1(3H),2'-[2H]thiopyran]-3',4',5'-triol

### 1) Synthesis of 2-bromo-1,4-bis(1-methoxy-1-methyl-ethoxymethyl)benzene

Under nitrogen stream, to a solution of (2-bromo-4-hydroxymethyl-phenyl)-methanol (3.22g, 14.83mmol), p-toluenesulfonic acid pyridium (93mg, 0.37mmol) in THF (16 ml) was added 2-methoxypropene (4.26ml, 44.48mmol) under ice cooling, and the mixture was stirred at the same temperature for one hour. A saturated aqueous solution of potassium carbonate was added thereto and the resulting mixture was extracted with hexane. The organic layer was washed with saturated aqueous solution of NaCl, dried over anhydrous magnesium sulfate, and a solvent was removed under reduced pressure to give the title compound (5.28 g, 99%).

¹H-NMR (CDCl₃) d: 1.42 (6H, s), 1.44 (6H, s), 3.23 (3H, s), 3.24 (3H, s), 4.44 (2H, s), 4.52 (2H, s), 7.25-7.28 (1H, m), 7.47-7.53 (2H, m).

### 2) Synthesis of (1S,3'R,4'S,5'S,6'R)-3',4',5'-tris(benzyloxy)-6'-(benzyloxymethyl)-3',4',5',6'-tetrahydro-6-(hydroxymethyl)-spiro[isobenzofuran-1(3H),2'-[2H]thiopyran]

Under nitrogen stream, to a solution of 2-bromo-1,4-bis-(1-methoxy-1-methyl-ethoxymethyl)-benzene (39 mg, 0.11 mmol) in toluene (0.44ml) was added dropwise n-butyllithium in hexane (2.67 M, 40 µl, 0,11mmol) at room temperature, and the mixture was stirred for 20 minutes. The mixture was added dropwise to a solution of (3R,4S,5S,6R)-3,4,5-tris-benzyloxy-6-(benzyloxymethyl)-tetrahydro-thiopyran-2-one (43 mg, 0.08 mmol) in toluene (0.44ml) at -78 °C, and then the resulting mixture was stirred at the same temperature for 30 minutes. A saturated aqueous solution of ammonium chloride was added thereto and the mixrure was extracted with ethyl acetate. The organic layer was washed with saturated aqueous solution of NaCl, dried over anhydrous magnesium sulfate, and a solvent was removed under reduced pressure. To the resulting residue was added THF (0.12ml), methanol (0.08ml) and p-toluenesulfonic acid (3.5mg, 0.02mmol) and the mixture was stirred for 3 hours. A saturated aqueous solution of sodium hydrogen carbonate was added thereto and the mixture was extracted with ethyl acetate. The organic layer was dried over anhydrous magnesium sulfate, and a solvent was removed under reduced pressure. The resulting residue was purified with silicagel column chromatography (eluting solvent = ethyl acetate : n-hexane (1:5)) to give the title compound (12.5 mg, 24%).

¹H-NMR (CDCl₃) d: 3.45-3.50 (1H, m), 3.59-3.63 (1H, m), 3.91-3.96 (1H, m), 4.02-4.08 (2H, m), 4.18-4.21 (2H, m), 4.49 (2H, dd, J=12.76, 14.27Hz), 4.60-4.67 (4H, m), 4.88-4.96 (3H, m), 5.22 (2H, dd, J=12.35, 20.31Hz), 6.63-6.66 (2H, m), 7.03-7.32 (21H, m).

### 3) Synthesis of (1S,3'R,4'S,5'S,5'R)-3',4',5'-tris(benzyloxy)-6'-(benzyloxymethyl)-3',4',5',6'-tetrahydro-6-(methoxycarbonyloxymethyl)-spiro[isobenzofuran-1(3H),2'-[2H]thiopyran]

Under nitrogen stream, to a solution of (1S,3'R,4'S,5'S,6'R)-3', 4', 5'-tris (benzyloxy)-6'-(benzyloxymethyl)-3',4',5',6'-tetrahydro-6-(hydroxymethyl)-spiro[isobenzofuran-1(3H),2'-[2H]thiopyran] (12.5 mg, 0.02 mmol) in methylene chloride (0.3 ml) was added 4-dimethylaminopyridine (29 mg, 0.24 mmol), methyl chloroformate (17 µl, 0.22 mmol) at 0°C, and the mixture was stirred for 2.5 hours. A 10% aqueous solution of potassium hydrogen sulfate was added thereto and the mixture was extructed with ethyl acetate. The organic layer was washed with saturated aqueous solution of sodium hydrogen carbonate, dried over anhydrous magnesium sulfate, and a solvent was removed under reduced pressure. The resulting residue was purified by silicagel column chromatography (eluting solvent = methylene chloride : n-hexane (1:1)) to give the title compound (12.8 mg, 94%).

¹H-NMR (CDCl₃) d: 3.45-3.48 (1H, m), 3.59-3.63 (4H, m), 3.91-4.22 (5H, m), 4.45-4.66 (4H, m), 4.89-5.26 (7H, m), 6.58-6.61 (2H, m), 7.02-7.37 (21H, m).

### 4) Synthesis of (1S,3'R,4'S,5'S,6'R)-3',4',5'-tris(benzyloxy)-6'-(benzyloxymethyl)-6-[(4-ethylphenyl)methyl]-3',4',5',6'-tetrahydro-spiro[isobenzofuran-1(3H),2'-[2H]thiopyran]

Under nitrogen stream, to a mixture of (1S,3'R,4'S,5'S,6'R)-3',4',5'-tris(benzyloxy)-6'-(benzyloxymethyl)-3',4',5',6'-tetrahydro-6-(methoxycarbonyloxymethyl)-spiro[isobenzofuran-1(3H),2'-[2H]thiopyran] (734 mg, 1.002 mmol), ethylphenylboronic acid (210mg.1.400mmol), potassium carbonate (138 mg,0.998 mmol), palladium acetate (II) (45 mg, 0.200 mmol), 1,1'-bis(diphenylphosphino)ferrocene (133 mg, 0.24 mmol), and 1,2-dimethoxyethane (2.00 ml) was stirred at 83°C for 3.5 hours. After cooling to room temperature, the mixture was purified by silicagel flash column chromatography (eluting solvent = ethyl acetate : n-hexane (1:3)) to give the title compound (660 mg, 87%).

¹H-NMR (CDCl₃) d: 1.12 (3H, t, J=7.69Hz), 2.53 (2H, q, J=7.69Hz), 3.43-3.47 (1H, m), 3.60 (1H, dd, J=2.74, 9.88Hz), 3.91-4.14 (7H, m), 4.45-4.65 (4H, m), 4.85-4.95 (3H, m), 5.14-5.23 (2H, m), 6.58 (2H, dd, J=1.37, 7.96Hz), 6.92-7.32 (25H, m).

### 5) Synthesis of (1S,3'R,4'S,5'S,6'R)-6-[(4-ethylphenyl)methyl]-3',4',5',6'-tetrahydro-6'-(hydroxymethyl)-spiro[isobenzofuran-1(3H),2'-[2H]thiopyran]-3',4',5'-triol

Under nitrogen stream, to a solution of (1S,3'R,4'S,5'S,6'R)-3',4',5'-tris(benzyloxy)-6'-(benzyloxymethyl)-6-[(4-ethylphenyl)methyl]-3',4',5',6'-tetrahydro-spiro[isobenzofuran-1(3H),2'-[2H]thiopyran] (140 mg, 0.183 mmol) and pentamethylbenzene (270 mg, 1.82 mmol) in methylene chloride (10 ml) was added, at -78 °C, a solution of boron trichloride in methylene chloride (1.0 M, 1.8 ml, 1.8 mmol), and the mixture was stirred at the same temperature for 2 hours. Methanol (10ml) was added thereto, and then the mixture was warmed to room temperature. A saturated aqueous solution of sodium hydrogen carbonate was added and the mixture was extracted with ethyl acetate. The organic layer was dried over anhydrous potassium carbonate, and solvent was removed under reduced pressure. The resulting residue was purified by silicagel column chromatography (eluting solvent = methylene chloride : methanol (9:1)) to give the title compound (25 mg, 34%).

¹H-NMR (CD₃OD) d: 1.19 (3H, t, J=7.69Hz), 2.59 (2H, q, J=7.69Hz), 3.18-3.22 (1H, m), 3.67-3.70 (2H, m), 3.78-4.01 (5H, m), 5.11 (2H, s), 7.03-7.16 (7H, m).

MS (ESI⁺) : 403 [M+1]⁺.

The compounds listed in Tables 1-1 to 1-6 can be easily produced in the same manner as described in Example or in the production processes indicated above, or by applying slight modifications to such processes that would be obvious to a person skilled in the art.

[Table 1-1]

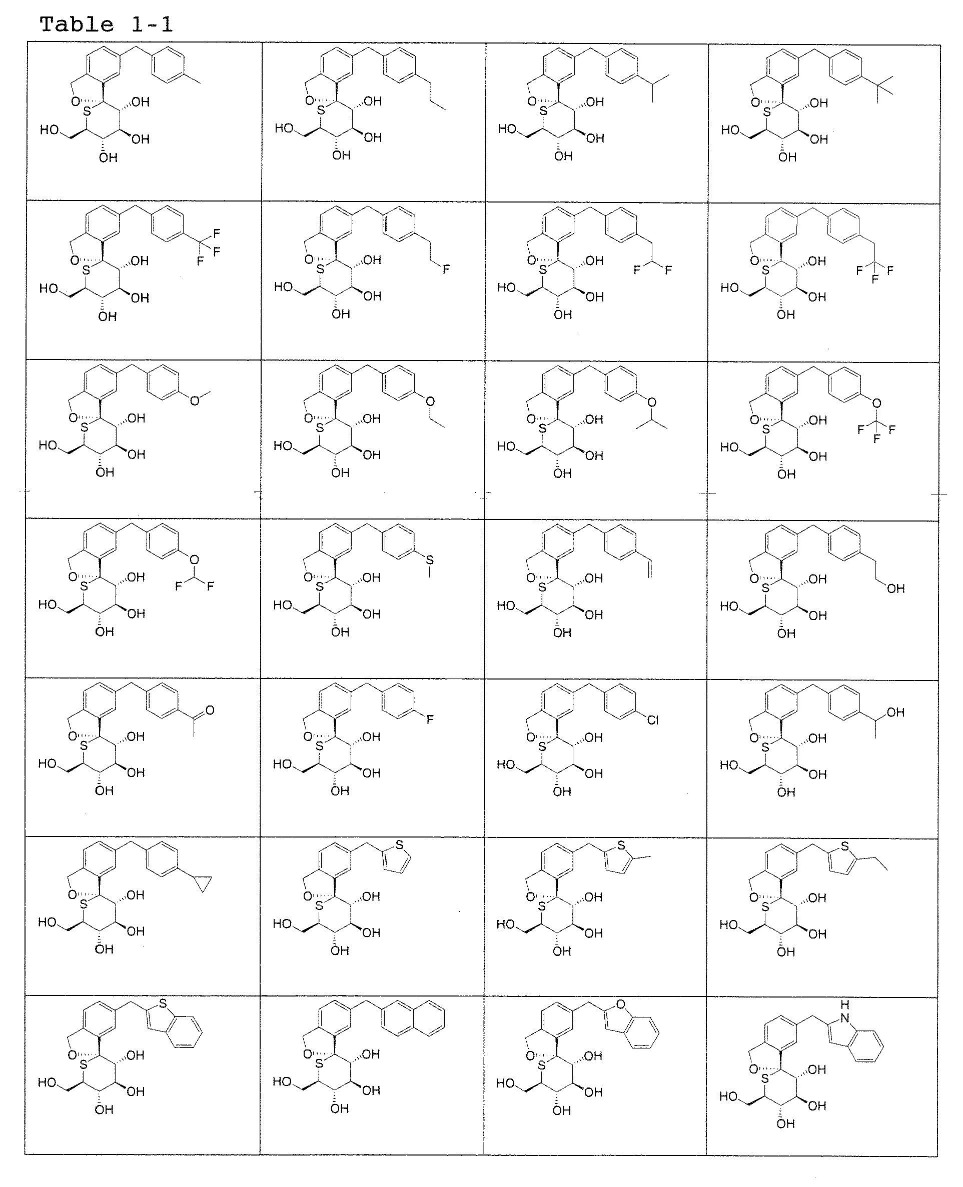

[Table 1-2]

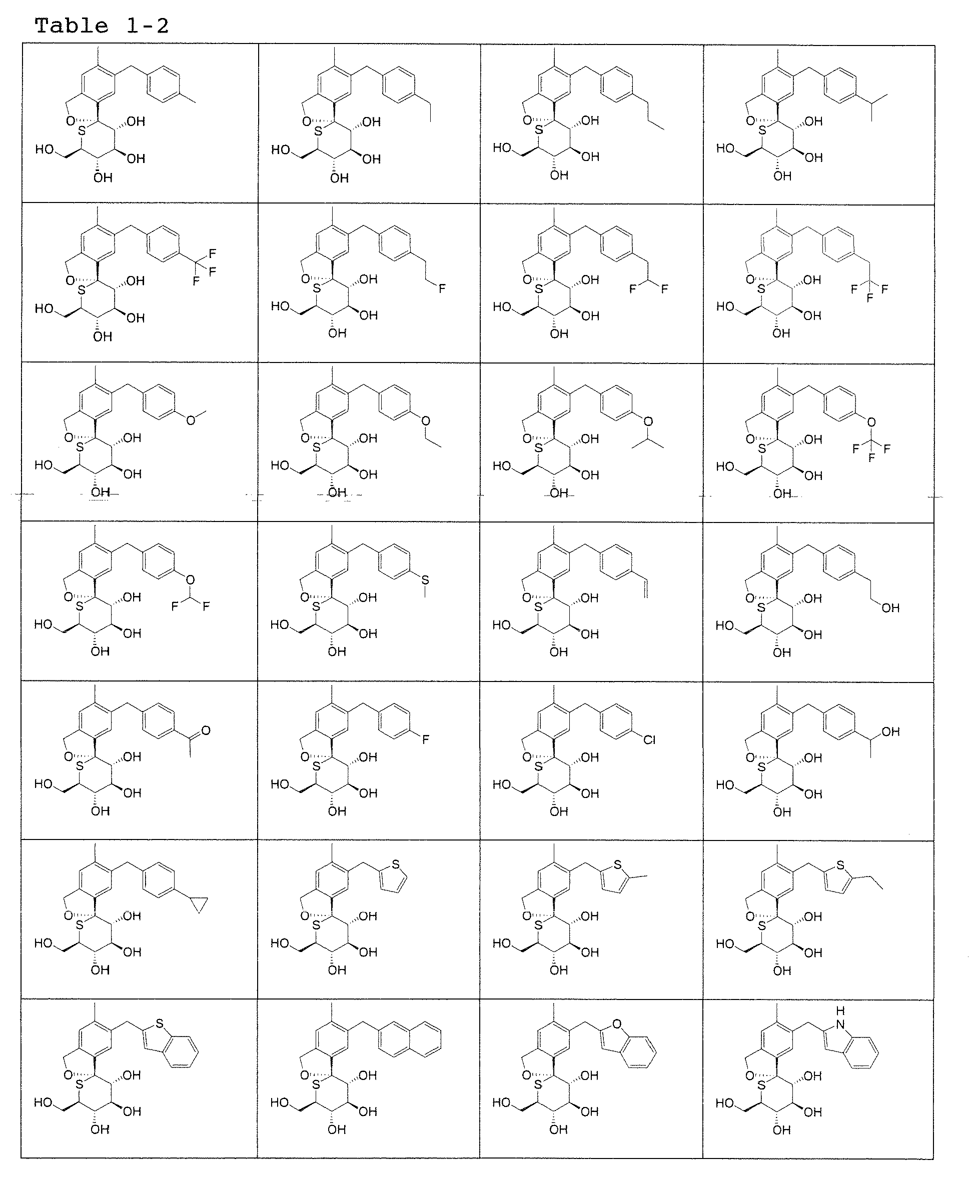

[Table 1-3]

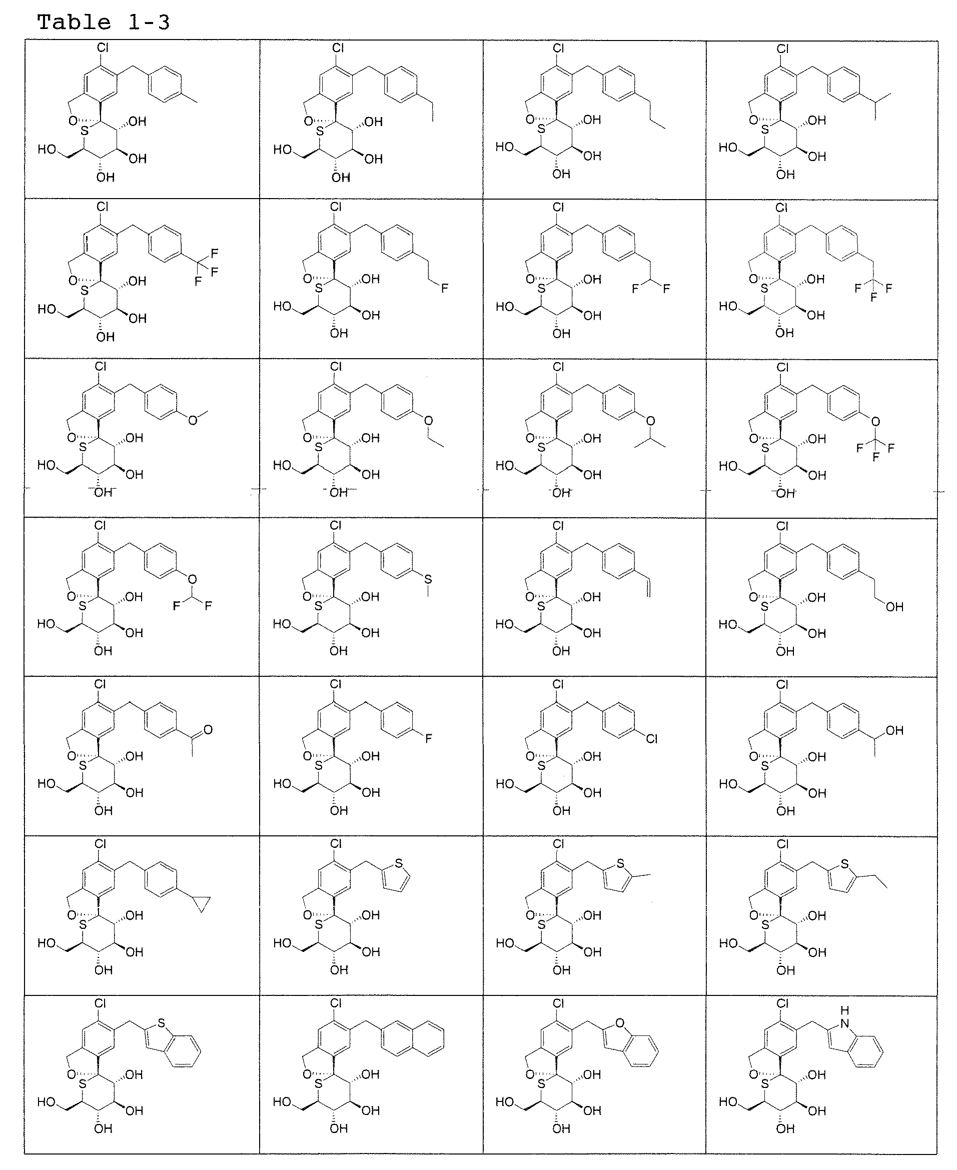

[Table 1-4]

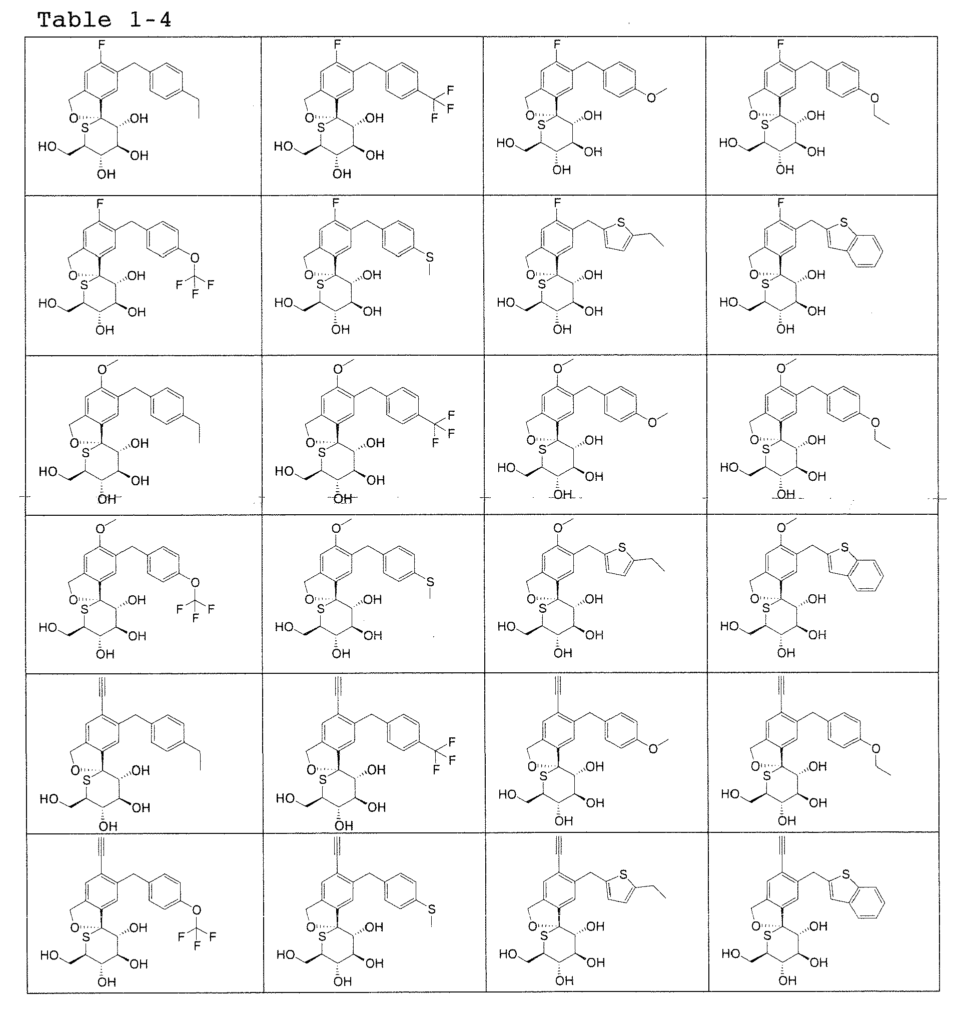

[Table 1-5]

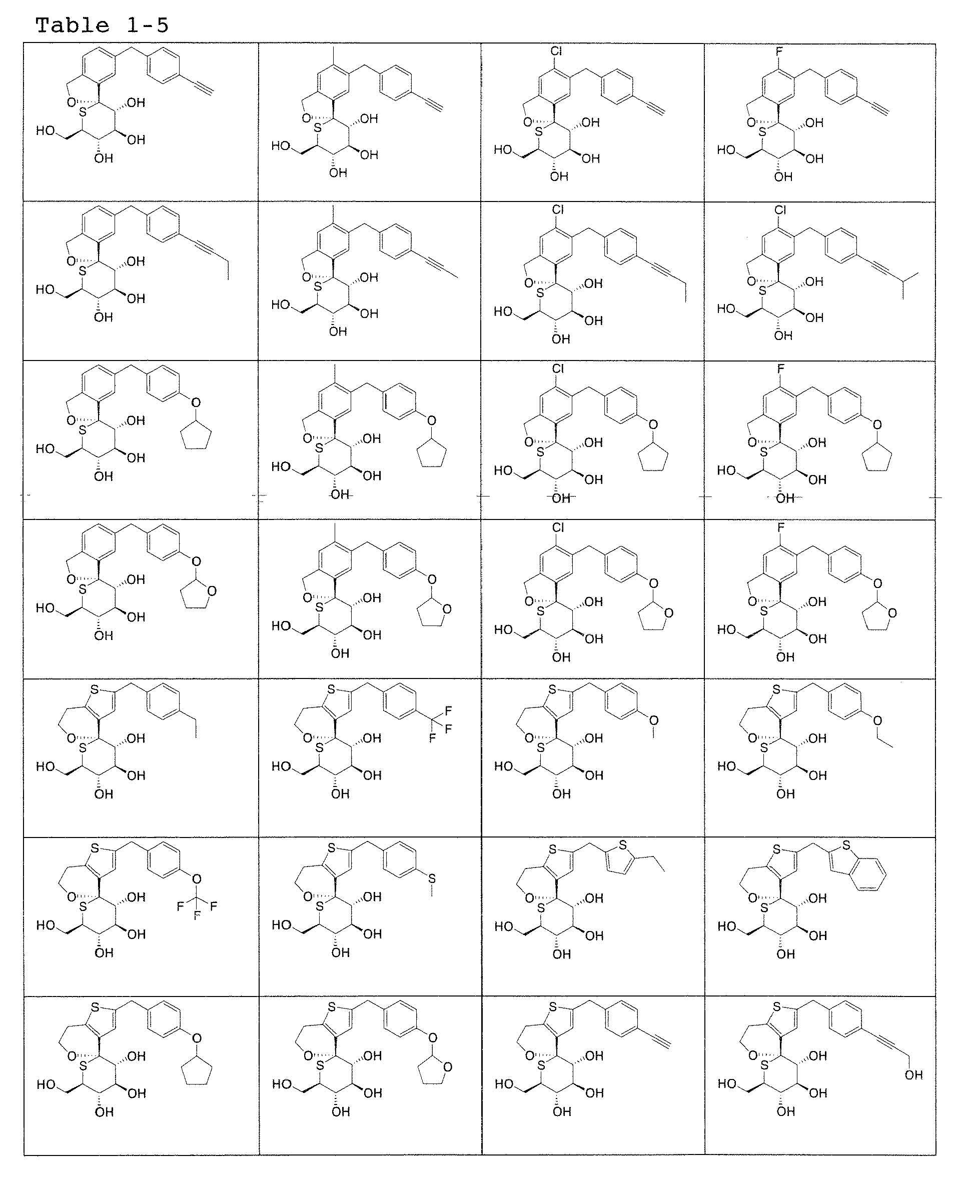

[Table 1-6]

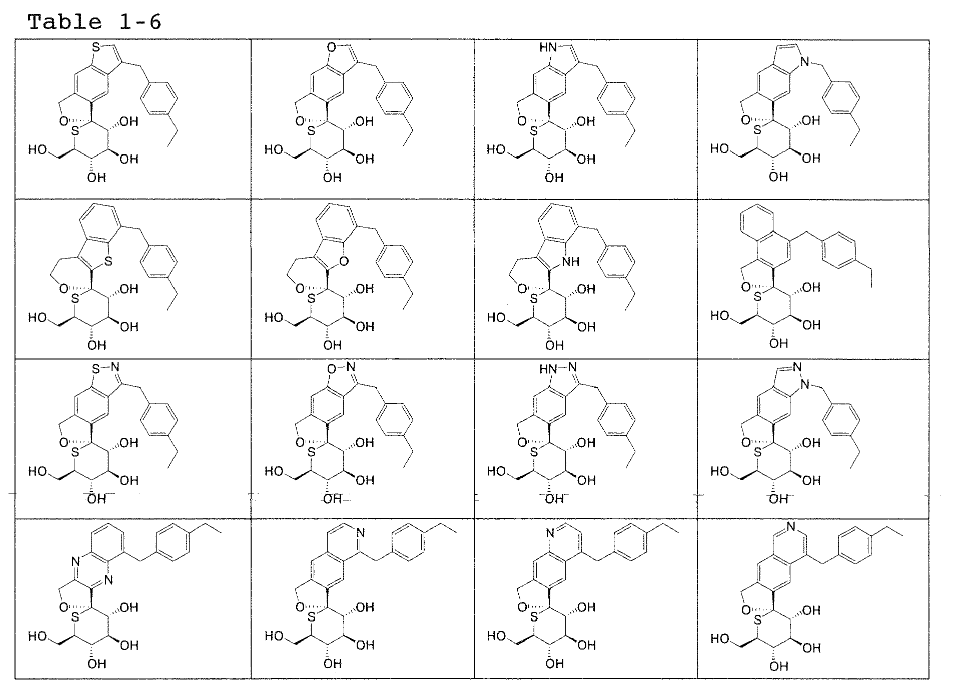

### Test Example 1

### Assay for evaluating inhibition of activity of human Na⁺-glucose cotransporter (SGLT1 and SGLT2)

### 1) Construction of human SGLT1 expression vector

Human SGLT1 cDNA was amplified by PCR with a cDNA library derived from human small intestine (Clontech) as a template, synthetic DNA primers, and KOD+ DNA Polymerase (Toyobo Co., Ltd., Japan). The amplified cDNA was inserted into pcRII-Topo vector by using a Topo TA Cloning Dual Promoter kit (Invitrogen). *E. coli* competent cells (Invitrogen, TOP10) were transformed with the plasmid vector, cultured in LB medium containing ampicillin (50 mg/L) to grow ampicillin-resistant clones. The plasmid vector containing human SGLT1 cDNA was purified from the clone in a standard manner (see Maniatis et al., Molecular Cloning). Human SGLT1 cDNA added restriction enzyme recognition sites (*Eco RI* at 5'-end, Hind *III* at 3'-end) was amplified by PCR with the plasmid vector as a template, synthetic DNA primers containing an additional restriction enzyme recognition site, and KOD+ DNA Polymerase. This amplified cDNA was digested with Eco RI and Hind III and the resulting fragment was ligated into expression vector pcDNA3.1(-) (Invitrogen) digested with *Xho I* and Hind *III* by a Rapid DNA Ligation kit (Roche Diagonostics). *E. coli* competent cells (Invitrogen, DH5α) were transformed with the ligated expression vector and grown in ampicillin-containing LB medium. Human SGLT1 expression vector was purified from the ampicillin-resistant clone in a standard manner.

### 2) Construction of human SGLT2 expression vector

Human SGLT2 cDNA was amplified by PCR with a cDNA library derived from human kidney (Clontech) as a template, synthetic DNA primers, and KOD+ DNA Polymerase. The amplified cDNA was inserted into pcRII-Topo vector by using a Topo TA Cloning Dual Promoter kit. *E. coli* competent cells (TPO10) were transformed with the plasmid vector, and then cultured in LB medium containing ampicillin (50 mg/L) to grow ampicillin-resistant clones. The plasmid vector containing human SGLT2 cDNA was purified from the clone in a standard manner. Human SGLT2 cDNA added restriction enzyme recognition sites (*Xho I* at 5'-end, Hind *III* at 3'-end) was amplified by PCR with the plasmid vector as a template, synthetic DNA primers containing an additional restriction enzyme recognition site, and KOD+ DNA Polymerase. This amplified cDNA was digested with *Xho I* and Hind *III,* and the resulting fragment was ligated into expression vector pcDNA3.1(-) digested with *Xho I* and Hind *III* by using a Rapid DNA Ligation kit. *E. coli* competent cells (DH5α) were transformed with the ligated expression vector and grown in ampicillin-containing LB medium. Human SGLT2 expression vector was purified from the ampicillin-resistant clone in a standard manner.

### 3) Establishment of cell lines stably expressing human SGLT1 or human SGLT2

The human SGLT1 expression vector or the human SGLT2 expression vector was digested with the restriction enzyme Pvu I and transfected into CHO-K1 cells with FuGENE (Roche Diagonostics). After the transfection, the cells were cultured at 37°C in the presence of 5% CO₂ for about 3 weeks in DMEM medium (Gibco) containing penicillin (50 U/mL, SIGMA), streptomycin (50 mg/L, SIGMA), geneticin (200 mg/L, Nacalai Tesque, Inc., Japan) and 20% fetal bovine serum to obtain geneticin-resistant clones. Among these clones, clones stably expressing human SGLT1 or human SGLT2 were selected by evaluating the sodium-dependent uptake activity of sugar (methyl-α-D-glucopyranoside).

### 4) Evaluation of inhibitory activity against methyl-α-D-glucopyranoside uptake

CHO Cell lines stably expressing human SGLT1 or human SGLT2 were seeded in 96-well culture plates at a density of 30000 to 40000 cells/well and cultured for 4 to 6 days. The medium in these plates was removed and replaced by 150 µL/well pretreatment buffer (i.e., a buffer containing 140 mM choline chloride, 2 mM potassium chloride, 1 mM calcium chloride, 1 mM magnesium chloride, 10 mM 2-[4-(2-hydroxyethyl)-1-piperazinyl]ethanesulfonic acid and tris(hydroxymethyl)aminomethane, pH 7.4), and the plates were incubated at 37°C for 20 minutes. The pretreatment buffer in the plates was removed, replaced by 50 µL/well fresh pretreatment buffer, and the plates were incubated at 37°C for 20 minutes. Methyl-α-D-(U-¹⁴C)glucopyranoside (6.3 mL, Amersham Pharmacia Biotech, 200 mCi/L) was added to and mixed with 100 mL buffer (i.e., a buffer containing 140 mM sodium chloride, 2 mM potassium chloride, 1 mM calcium chloride, 1 mM magnesium chloride, 1 mM methyl-α-D-glucopyranoside, 10 mM [4-(2-hydroxyethyl)-1-piperazinyl]ethanesulfonic acid and tris(hydroxymethyl)aminomethane, pH 7.4), which was used as uptake buffer. Test compounds were dissolved into uptake buffer and these test compound solutions were used for evaluating inhibitory activity. Uptake buffer without a test compound was used as a control solution. Moreover, for use in measuring baseline uptake in the absence of sodium, sodium-free solution was prepared in the same manner to contain 140 mM choline chloride instead of sodium chloride. The pretreatment buffer was removed from each well of the plates and replaced by 35 µL/well test compound solutions, and the plates were incubated at 37°C for 45 minutes. The solutions were removed and replaced by 300 µL/well washing buffer (i.e., a buffer containing 140 mM choline chloride, 2 mM potassium chloride, 1 mM calcium chloride, 1 mM magnesium chloride, 10 mM methyl-α-D-glucopyranoside, 10 mM 2-[4-(2-hydroxyethyl)-1-piperazinyl]ethanesulfonic acid and tris(hydroxymethyl)aminomethane, pH 7.4). The washing buffer was removed immediately. This washing procedure was repeated once again, and a cell lysis solution (1 M sodium hydroxide, 0.1% sodium lauryl sulfate) was added in a volume of 30 µL/well to solubilize the cells. 2 M hydrochloric acid (15 µL) was added to the cell lysate in each well, and 40 µL of the resulting solution was transferred to a LumaPlate (Packard). The LumaPlate were left overnight at room temperature to evaporate the solvent. The samples on the plate were measured for their radioactivity with a TopCount NXT(Packard). Assuming that the value obtained by subtracting the baseline uptake level from the uptake level of the control sample was set to 100%, the concentration required for a test compounds to cause 50% inhibition of the uptake level (IC₅₀ value) were calculated from the concentration-dependent inhibition curve using ELfit ver.3. As a result, the compounds of the present invention were found to show a remarkable inhibitory effect on SGLT2. The IC₅₀ value for the inhibition of SGLT2 of the compounds prepared in Example 1 is 12 nM.

### INDUSTRIAL APPLICABILITY

The present invention provides thioglucose spiro-compounds exhibiting excellent inhibition effect on SGLT2 activity or prodrugs or pharmacologically acceptable salts thereof. The compounds of the present invention are useful as preventive or therapeutic drugs for diabetes, diabetes-related diseases or diabetic complications.

## Claims

1. A compound represented by Formula (II): 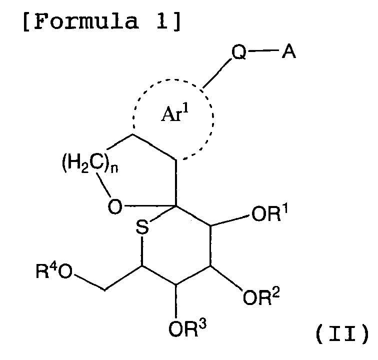 wherein R¹, R², R³, and R⁴ are each independently selected from a hydrogen atom, a C₁-C₆ alkyl group which may be substituted with one or more Ra, a C₇-C₁₄ aralkyl group which may be substituted with one or more Rb, and -C(=O)Rx;
Rx is a C₁-C₆ alkyl group which may be substituted with one or more Ra, an aryl group which may be substituted with one or more Rb, a heteroaryl group which may be substituted with one or more Rb, a C₁-C₆ alkoxy group which may be substituted with one or more Ra, or -NReRf;
Ar¹ is an aromatic carbocyclic ring which may be substituted with one or more Rb, or an aromatic heterocyclic ring which may be substituted with one or more Rb, in which the aromatic carbocyclic ring and the aromatic heterocyclic ring may form a condensed ring;
Q is -(CH₂)ₘ-(L)ₚ- or -(L)ₚ-(CH₂)ₘ-:
m is an integer selected from 0 to 2, n is an integer selected from 1 and 2, and p is an integer selected from 0 and 1;
L is -O-, -S- or -NR⁵-,
R⁵ is selected from a hydrogen atom, a C₁-C₆ alkyl group which may be substituted with one or more Ra, and -C(=O)Rx;
A is an aryl group which may be substituted with one or more Rb or a heteroaryl group which may be substituted with one or more Rb, wherein the aryl group or the heteroaryl group may form a condensed ring together with an aromatic carbocyclic ring or an aromatic heterocyclic ring;
Ra is each independently selected from a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRgRh, a C₁-C₆ alkoxycarbonyl group which may be substituted with one or more Rc, and a C₁-C₆ alkylcarbonyl group which may be substituted with one or more Rc;
Rb is each independently selected from a C₁-C₆ alkyl group which may be substituted with one or more Rc, a C₃-C₈ cycloalkyl group which may be substituted with one or more Rc, a C₂-C₆ alkenyl group which may be substituted with one or more Rc, a C₂-C₆ alkynyl group which may be substituted with one or more Rc, a C₇-C₁₄ aralkyl group which may be substituted with one or more Rd, a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRiRj, a C₁-C₆ alkylcarbonyl group which may be substituted with one or more Rc, a C₁-C₆ alkoxycarbonyl group which may be substituted with one or more Rc, a C₁-C₃ alkylenedioxy group, a heterocyclyl group, and a heterocyclyloxy group;
Rc is each independently selected from a halogen atom, a hydroxy group, a cyano group, a nitro group, a carboxy group, a C₁-C₆ alkoxy group, an aryl group which may be substituted with one or more Rd, an aryloxy group which may be substituted with one or more Rd, a heteroaryl group which may be substituted with one or more Rd, a heteroaryloxy group which may be substituted with one or more Rd, an amino group, a C₁-C₆ alkylamino group, and a di(C₁-C₆ alkyl)amino group;
Rd is each independently selected from a C₁-C₆ alkyl group which may be substituted with one or more halogen atoms, a C₇-C₁₄ aralkyl group, a halogen atom, a hydroxy group, a cyano group, a nitro group, an amino group, a C₁-C₆ alkylamino group, and a di(C₁-C₆ alkyl)amino group;
Re is a hydrogen atom, a C₁-C₆ alkyl group which may be substituted with one or more Rc, an aryl group which may be substituted with one or more Rd, or a heteroaryl group which may be substituted with one or more Rd;
Rf, Rg and Ri are each independently selected from a hydrogen atom, and a C₁-C₆ alkyl group which may be substituted with one or more Rc;
Rh and Rj are each independently selected from a hydrogen atom, a C₁-C₆ alkyl group which may be substituted with one or more Rc, a C₁-C₆ alkylcarbonyl group which may be substituted with one or more Rc, an aryl group which may be substituted with one or more Rd, a heteroaryl group which may be substituted with one or more Rd, a carbamoyl group, a C₁-C₆ alkoxycarbonyl group which may be substituted with one or more Rc, and a C₁-C₆ alkylsulfonyl group which may be substituted with one or more Rc; or
Re and Rf, Rg and Rh, and Ri and Rj together with the nitrogen atom to which they are attached may form a 4- to 7-membered heterocyclic ring;
or a prodrug thereof or a pharmaceutically acceptable salt thereof.

2. A compound represented by Formula (IIa): 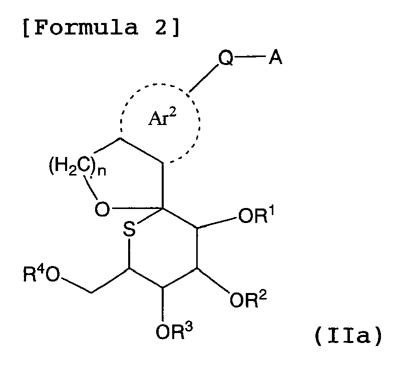 wherein R¹, R², R³, and R⁴ are each independently selected from a hydrogen atom, a C₁-C₆ alkyl group which may be substituted with one or more Ra, a C₇-C₁₄ aralkyl group which may be substituted with one or more Rb, and -C(=O)Rx;
Rx is a C₁-C₆ alkyl group which may be substituted with one or more Ra, an aryl group which may be substituted with one or more Rb, a heteroaryl group which may be substituted with one or more Rb, a C₁-C₆ alkoxy group which may be substituted with one or more Ra, or -NReRf;
Ar² is a monocyclic aromatic carbocyclic ring which may be substituted with one or more Rb or a monocyclic aromatic heterocyclic ring which may be substituted with one or more Rb;
Q is -(CH₂)ₘ-(L)ₚ- or -(L)ₚ-(CH₂)ₘ-;
m is an integer selected from 0 to 2, n is an integer selected from 1 and 2, and p is an integer selected from 0 and 1;
L is -O-, -S- or -NR⁵-,
R⁵ is selected from a hydrogen atom, a C₁-C₆ alkyl group which may be substituted with one or more Ra, and -C(=O)Rx;
A is an aryl group which may be substituted with one or more Rb or a heteroaryl group which may be substituted with one or more Rb, wherein the aryl group or the heteroaryl group may form a condensed ring together with an aromatic carbocyclic ring or an aromatic heterocyclic ring;
Ra is each independently selected from a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRgRh, a C₁-C₆ alkoxycarbonyl group which may be substituted with one or more Rc, and a C₁-C₆ alkylcarbonyl group which may be substituted with one or more Rc;
Rb is each independently selected from a C₁-C₆ alkyl group which may be substituted with one or more Rc, a C₃-C₈ cycloalkyl group which may be substituted with one or more Rc, a C₂-C₆ alkenyl group which may be substituted with one or more Rc, a C₂-C₆ alkynyl group which may be substituted with one or more Rc, a C₇-C₁₄ aralkyl group which may be substituted with one or more Rd, a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRiRj, a C₁-C₆ alkylcarbonyl group which may be substituted with one or more Rc, a C₁-C₆ alkoxycarbonyl group which may be substituted with one or more Rc, a C₁-C₃ alkylenedioxy group, a heterocyclyl group, and a heterocyclyloxy group;
Rc is each independently selected from a halogen atom, a hydroxy group, a cyano group, a nitro group, a carboxy group, a C₁-C₆ alkoxy group, an aryl group which may be substituted with one or more Rd, an aryloxy group which may be substituted with one or more Rd, a heteroaryl group which may be substituted with one or more Rd, a heteroaryloxy group which may be substituted with one or more Rd, an amino group, a C₁-C₆ alkylamino group, and a di(C₁-C₆ alkyl)amino group;
Rd is each independently selected from a C₁-C₆ alkyl group which may be substituted with one or more halogen atoms, a C₇-C₁₄ aralkyl group, a halogen atom, a hydroxy group, a cyano group, a nitro group, an amino group, a C₁-C₆ alkylamino group, and a di(C₁-C₆ alkyl)amino group;
Re is a hydrogen atom, a C₁-C₆ alkyl group which may be substituted with one or more Rc, an aryl group which may be substituted with one or more Rd, or a heteroaryl group which may be substituted with one or more Rd;
Rf, Rg and Ri are each independently selected from a hydrogen atom, and a C₁-C₆ alkyl group which may be substituted with one or more Rc;
Rh and Rj are each independently selected from a hydrogen atom, a C₁-C₆ alkyl group which may be substituted with one or more Rc, a C₁-C₆ alkylcarbonyl group which may be substituted with one or more Rc, an aryl group which may be substituted with one or more Rd, a heteroaryl group which may be substituted with one or more Rd, a carbamoyl group, a C₁-C₆ alkoxycarbonyl group which may be substituted with one or more Rc, and a C₁-C₆ alkylsulfonyl group which may be substituted with one or more Rc; or
Re and Rf, Rg and Rh, and Ri and Rj together with the nitrogen atom to which they are attached amy form a 4- to 4-membered heterocyclic ring;
or a prodrug thereof or a pharmaceutically acceptable salt thereof.

3. A compound represented by Formula (IIb): 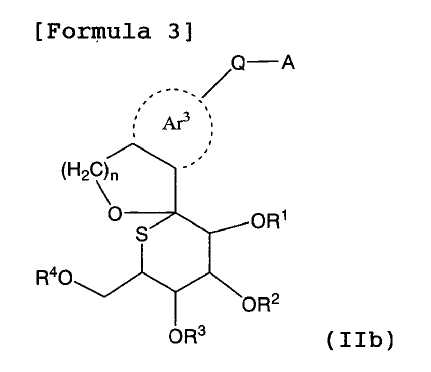 wherein R¹, R², R³, and R⁴ are each independently selected from a hydrogen atom, a C₁-C₆ alkyl group which may be substituted with one or more Ra, a C₇-C₁₄ aralkyl group which may be substituted with one or more Rb, and -C(=O)Rx;
Rx is a C₁-C₆ alkyl group which may be substituted with one or more Ra, an aryl group which may be substituted with one or more Rb, a heteroaryl group which may be substituted with one or more Rb, a C₁-C₆ alkoxy group which may be substituted with one or more Ra, or -NReRf;
wherein Ar³ is selected from quinoline, isoquinoline, 4H-quinolidine, phthalazine, naphthyridine, quinoxaline, quinazoline, cinnoline, pteridine, indole, indoline, benzothiophene, 1-methyl-1H-indole, benzofuran, naphthalene, benzisothiazole, benzisoxazole, indazole, benzimidazole, benzotriazole and indene;
Q is -(CH₂)ₘ(L)ₚ- or -(L)ₚ-(CH₂)ₘ;
m is an integer selected from 0 to 2, n is an integer selected from 1 and 2, and p is an integer selected from 0 and 1;
L is -O-, -S- or -NR⁵-,
R⁵ is selected from a hydrogen atom, a C₁-C₆ alkyl group which may be substituted with one or more Ra, and -C(=O)Rx;
A is an aryl group which may be substituted with one or more Rb or a heteroaryl group which may be substituted with one or more Rb, wherein the aryl group or the heteroaryl group may form a condensed ring together with an aromatic carbocyclic ring or an aromatic heterocyclic ring;
Ra is each independently selected from a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRgRh, a C₁-C₆ alkoxycarbonyl group which may be substituted with one or more Rc, and a C₁-C₆ alkylcarbonyl group which may be substituted with one or more Rc;
Rb is each independently selected from a C₁-C₆ alkyl group which may be substituted with one or more Rc, a C₃-C₈ cycloalkyl group which may be substituted with one or more Rc, a C₂-C₆ alkenyl group which may be substituted with one or more Rc, a C₂-C₆ alkynyl group which may be substituted with one or more Rc, a C₇-C₁₄ aralkyl group which may be substituted with one or more Rd, a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRiRj, a C₁-C₆ alkylcarbonyl group which may be substituted with one or more Rc, a C₁-C₆ alkoxycarbonyl group which may be substituted with one or more Rc, a C₁-C₃ alkylenedioxy group, a heterocyclyl group and a heterocyclyloxy group;
Rc is each independently selected from a halogen atom, a hydroxy group, a cyano group, a nitro group, a carboxy group, a C₁-C₆ alkoxy group, an aryl group which may be substituted with one or more Rd, an aryloxy group which may be substituted with one or more Rd, a heteroaryl group which may be substituted with one or more Rd, a heteroaryloxy group which may be substituted with one or more Rd, an amino group, a C₁-C₆ alkylamino group and a di(C₁-C₆ alkyl)amino group;
Rd is each independently selected from a C₁-C₆ alkyl group which may be substituted with one or more halogen atoms, a C₇-C₁₄ aralkyl group, a halogen atom, a hydroxy group, a cyano group, a nitro group, an amino group, a C₁-C₆ alkylamino group and a di(C₁-C₆ alkyl)amino group;
Re is a hydrogen atom, a C₁-C₆ alkyl group which may be substituted with one or more Rc, an aryl group which may be substituted with one or more Rd, or a heteroaryl group which may be substituted with one or more Rd;
Rf, Rg and Ri are each independently selected from a hydrogen atom, and a C₁-C₆ alkyl group which may be substituted with one or more Rc;
Rh and Rj are each independently selected from a hydrogen atom, a C₁-C₆ alkyl group which may be substituted with one or more Rc, a C₁-C₆ alkylcarbonyl group which may be substituted with one or more Rc, an aryl group which may be substituted with one or more Rd, a heteroaryl group which may be substituted with one or more Rd, a carbamoyl group, a C₁-C₆ alkoxycarbonyl group which may be substituted with one or more Rc, and a C₁-C₆ alkylsulfonyl group which may be substituted with one or more Rc; or
Re and Rf, Rg and Rh, and Ri and Rj together with the nitrogen atom to which they are attached may form a 4- to 7-membered heterocyclic ring;
or a prodrug thereof or a pharmaceutically acceptable salt thereof.

4. A compound represented by Formula (IIc): 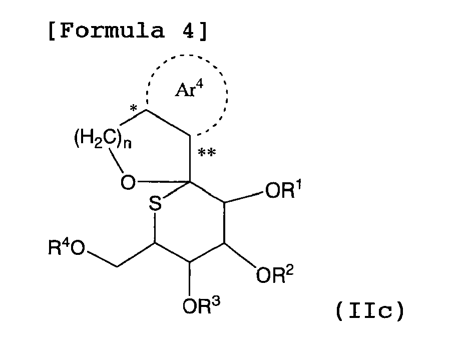 wherein R¹, R², R³, and R⁴ are each independently selected from a hydrogen atom, a C₁-C₆ alkyl group which may be substituted with one or more Ra, a C₇-C₁₄ aralkyl group which may be substituted with one or more Rb, and -C(=O)Rx;
Rx is a C₁-C₆ alkyl group which may be substituted with one or more Ra, an aryl group which may be substituted with one or more Rb, a heteroaryl group which may be substituted with one or more Rb, a C₁-C₆ alkoxy group which may be substituted with one or more Ra, or -NReRf;
wherein ring Ar⁴ is selected from the groups represented by Formula (a) to (m): 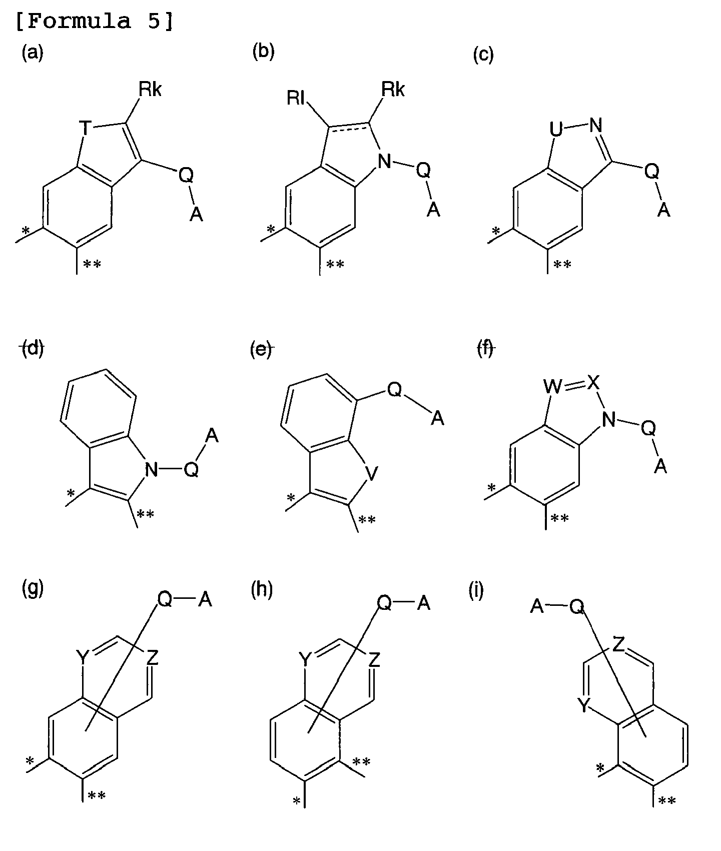 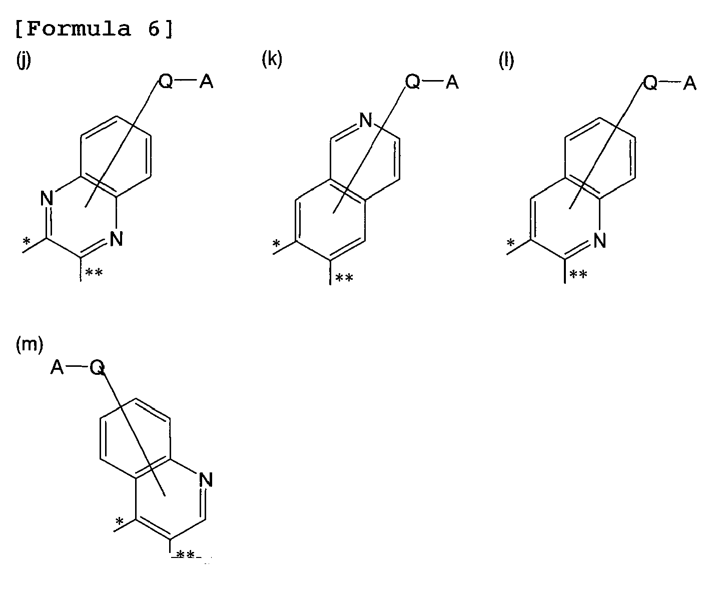 wherein T is an oxygen atom, a sulfur atom, CH₂ or N-Rm;
Rk and R1 are independently selected from a hydrogen atom, a halogen atom, and a C₁-C₆ alkyl group;
U is N-Rm, an oxygen atom or a sulfur atom;
V is a sulfur atom, an oxygen atom or N-Rm;
Rm is a hydrogen atom or C₁-C₆ alkyl group;
W and X are independently selected from a nitrogen atom and carbon atom, with the proviso that at least one of W and X is a nitrogen atom;
Y and Z are independently selected from a nitrogen atom and a carbon atom, with the proviso that when Y or Z is an nitrogen atom, the nitrogen atom does not have the substituent -Q-A; and
[Formula 7]-̅-̅-̅-̅-̅-̅
represents a single bond or a double bond;
Q is -(CH₂)ₘ-(L)ₚ- or -(L)ₚ-(CH₂)ₘ-;
m is an integer selected from 0 to 2, n is an integer selected from 1 and 2, and p is an integer selected from 0 and 1;
L is -O-, -S- or -NR⁵-,
R⁵ is selected from a hydrogen atom, a C₁-C₆ alkyl group which may be substituted with one or more Ra, and -C(=O)Rx;
A is an aryl group which may be substituted with one or more Rb or a heteroaryl group which may be substituted with one or more Rb, wherein the aryl group or the heteroaryl group may form a condensed ring together with an aromatic carbocyclic ring or an aromatic heterocyclic ring;
* and ** respectively represent a bonding site;
Ra is each independently selected from a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRgRh, a C₁-C₆ alkoxycarbonyl group which may be substituted with one or more Rc, and a C₁-C₆ alkylcarbonyl group which may be substituted with one or more Rc;
Rb is each independently selected from a C₁-C₆ alkyl group which may be substituted with one or more Rc, a C₃-C₈ cycloalkyl group which may be substituted with one or more Rc, a C₂-C₆ alkenyl group which may be substituted with one or more Rc, a C₂-C₆ alkynyl group which may be substituted with one or more Rc, a C₇-C₁₄ aralkyl group which may be substituted with one or more Rd, a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRiRj, a C₁-C₆ alkylcarbonyl group which may be substituted with one or more Rc, a C₁-C₆ alkoxycarbonyl group which may be substituted with one or more Rc, a C₁-C₃ alkylenedioxy group, a heterocyclyl group, and a heterocyclyloxy group;
Rc is each independently selected from a halogen atom, a hydroxy group, a cyano group, a nitro group, a carboxy group, a C₁-C₆ alkoxy group, an aryl group which may be substituted with one or more Rd, an aryloxy group which may be substituted with one or more Rd, a heteroaryl group which may be substituted with one or more Rd, a heteroaryloxy group which may be substituted with one or more Rd, an amino group, a C₁-C₆ alkylamino group and a di(C₁-C₆ alkyl)amino group;
Rd is each independently selected from a C₁-C₆ alkyl group which may be substituted with one or more halogen atoms, a C₇-C₁₄ aralkyl group, a halogen atom, a hydroxy group, a cyano group, a nitro group, an amino group, a C₁-C₆ alkylamino group, and a di(C₁-C₆ alkyl)amino group;
Re is a hydrogen atom, a C₁-C₆ alkyl group which may be substituted with one or more Rc, an aryl group which may be substituted with one or more Rd, or a heteroaryl group which may be substituted with one or more Rd;
Rf, Rg and Ri are each independently selected from a hydrogen atom, and a C₁-C₆ alkyl group which may be substituted with one or more Rc;
Rh and Rj are each independently selected from a hydrogen atom, a C₁-C₆ alkyl group which may be substituted with one or more Rc, a C₁-C₆ alkylcarbonyl group which may be substituted with one or more Rc, an aryl group which may be substituted with one or more Rd, a heteroaryl group which may be substituted with one or more Rd, a carbamoyl group, a C₁-C₆ alkoxycarbonyl group which may be substituted with one or more Rc, and a C₁-C₆ alkylsulfonyl group which may be substituted with one or more Rc; or
Re and Rf, Rg and Rh, and Ri and Rj together with the nitrogen atom to which they are attached may form a 4- to 7-membered heterocyclic ring;
or a prodrug thereof or a pharmaceutically acceptable salt thereof.

5. A compound represented by Formula (III): 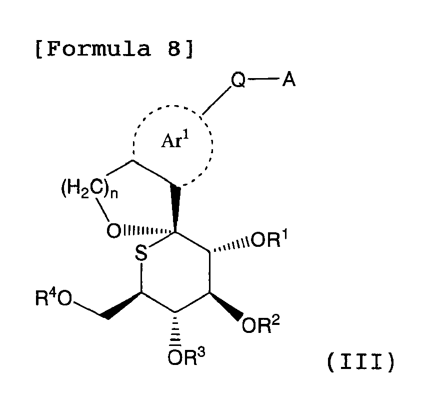 wherein R¹, R², R³, and R⁴ are each independently selected from a hydrogen atom, a C₁-C₆ alkyl group which may be substituted with one or more Ra, a C₇-C₁₄ aralkyl group which may be substituted with one or more Rb, and -C(=O)Rx;
Rx is a C₁-C₆ alkyl group which may be substituted with one or more Ra, an aryl group which may be substituted with one or more Rb, a heteroaryl group which may be substituted with one or more Rb, a C₁-C₆ alkoxy group which may be substituted with one or more Ra, or -NReRf;
Ar¹ is an aromatic carbocyclic ring which may be substituted with one or more Rb, or an aromatic heterocyclic ring which may be substituted with one or more Rb, in which the aromatic carbocyclic ring and the aromatic heterocyclic ring may form a condensed ring;
Q is -(CH₂)ₘ-(L)ₚ- or -(L)ₚ-(CH₂)ₘ-;
m is an integer selected from 0 to 2, n is an integer selected from 1 and 2, and p is an integer selected from 0 and 1;
L is -O-, -S- or -NR⁵-,
R⁵ is selected from a hydrogen atom, a C₁-C₆ alkyl group which may be substituted with one or more Ra, and -C(=O)Rx;
A is an aryl group which may be substituted with one or more Rb, or a heteroaryl group which may be substituted with one or more Rb, wherein the aryl group or the heteroaryl group may form a condensed ring together with an aromatic carbocyclic ring or an aromatic heterocyclic ring;
Ra is each independently selected from a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRgRh, a C₁-C₆ alkoxycarbonyl group which may be substituted with one or more Rc, and a C₁-C₆ alkylcarbonyl group which may be substituted with one or more Rc;
Rb is each independently selected from a C₁-C₆ alkyl group which may be substituted with one or more Rc, a C₃-C₈ cycloalkyl group which may be substituted with one or more Rc, a C₂-C₆ alkenyl group which may be substituted with one or more Rc, a C₂-C₆ alkynyl group which may be substituted with one or more Rc, a C₇-C₁₄ aralkyl group which may be substituted with one or more Rd, a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRiRj, a C₁-C₆ alkylcarbonyl group which may be substituted with one or more Rc, a C₁-C₆ alkoxycarbonyl group which may be substituted with one or more Rc, a C₁-C₃ alkylenedioxy group, a heterocyclyl group, and a heterocyclyloxy group;
Rc is each independently selected from a halogen atom, a hydroxy group, a cyano group, a nitro group, a carboxy group, a C₁-C₆ alkoxy group, an aryl group which may be substituted with one or more Rd, an aryloxy group which may be substituted with one or more Rd, a heteroaryl group which may be substituted with one or more Rd, a heteroaryloxy group which may be substituted with one or more Rd, an amino group, a C₁-C₆ alkylamino group and a di(C₁-C₆ alkyl)amino group;
Rd is each independently selected from a C₁-C₆ alkyl group which may be substituted with one or more halogen atoms, a C₇-C₁₄ aralkyl group, a halogen atom, a hydroxy group, a cyano group, a nitro group, an amino group, a C₁-C₆ alkylamino group, and a di(C₁-C₆ alkyl)amino group;
Re is a hydrogen atom, a C₁-C₆ alkyl group which may be substituted with one or more Rc, an aryl group which may be substituted with one or more Rd, or a heteroaryl group which may be substituted with one or more Rd;
Rf, Rg and Ri are each independently selected from a hydrogen atom, and a C₁-C₆ alkyl group which may be substituted with one or more Rc;
Rh and Rj are each independently selected from a hydrogen atom, a C₁-C₆ alkyl group which may be substituted with one or more Rc, a C₁-C₆ alkylcarbonyl group which may be substituted with one or more Rc, an aryl group which may be substituted with one or more Rd, a heteroaryl group which may be substituted with one or more Rd, a carbamoyl group, a C₁-C₆ alkoxycarbonyl group which may be substituted with one or more Rc, and a C₁-C₆ alkylsulfonyl group which may be substituted with one or more Rc; or
Re and Rf, Rg and Rh, and Ri and Rj together with the nitrogen atom to which they are attached may form a 4- to 7-membered heterocyclic ring;
or a prodrug thereof or a pharmaceutically acceptable salt thereof.

6. a compound represented by Formula (III): 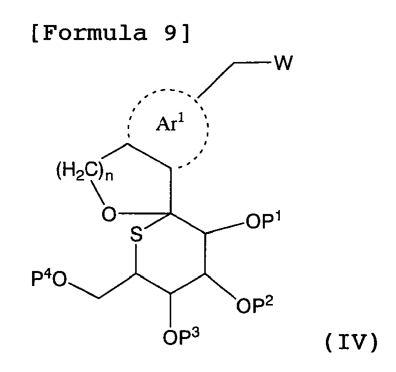 wherein n is an integer selected from 1 and 2;
Ar¹ is an aromatic carbocyclic ring which may be substituted with one or more Rb, or an aromatic heterocyclic ring which may be substituted with one or more Rb, in which the aromatic carbocyclic ring and the aromatic heterocyclic ring may form a condensed ring;
Rb is each independently selected from a C₁-C₆ alkyl group which may be substituted with one or more Rc, a C₃-C₈ cycloalkyl group which may be substituted with one or more Rc, a C₂-C₆ alkenyl group which may be substituted with one or more Rc, a C₂-C₆ alkynyl group which may be substituted with one or more Rc, a C₇-C₁₄ aralkyl group which may be substituted with one or more Rd, a halogen atom, a hydroxy group, a cyano group, a nitro group, a carboxy group, a C₁-C₆ alkoxy group which may be substituted with one or more Rc, an aryl group which may be substituted with one or more Rd, an aryloxy group which may be substituted with one or more Rd, a heteroaryl group which may be substituted with one or more Rd, a heteroaryloxy group which may be substituted with one or more Rd, a mercapto group, a C₁-C₆ alkylthio group which may be substituted with one or more Rc, a C₁-C₆ alkylsulfinyl group which may be substituted with one or more Rc, a C₁-C₆ alkylsulfonyl group which may be substituted with one or more Rc, -NRiRj, a C₁-C₆ alkylcarbonyl group which may be substituted with one or more Rc, a C₁-C₆ alkoxycarbonyl group which may be substituted with one or more Rc, a C₁-C₃ alkylenedioxy group, a heterocyclyl group, and a heterocyclyloxy group;
Rc is each independently selected from a halogen atom, a hydroxy group, a cyano group, a nitro group, a carboxy group, a C₁-C₆ alkoxy group, an aryl group which may be substituted with one or more Rd, an aryloxy group which may be substituted with one or more Rd, a heteroaryl group which may be substituted with one or more Rd, a heteroaryloxy group which may be substituted with one or more Rd, an amino group, a C₁-C₆ alkylamino group, and a di(C₁-C₆ alkyl)amino group;
Rd is each independently selected from a C₁-C₆ alkyl group which may be substituted with one or more halogen atoms, a C₇-C₁₄ aralkyl group, a halogen atom, a hydroxy group, a cyano group, a nitro group, an amino group, a C₁-C₆ alkylamino group, and a di(C₁-C₆ alkyl)amino group;
Ri is a hydrogen atom or a C₁-C₆ alkyl group;
Rj is each independently selected from a hydrogen atom, a C₁-C₆ alkyl group which may be substituted with one or more Rc, a C₁-C₆ alkylcarbonyl group which may be substituted with one or more Rc, an aryl group which may be substituted with one or more Rd, a heteroaryl group which may be substituted with one or more Rd, a carbamoyl group, a C₁-C₆ alkoxycarbonyl group which may be substituted with one or more Rc, and a C₁-C₆ alkylsulfonyl group which may be substituted with one or more Rc; or
Ri and Rj together with the nitrogen atom to which they are attached may form a 4- to 7-membered heterocyclic ring;
W is -O-Z or a halogen atom;
Z is a hydrogen atom, an acyl group or a benzyl group;
P¹, P², P³ and P⁴ are independently selected from a hydrogen atom, an acyl group and a benzyl group.

7. A pharmaceutical composition comprising the compound according to any one of claims 1 to 5, or a prodrug thereof or a pharmaceutically acceptable salt thereof.

8. A pharmaceutical composition according to claim 7, which is used as a Na⁺-glucose cotransporter inhibitor.

9. A pharmaceutical composition according to claim 7, which is used for prevention or treatment of diabetes including insulin-dependent diabetes (Type 1 diabetes) and non-insulin-dependent diabetes (Type 2 diabetes), hyperglycemia, diabetic complications, or obesity.

10. A method for preventing or treating diabetes, hyperglycemia, diabetic complications caused thereby, or obesity, comprising administering to a patient of an effective therapeutic dose of the compound according to any one of claims 1 to 5, or a prodrug thereof or pharmaceutically acceptable salt thereof.
